(19) Europäisches Patentamt
European Patent Office
Office européen des brevets

(11) EP 1 023 048 B1

(12) **EUROPEAN PATENT SPECIFICATION**

(45) Date of publication and mention of the grant of the patent:
**07.03.2007 Bulletin 2007/10**

(21) Application number: **98957320.9**

(22) Date of filing: **09.10.1998**

(51) Int Cl.:
***A61K 9/127*** *(2006.01)* ***C12N 15/88*** *(2006.01)*

(86) International application number:
**PCT/US1998/021500**

(87) International publication number:
**WO 1999/018933 (22.04.1999 Gazette 1999/16)**

(54) **METHODS FOR ENCAPSULATING NUCLEIC ACIDS IN LIPID BILAYERS**

VERFAHREN ZUR VERKAPSELUNG VON NUKLEINSÄUREN IN LIPIDDOPPELSCHICHTEN

METHODES D'ENCAPSULATION D'ACIDES NUCLEIQUES DANS DES BICOUCHES LIPIDIQUES

(84) Designated Contracting States:
**AT BE CH CY DE DK ES FI FR GB GR IE IT LI LU MC NL PT SE**
Designated Extension States:
**AL LT LV MK RO SI**

(30) Priority: **10.10.1997 US 63473 P**

(43) Date of publication of application:
**02.08.2000 Bulletin 2000/31**

(73) Proprietors:
- **INEX Pharmaceuticals Corp.
Burnaby,
British Columbia V5J 5J8 (CA)**
- **Saravolac, Edward G.
Vancouver,
British Columbia V6K 1X9 (CA)**
- **Zhang, Yuan-Peng
Vancouver,
British Columbia V6P 3E8 (CA)**
- **Wheeler, Jeffery J.
Vancouver,
British Columbia V3S 8T3 (CA)**
- **Cullis, Pieter R.
Vancouver,
British Columbia V6R 1H4 (CA)**
- **Scherrer, Peter
Vancouver,
British Columbia V6H 2T5 (CA)**
- **Kojic, Ljiljana D.
Vancouver,
British Columbia V5T 3B2 (CA)**
- **Ludkovski, Olga
Port Coquitlam,
British Columbia V3C 6H2 (CA)**

(72) Inventors:
- **SARAVOLAC, Edward, G.
Vancouver, British Columbia V6K 1X9 (CA)**
- **ZHANG, Yuan-Peng
Vancouver, British Columbia V6P 3E8 (CA)**
- **WHEELER, Jeffery, J.
Vancouver, British Columbia V3S 8T3 (CA)**
- **CULLIS, Pieter, R.
Vancouver, British Columbia V6R 1H4 (CA)**
- **SCHERRER, Peter
Vancouver, British Columbia V6H 2T5 (CA)**
- **KOJIC, Ljiljana, D.
Vancouver, British Columbia V5T 3B2 (CA)**
- **LUDKOVSKI, Olga
Port Coquitlam, British Columbia V3C 6H2 (CA)**

(74) Representative: **Bizley, Richard Edward et al
HLBBshaw
Merlin House
Falconry Court
Baker's Lane
Epping, Essex CM16 5DQ (GB)**

(56) References cited:
**WO-A-96/37194 WO-A-96/40964**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

This invention relates to lipid-based formulations for nucleic acid delivery to cells, methods for the preparation of such formulations and, in particular, to lipid encapsulated plasmids. The compositions are safe and practical for clinical use.

### BACKGROUND OF THE INVENTION

Gene therapy is an area of current interest which involves the introduction of genetic material into a cell to facilitate expression of a deficient protein. Plasmid DNA has been encapsulated or complexed with lipid-based carriers by a number of methods including reverse phase evaporation (*Fraley, et al., J. Biol. Chem.* , **255:**10431-10435 (1980); Soriano, *et al., Proc. Natl. Acad. Sci. USA,* **80:**7128-7131 (1983); Nakanishi, *et al., Exper. Cell Res.* , **159:**399-409 (1985); Nandi, *et al., J. Biol. Chem.* , **261:**16722-16726 (1986); and Alino, *et al., Biochem. Biophys. Res. Commun.* , **192:**174-181 (1993)); $Ca^{2+}$ EDTA chelation (Szelei, *et al., Biochem. J.* , **259:**549-553 (1989)); detergent dialysis (Wang, *et al., Proc. Natl. Acad. Sci. USA,* **84:**7851-7855 (1987)); lipid hydration (Lurquin, *Nucleic Acids Res.,* **6:**3773-3784 (1979); *Yagi, et al., Biochem. Mol. Biol. International,* **32:**167-171 (1994)); ether injection (Fraley, *et al., Proc. Natl. Acad. Sci.* , **76:**3348-3352 (1979); *Nicolau, et al., Biochem. Biophys. Res. Comm.* , **108:**982-986 (1982)); and sonication (Jay, *et al., Bioconj. Chem.* , **6:**187-194 (1987) and *Puyal, et al., Eur. J. Biochem.* , **228**:697-703 (1993)).

Reverse phase techniques typically encapsulate only about 10 to 20 % of the DNA in solution and the final DNA to lipid ratio is quite low. For example, Nakanishi, *et al.* (*Exper. Cell* Res., **159**:399-409 (1985)) reported a final DNA to lipid ratio of 1.5 μg DNA to 2.5 mg lipid, while Soriano, *et al. (Proc. Natl. Acad. Sci. USA,* **80:**7128-7131 (1983)) reported a DNA to lipid ratio of about 14 μg DNA to 60 μmol of lipids. The maximum theoretical encapsulation efficiency expected by reverse phase is only about 40%. Other methods, such as rehydration of freeze dried vesicles with DNA, have been shown to yield trapping efficiencies between 30 and 40% (Baru, *et al., Gene,* 161:143-150 (1995)). Others have sought to increase the entrapment of DNA by the inclusion of cationic lipids in the lipid suspension (Stavridis, *et al.,* 1986; Puyal, *et al., Eur. J. Biochem.,* **228**:697-703 (1995)), or by rendering the DNA positively charged by coating it with basic proteins such as lysozymes (Jay, *et al., Proc. Natl. Acad. Sci. USA,* **84:**1978-1980 (1987)). Although trapping efficiencies as high as 50% were achieved by the lysozyme method, the amount of DNA loaded per mg of lipid was low (5 μg/mg lipid) and the largest DNA molecule tested was only 1 kb. Trapping efficiencies as high as 60-90% were achieved by Puyal, *et al.* (*Eur. J. Biochem.,* **228**:697-703 (1995)) with a higher DNA to lipid ratio (13 μg/μmole lipid) using a 6.3 kb ssDNA (M13 phage). The major drawback of this technique and the one described by Jay, *et al., Bioconj. Chem.,* 6:187-194 (1987)) is that sonication was used. Sonication of DNA typically leads to some degradation of the lipid vesicle.

Detergent dialysis is a method of encapsulation which has no deleterious effects on the DNA. Wang, *et al., Proc. Natl. Acad. Sci USA,* **84:**7851-7855 (1987) applied a detergent dialysis technique followed by extrusion through a 0.2 μm polycarbonate filter. A 4.6 kb plasmid was entrapped in vesicles approximately 200 nm in diameter with a trapping efficiency of about 14-17 %, giving a DNA to lipid ratio of about 26 μg DNA to 10 μmole lipid.

Nucleic acid-lipid particles for nucleic acid delivery to cells and methods for their preparation are disclosed in international patent application number WO 96/40964, The nucleic acid-lipid particles disclosed in WO 96/40964 are serum-stable, and the nucleic acid In the nucleic acid-lipid particle is protected from nuclease degradation.

Ideally, a delivery vehicle for a nucleic acid or plasmid will have the following characteristics: a) small enough and long lived enough to distribute from local injection sites when given intravenously, b) capable of carrying a large amount of DNA per particle to enable transfection of all sizes of genes and to reduce the volume of injection, c) homogeneous, d) reproducible, e) protective of DNA from extracellular degradation and f) capable of transfecting target cells in such a way that the DNA is not digested intracellularly.

The present invention provides such compositions and methods for their preparation and use.

### SUMMARY OF THE INVENTION

In one aspect, the present invention provides compositions which are nucleic acid (*e.g.*, plasmid)-lipid compositions. In these compositions, a nucleic acid (*e.g.,* plasmid or an antisense molecule) is encapsulated in a self-assembling lipid vesicle in an amount of from about 20 μg nucleic acid/mg lipid to about 400 μg nucleic acid/mg lipid. The lipid vesicle will typically be a liposome or lipid particle (a bilayer vehicle coating the plasmid and having little or no aqueous interior). The lipid vesicle can be prepared from a wide variety of lipids or combinations of lipids. The compositions can also include targeting groups and modified lipids (*e.g.*, ATTA-lipids, gangliosides, such as ganglioside $G_{M1}$), PEG-lipids, such as PEG-ceramides, and lipids having reactive functional groups for the attachment of targeting groups or circulation stabilizers). Preferably, the lipid vesicles will comprise cationic lipids and fusogenic lipids. Additionally, the nucleic acid (*e.g.*, plasmid)-lipid compositions described herein can be prepared having a narrow size distribution (typically 50 nm to about 150 nm) without the use of sizing methods, such as extrusion and sonication methods.

In another aspect, the present invention provides methods for the encapsulation of nucleic acids, antisense, ribozymes and, particularly, plasmids in a lipid bilayer carrier. Such methods are related to a detergent dialysis method using cationic lipids of any desired concentration in combination with a dialysis buffer of an ionic strength (salt concentration, type of ions) specific for the given cationic lipid concentration. With the dialysis buffer of appropriate ionic strength, the methods provide encapsulation of 40-80% of the nucleic acid solution. The compositions above, and those formed by the methods described below, exhibit preferably less than about 30 % degradation, more preferably, less than about 15 % degradation and, even more preferably, less than about 5 % degradation when digested with 0.1 to 10 U and, more preferably, 1 U of a nuclease after 30 minutes at 37°C.

In particular, the invention provides a method for encapsulating a nucleic acid in a lipid bilayer carrier, comprising:

(a) combining a nucleic acid with a lipid-detergent mixture comprising an aggregation-preventing agent (*e.g.,* an ATTA-lipid, a PEG-lipid, such as a PEG-ceramide, a ganglioside, *etc.*) in an amount of about 5 mol % to about 20 mol%, cationic lipids in an amount of about 0.5 mol % to about 50 mol % by weight, neutral or fusogenic lipids in an amount of from about 30 mol % to about 70 mol % and a detergent, to provide a nucleic acid-lipid-detergent mixture; and

(b) dialyzing the nucleic acid-lipid-detergent mixture against a buffered salt solution and to encapsulate the nucleic acid in a lipid bilayer carrier. In these methods, the ionic strength (salt concentration) is adjusted for the cationic lipid concentration used in the lipid mixture and when necessary for the polynucleotide selected for encapsulation to entrap from about 40 % to about 80 % of the nucleic acid for any given concentration of cationic lipid.

In another aspect, the present invention provides methods for introducing nucleic acids into cells and for inhibiting tumor growth in cells using the lipid-nucleic acid formulations described above.

Other features, objects and advantages of the invention and its preferred embodiments will become apparent from the detailed description which follows.

## BRIEF DESCRIPTION OF THE DRAWINGS

**Figure 1** provides an illustration of the phase properties of lipids.

**Figure 2** illustrates the conical form of one fusogenic lipid and further illustrates how fusion with another membrane can occur.

**Figure 3** provides an illustration of the detergent dialysis procedure for entrapping nucleic acids in fusogenic lipid vesicles.

**Figure 4** illustrates the structures of three PEG-Ceramide conjugates and also provides half-times for their dissociation from a lipid vesicle.

**Figure 5.** Encapsulation of pINEXL018 plasmid using DODAC/DOPE/PEG-Cer-C8 (30:55:15 mol%) by detergent dialysis in citrate buffer. Effect of varying NaCl concentration with constant citrate concentration (100 mM Na citrate, 5 mM HEPES, pH:7.2) is illustrated. The encapsulation efficiency and polydispersity, $\chi^2$ (a measure of formulation homogeneity), of formulations are plotted as functions of NaCl. Successful formulations are indicated by the high encapsulation and low $\chi^2$.

**Figure 6.** Encapsulation of pINEX L018 plasmid using DODAC/DOPE/PEG-Cer-C8 system by detergent dialysis in citrate buffer containing constant NaCl concentration (150 mM) and 5 mM HEPES, pH:7.2. The relationship between varying DODAC mol % and the optimal citrate concentration and the effect upon encapsulation efficiency is demonstrated. Each point represents a formulation of acceptable size and encapsulation efficiency. The optimal range of citrate and DODAC concentrations are indicated by the solid line. Typically, those preparations prepared below the optimal citrate concentration have large sizes or aggregate, and those formulations prepared above the optimal citrate concentration have low encapsulation efficiencies (0-30%).

**Figure 7.** Effect of DODAC concentration on plasmid encapsulation. In this study, the effect of small (1 mol%) changes of DODAC concentration were tested at constant lipid (10 mg/mL), plasmid (400 μg/mL) and buffer concentrations. Encapsulation efficiency dropped significantly with a decrease in DODAC concentration, indicating that care must be taken to precisely deliver DODAC at a given NaCl concentration. pINEXL002 was formulated in 150 mM $NaPO_4$, 175 mM NaCl, pH 7.4, and pINEXP005 was formulated in 150 mM $NaPO_4$, 150 mM NaCl pH 7.4.

**Figure 8.** Encapsulation of pINEXP005. Effect of varying NaCl with constant $NaPO_4$ concentration. The relationship between varying the salt concentration on the encapsulation of plasmid over a range of INEX TCS DODAC concentrations is illustrated. Negative NaCl concentrations indicate where the buffer concentration was decreased to an extent where no NaCl was included in the dialysis buffer, and the phosphate buffer concentration alone was decreased to achieve encapsulation. Formulations were prepared containing 10 mg/mL total lipid, 400 μg/mL plasmid DNA. In each 1.0 ml formulation, the PEG-C8 concentration was maintained at 15 mol%, the DODAC concentration was varied as indicated and the balance of the lipid was DOPE. At each DODAC concentration, formulations were dialyzed against a range of buffer salt concentrations. This study demonstrates that a range of encapsulation efficiencies can be achieved by adjusting the NaCl concentration in a phosphate buffer. Thus, the association of the DNA with the lipid particles can be regulated.

**Figure 9.** Encapsulation of pINEXL018. Effect of varying $NaPO_4$ and total lipid concentration. The effect of varying the lipid and phosphate buffer concentration on the encapsulation of plasmid over a range of INEX TCS DODAC concentrations is demonstrated. Formulations were prepared containing either 5 or 10 mg/mL total lipid, 400 μg/mL plasmid DNA. As in Figure 3, the formulations were prepared containing 15 mol % PEG-C8, the range of indicated DODAC concentrations and the balance of lipid made up with DODAC. In this study, the concentration of the dialysis phosphate buffer alone could be decreased to achieve encapsulation of a plasmid over this range of DODAC concentrations.

**Figure 10.** Effect of lipid concentration on the encapsulation of pINEXP005 in INEX 351. Formulations were prepared in 1 mL formulations containing 200 μg/mL plasmid and total lipids ranging from 1.25 mg/mL to 10 mg/mL. INEX 351 indicates a formulation containing 42.5 mol % DODAC, 42.5 mol% DOPE and 15 mol% PEG-C8. The formulations were dialyzed against 150 mM $NaPO_4$, 150 mM NaCl, pH 7.4. This study demonstrates that an increase in lipid concentration increases the extent of plasmid concentration. Thus, sufficient total lipid is required for loading of the plasmid into the particles.

**Figure 11.** Effect of plasmid concentration on encapsulation efficiency in INEX 351 particles were prepared in 1 mL formulations containing 5 mg/mL total lipid and the concentration of the plasmids ranged from 100 to 1000 μg/mL. Formulations containing pINEXP005 and pINEXL002 were dialyzed against 150 mM $NaPO_4$, pH 7.4 containing 150 mM and 175 mM NaCl, respectively. This study demonstrates that with increasing plasmid concentration, there is a decrease in the encapsulation efficiency. Thus, as seen in Figure 5, sufficient lipid is required in order to have significant loading of the plasmid into particles.

**Figure 12.** Sucrose density gradient isolation of an INEX TCS. Panel A: Separation of TCS formulation loaded with plasmid (lower band) from empty vesicles (upper bands) on the gradient after 12 hr centrifugation at 36,000 rpm (SW 41 Ti Rotor). Panel B: Removal of empty liposomes (non-DNA associated). Panel C: Removal of the DNA-loaded TCS from the gradient. Empty vesicles are removed from the TCS formulation using sucrose gradient isolation after the nonencapsulated plasmids are removed on a column of DEAE-Sepharose. In this preparation, a formulation containing 24 mol% DODAC (200 μg DNA/10 mg plasmid) was loaded onto a typical sucrose gradient which contained (from bottom to top) 10 % (2 mL), 5% (4 mL), and 2.5% (3 mL) sucrose in 20 mM HEPES buffered saline. The gradient was centrifuged for 14 hr at room temp. In this case, the DNA loaded TCS were removed using a syringe; however, fractions can be removed from the top of the gradient.

**Figure 13.** Distribution of lipid and DNA for a typical TCS containing 21 mol % DODAC after centrifugation on a sucrose density gradient for 5 hrs at 36000 rpm at 20°C using a Beckman ultracentrifuge with a SW41 Ti rotor. Fractions of 2 mL were removed from top to bottom and assayed for DNA and lipid concentration. Upper panel: distribution of $^3H$-labelled DNA. Lower panel: distribution of $^{14}C$-labelled lipid on the sucrose gradient.

**Figure 14.** Electron-microscopy (EM) of various INEX TCS formulations. (A) Freeze-fracture EM of a formulation containing 20 mol% DODAC. (B) Freeze-fracture EM of a formulation containing 42.5 mol % DODAC formulation.

**Figure 15.** Serum stability assay of INEX TCS. Sepharose CL-4B gel filtration chromatography after treatment with 80% normal mouse serum at 37°C for 60 minutes. Upper panel: free DNA vs. TCS containing 21 mol % DODAC (unencapsulated DNA removed). Lower panel: TCS containing 21 mol% DODAC after isolation on a sucrose density gradient. TCS when not incubated with serum, or incubated but protected from serum degradation was eluded at the void volume (fractions 5-8). DNA when degraded by serum was eluded in later fractions (fractions #10-20). The results showed that DNA was protected 74% and 84 % in the cleaned TCS (by DEAE column) and the isolated TCS respectively.

**Figure 16.** Serum and DNAse stability of plasmid, Free, in complexes (INEX100.3, complexes prepared by mixing DODAC/DOPE liposomes with pINEXL018) and encapsulated in INEX TCS. Agarose gel electrophoresis of pINEXL018 phenol-chloroform extracted after treatment with 80% normal mouse serum. Lanes 1-4: Free pINEXL018; Lanes 5-8: pINEXL018 encapsulated 42.5% DODAC containing TCS; Lanes 9-12: DOPE:DODAC complexed-pINEXL018. This study illustrates that the encapsulated DNA remains intact after treatment with serum and DNAse, while demonstrating that complexed DNA is not as nuclease stable.

**Figure 17.** Effect of DODAC concentration in TCS on the transfection of COS-7 cells in culture using isolated TCSs prepared with pINEXL018 and DODAC/DOPE/PEG-Cer-C8 by the detergent dialysis method using citrate buffer. Cells (40,000/well) were seeded in 24 well plates 24 hr before transfection. The dose was 1.0 μg/well and the

luciferase activity was assayed at 48 hr time point (n=3). INEX100.3 represents complexes prepared by mixing DODAC/DOPE liposomes with pINEXL018.

**Figure 18.** Effect of DODAC concentration in TCS on the transfection of Hep-G2 cells in culture using isolated TCSs prepared with pINEXL018 and DODAC/DOPE/PEG-Cer-C8 by the detergent dialysis method using citrate buffer. Cells (40,000/well) were seeded in 24 well plates 24 hr before transfection. The dose was 0.3μ g/well and the luciferase activity was assayed at 48 hr time point (n=3). INEX100.3 represents complexes prepared by mixing DODAC/DOPE liposomes with pINEXL018.

**Figure 19.** Effect of dose on the transfection of Hep-G2 cells in culture in isolated TCS prepared with pINEXL018 and DODAC/DOPE/PEG-Cer-C8 by the detergent dialysis method using citrate buffer. Cells (40,000/well) were seeded in 24 well plates 24 hr before transfection. The doses were 0.05, 0.3 & 0.7 μg/well and the luciferase activity was assayed at 48 hr time point (n=3). INEXice represents complexes prepared by mixing DODAC/DOPE liposomes with pINEXL018.

**Figure 20.** *In vitro* transfection study. Effect of the dose and DODAC concentrations of TCS prepared by phosphate buffer dialysis on the transfection and viability of COS-7 cells in culture. INEX TCS formulations containing 42.5, 38, 34 and 30 mol% DODAC (expressed as DOPE/DODAC ratios 50/50, 55/45, 60/40 and 63/35, respectively) were incubated with 35,000 cells/well at doses of 0.05, 0.1, 0.5 μg DNA. After 24 hr incubation, the cells were resuspended, lysed and measured for viability and luciferase activity (expressed as relative luminescence units). This study demonstrates that decreasing the DODAC concentration in non-isolated TCS increases the transfection activity and decreases the relative toxicity of the formulation.

**Figure 21.** Time course of transfection activity of TCS prepared by the phosphate dialysis method. COS-7 cells were incubated with 0.5 μg non-isolated TCS under the conditions described in **Figure 14.** After incubation for 24, 48 and 72 hours, transfection was measured as luciferase activity. This study illustrates that *in vitro* transfection increases for up to 48 hr and is sustained for well over 72 hr. As observed in **Figure 14,** transfection activity increased with decreasing DODAC concentration.

**Figure 22.** Effect of sucrose density gradient isolation of TCS prepared by the phosphate dialysis method on the transfection and viability of COS-7 cells in culture. COS-7 cells were incubated with both isolated and nonisolated TCS at 0.05, 0.1, 0.5 and 1.0 μg of DNA. TCS contained DODAC concentrations of 20, 24, 30 and 42.5 mol%. After 24 hours the cell viability and luciferase activity was measured. In total, **Figures 14-16** demonstrate that TCS mediated transfection is dose dependent. Maximal *in vitro* transfection activity is obtained at 30 mol % DODAC. Decreasing the TCS DODAC concentration reduces toxicity. Removal of empty TCS by sucrose density gradient isolation also decreases the toxicity of the formulations and increases the *in vitro* transfection activity.

**Figure 23.** *In vivo,* intraperitoneal transfection study for TCS containing various DODAC concentrations in TCS prepared by detergent dialysis in citrate buffer after isolation on transfection of B16 i.p. tumors. The TCSs were composed of pINEXL018/DODAC/DOPE/PEG-Cer-C8. TCS (30 μg DNA/500 μl/mouse) formulations were injected i.p. into mice 7 days after tumor seeding. Tumors were removed from mice 24 hours after treatment and were assayed for luciferase activity.

**Figure 24.** Effect of time on the transfection of i.p. B16 tumors. Isolated TCS (30 μg DNA/500 μl/mouse) of various DODAC concentrations prepared by detergent dialysis in citrate buffer were tested for luciferase transfection activity 24 and 48 hours after injection. The TCSs were composed of pINEXL018/DODAC/DOPE/PEG-Cer-C8.

**Figure 25.** Comparison of the transfection activity of isolated vs. non-isolated TCS formulations. TCS containing DODAC concentrations of 42.5 mol% 30 mol%, 24 mol% and 20 mol % DODAC prepared by phosphate dialysis were injected i.p. at 30 μg DNA into mice 7 days after tumor seeding. Tumors removed from mice 24 hours after administration were assayed for luciferase activity. This study demonstrates that these TCS formulations transfect *in vivo* as well as *in vitro.* In addition, removal of the empty liposomes by sucrose density gradient isolation results in increased transfection activity.

**Figure 26.** The effect of DODAC TCS concentration on the transfection of mouse spleens after i.p. administration. TCS formulations described in **Figure 19** were injected i.p. at 30 μg DNA into mice 7 days after tumor seeding. Spleens removed from the tumor bearing mice 24 hours after administration were assayed for luciferase activity.

**Figure 27.** The effect of DODAC TCS concentration on the transfection of mouse liver after i.p. administration. TCS formulations described in **Figure 19** were injected i.p. at 30 μg DNA into mice 7 days after tumor seeding. Livers removed from the tumor bearing mice 24 hours after administration were assayed for luciferase activity. The studies described in Figures 21 and 22 demonstrate that it is possible to transfect normal organs as well as tumor tissue after i.p. administration of TCS formulation.

**Figure 28.** TCS toxicity study was carried out by monitoring the plasma aspartate aminotransferase (AST) levels after i.p. administration of TCS containing various concentrations of DODAC. Isolated TCS composed of pINEXL018/DODAC/DOPE/PEG-Cer-C8 prepared by detergent dialysis in citrate buffer were injected i.p. into mice 7 days after tumor seeding. Blood was removed from mice 24 hours after administration and was assayed for AST activity. This study demonstrates that there is little significant tissue or organ damage associated with TCS formulations administered by the i.p. route.

**Figure 29.** Effect of repeated i.v. administration of INEX324-LacZ on serum levels of anti-β-gal antibodies. Mice received three i.v. injections of INEX324-LacZ and the level of antibodies against transgene product was measured using capture ELISA assay.

**Figure 30.** Effect of DNA concentration on the expression of β-galactosidase by BHK-21 cells in vitro. Cells were incubated with either INEX324-luc or INEX324-LacZ for 24 h and expression of β-galactosidase was determined by FACS using the FDG assay.

**Figure 31.** Time course of luciferase expression after i.v. administration of INEX324-luc formulation. Mice were given a single i. v. injection of INEX324-1uc (100 μg DNA) and the expression of luciferase was determined 12 h and 24 h post-injection.

**Figure 32.** Transfection of the spleen after intravenous administration of INEX324-Luc formulation. Mice received three consecutive injections of INEX324-LacZ or INEX324 lipid, followed by a single injection of INEX324-Luc. Spleens were harvested 12 h after INEX324-Luc administration and the levels of luciferase were assayed.

**Figure 33.** Transfection of the liver after intravenous administration of INEX324-Luc formulation. Mice received three consecutive injections of INEX324-LacZ or 324 lipid, followed by single injection of INEX324-Luc. Livers were harvested 12 h after INEX324-Luc administration and the levels of luciferase were assayed.

## DETAILED DESCRIPTION OF THE INVENTION

CONTENTS

I. Glossary
II. General - Plasmid-Lipid Compositions
III. Methods of Forming Plasmid-Lipid Particles
IV. Pharmaceutical Preparations
V. Administration of Plasmid-Lipid Particle Formulations
VI. Example 1
VII. Example 2
VIII. Conclusion

### I. Glossary

The following abbreviations are used herein: DC-Chol, 3β-(N-(N',N'-dimethylaminoethane)carbamoyl)cholesterol *(see,* Gao, *et al., Biochem. Biophys. Res. Comm.,* **179**:280-285 (1991)); DDAB, N,N-distearyl-N,N-dimethylammonium bromide; DMRIE, N-(1,2-dimyristyloxyprop-3-yl)-N,N-dimethyl-N-hydroxyethyl ammonium bromide; DODAC, N,N-dioleyl-N,N-dimethylammonium chloride *(see,* commonly owned patent application USSN 08/316,399, incorporated herein by reference); DOGS, diheptadecylamidoglycyl spermidine; DOPE, 1,2-*sn*-dioleoylphoshatidyethanolamine; DOSPA, N-(1-(2,3-dioleyloxy)propyl)-N-(2-(sperminecarboxamido)ethyl)-N,N-dimethylammonium trifluoroacetate; DOTAP, N-(1-(2,3-dioleyloxy)propyl)-N,N,N-trimethylammonium chloride; DOTMA, N-(1-(2,3-dioleyloxy)propyl)-N,N,N-trimethylammonium chloride; EPC, egg phosphatidylcholine; RT, room temperature; HEPES, 4-(2-hydroxyethyl)-1-piperazineethanesulfonic acid; HBS, HEPES buffered saline (150 mM NaCl and 20 mM HEPES); PEG-Cer-$C_{20}$, 1-O-(2'-(ω-methoxypolyethyleneglycol)succinoyl)-2-N-arachidoyl-sphingosine; PEG-Cer-$C_{14}$, 1-O-(2'-(ω-methoxypolyethyleneglycol)succinoyl)-2-N-myristoyl-sphingosine; PBS, phosphate-buffered saline; EGTA, ethylenebis(oxyethylenenitrilo)-tetraacetic acid; OGP, n-octyl β-D-glycopyranoside (Sigma Chemical Co., St. Louis, MO); POPC, palmitoyl oleoyl phosphatidylcholine (Northern Lipids, Vancouver, BC); QELS, quasielastic light scattering; TBE, 89 mM Tris-borate with 2 mM EDTA; and EDTA, Ethylenediaminetetraacetic acid (Fisher Scientific, Fair Lawn, NJ);

The term "acyl" refers to a radical produced from an organic acid by removal of the hydroxyl group. Examples of acyl radicals include acetyl, pentanoyl, palmitoyl, stearoyl, myristoyl, caproyl and oleoyl.

The term "lipid" refers to any fatty acid derivative which is capable of forming a bilayer such that a hydrophobic portion of the lipid material orients toward the bilayer while a hydrophilic portion orients toward the aqueous phase. Hydrophilic characteristics derive from the presence of phosphato, carboxylic, sulfato, amino, sulfhydryl, nitro, and other like groups. Hydrophobicity could be conferred by the inclusion of groups that include, but are not limited to, long chain saturated and unsaturated aliphatic hydrocarbon groups and such groups substituted by one or more aromatic, cycloaliphatic or heterocyclic group(s). Preferred lipids are phosphoglycerides and sphingolipids, representative examples of which include phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine or dilinoleoylphosphatidylcholine could be used. Other compounds lacking in phosphorus, such as sphingolipid and glycosphingolipid families are also within the group designated as lipid. Additionally, the amphipathic lipids described above may be mixed with other lipids including triglycerides and sterols.

The term "cationic lipid" refers to any of a number of lipid species which carry a net positive charge at physiological pH or have a protonatable group and are positively charged at pH lower than the $pK_a$. Such lipids include, but are not limited to, DODAC, DOTMA, DOGS, DDAB, DOTAP, DC-Chol, DMRIE and amino lipids. The term "amino lipids" is meant to include lipids with an amino head group (including alkylamino or dialkylamino group) which is protonated to form a cationic lipid below its $pK_a$. Commercial preparations of cationic liposomes prepared from cationic lipids are generally not useful unless the liposomes are first disrupted to provide lipid mixtures. The compositions and methods described herein use lipids and lipid mixtures in a self-assembling process which occurs in the presence of a plasmid or other nucleic acid.

The terms "transfection" and "transformation" are used herein interchangeably, and refer to the introduction of polyanionic materials, particularly nucleic acids and plasmids, into cells. The term "lipofection" refers to the introduction of such materials using liposome or lipid-based complexes. The polyanionic materials can be in the form of DNA or RNA which is linked to expression vectors to facilitate gene expression after entry into the cell. The plasmids used in the present invention include DNA having coding sequences for structural proteins, receptors and hormones, as well as transcriptional and translational regulatory elements (*i.e.*, promoters, enhancers, terminators and signal sequences) and vectors. Methods of incorporating particular nucleic acids into expression vectors are well known to those of skill in the art, but are described in detail in, for example, Sambrook, *et al., Molecular Cloning: A Laboratory Manual* (2nd ed.), Vols. 1-3, Cold Spring Harbor Laboratory, (1989) or *Current Protocols in Molecular Biology,* F. Ausubel, *et al.,* ed. Greene Publishing and Wiley-Interscience, New York (1987), both of which are incorporated herein by reference.

The term "contacting" is used herein interchangeably with the following: combined with, added to, mixed with, passed over, incubated with, flowed over, *etc.* Moreover, the compounds of present invention can be "administered" by any conventional method such as, for example, parenteral, oral, topical and inhalation routes as described herein. "An amount sufficient," "an effective amount," or "therapeutically effective amount" refer to an amount of a compound or composition effective to depress, suppress or regress cell growth or result in amelioration of symptoms associated with cancerous diseases. The desired result can be either a subjective relief of a symptom(s) or an objectively identifiable improvement in the recipient of the dosage, a decrease in tumor size, a decrease in the rate of growth of cancer cells as noted by the clinician or other qualified observer.

The terms "treating cancer," "therapy," and the like refer generally to any improvement in the mammal having the cancer wherein the improvement can be ascribed to treatment with the compounds and compositions of the present invention. The improvement can be either subjective or objective. For example, if the mammal is human, the patient may note improved vigor or vitality or decreased pain as subjective symptoms of improvement or response to therapy. Alternatively, the clinician may notice decrease in tumor size or tumor burden based on physical exam, laboratory parameters, tumor markers or radiographic findings. Some laboratory signs that the clinician may observe for response to therapy include normalization of tests such as white blood cell count, red blood cell count, platelet count, erythrocyte sedimentation rate, and various enzyme levels. Additionally, the clinician may observe a decrease in a detectable tumor marker. Alternatively, other tests can be used to evaluate objective improvement such as sonograms, nuclear magnetic resonance testing and positron emissions testing.

"Inhibiting the growth of tumor cells" can be evaluated by any accepted method of measuring whether growth of the tumor cells has been slowed or diminished. This includes direct observation and indirect evaluation such as subjective symptoms or objective signs as discussed above.

"Expression vectors," "cloning vectors," or "vectors" are often plasmids or other nucleic acid molecules that are able to replicate in a chosen host cell. Expression vectors may replicate autonomously, or they may replicate by being inserted into the genome of the host cell, by methods well known in the art. Vectors that replicate autonomously will have an origin of replication or autonomous replicating sequence (ARS) that is functional in the chosen host cell(s). Often, it is desirable for a vector to be usable in more than one host cell, *e.g.,* in *E. coli* for cloning and construction, and in a mammalian cell for expression.

## II. General - Plasmid-Lipid Compositions

The present invention derives from the discovery that nucleic acid (*e.g*, plasmid) can be encapsulated in lipid bilayer carriers in an amount significantly above that which has been previously demonstrated. In particular, the present invention provides lipid-plasmid compositions in which nucleic acids are encapsulated in self-assembling lipid vesicles in an amount of from about 5 μg to about 800 μg per milligram of lipid, preferably in an amount of from about 40 μg to about 400 μg per milligram of lipid and, more preferably, in an amount of from about 100 μg to about 400 μg per milligram of lipid. Additionally, the nucleic acid-lipid compositions which are described herein, form in a self-assembling process to yield particles having a narrow distribution of sizes (<i>e.g</i>., 50 nm to about 150 nm). The precise size of the compositions formed will depend on several factors including, for example, the choice of lipids and the size of the nucleic acid that is encapsulated. However, the size distribution is relatively narrow and is achieved without harsh sizing steps such as, for example, extrusion or sonication. Still further, the compositions described herein are nuclease resistant and can be concentrated in an aqueous solution without the formation of aggregate complexes. As used herein, the term "nuclease resistant" when used to describe a nucleic acid-lipid composition refers to a composition in which the nucleic acid portion is less than about 30% degraded, more preferably, less than about 15% degraded and, even more preferably, less than about 5 % degraded when the composition is incubated with 0.1 to 10 U and, more preferably, 1 U of a nuclease (<i>e.g</i>., DNAse or normal serum) after 30 minutes at 37°C.

More particularly, it has now been discovered that encapsulation efficiency in detergent dialysis methods is dependent on the lipid composition as well as the dialysis buffer which is used in forming the lipid bilayer carriers. Optimal lipid bilayer carriers can now be constructed depending on the encapsulate (*<i>e.g</i>.,* plasmid, antisense, ribozyme or other polyanionic therapeutic agent), the environment for transfection (*<i>e.g</i>.,* diagnostics or *in vivo* or *in vitro* transfection) and other factors such as desired circulation lifetimes and fusogenic properties. Accordingly, particular lipid compositions can now be selected to exhibit certain circulation and targeting characteristics and formulated by control of salt concentrations to increase the amounts of plasmid or other nucleic acid which are encapsulated.

The unique detergent dialysis method by which the present compositions are prepared yields DNA to lipid ratios in excess of 20 μg DNA to 1 mg lipid. In some embodiments, the lipid coated DNA particle will have DNA to lipid ratios in excess of 200 μg DNA to 1 mg lipid.

Plasmids which are useful for the instant compositions are typically nucleotide polymers which are to be administered to a subject for the purpose of repairing or enhancing the expression of a cellular protein. Accordingly, the nucleotide polymers can be polymers of nucleic acids including genomic DNA, cDNA, or mRNA. Still further, the plasmids can encode promoter regions, operator regions, structural regions, *etc.* The plasmids are preferably double-stranded DNA or DNA-RNA hybrids. Examples of double-stranded DNA include, but are not limited to, structural genes, genes including operator control and termination regions, and self-replicating systems such as plasmid DNA.

Multiple genetic sequences can also be used in the present compositions. Thus, the sequences for different proteins can be located on one strand or plasmid. Promoter, enhancer, stress or chemically-regulated promoters, antibiotic-sensitive or nutrient-sensitive regions, as well as therapeutic protein encoding sequences, can be included as required. Nonencoding sequences can be also be present to the extent they are necessary to achieve appropriate expression.

Plasmids used in the present method can be isolated from natural sources, obtained from such sources as ATCC or GenBank libraries or prepared by synthetic methods. The compositions of the present invention can be prepared from plasmids of essentially any size. In preferred embodiments, the plasmid is from about 2 kilobases to about 15 kilobases, more preferably from about 4 kilobases to about 10 kilobases.

In some embodiments, the plasmid will be replaced with other nucleic acids (<i>e.g</i>., single-stranded DNA or RNA, antisense, ribozymes or nucleic acids). When nucleic acids other than plasmids are used, the nucleic acids can contain nucleic acid analogs, for example, the antisense derivatives described in a review by Stein*, et al., Science* **261:** 1004-1011 (1993) and in U.S. Patent Nos. 5,264,423 and 5,276,019, the disclosures of which are incorporated herein by reference.

Single-stranded nucleic acids include antisense oligonucleotides (complementary to DNA and RNA), ribozymes and triplex-forming oligonucleotides. In order to have prolonged activity, the single-stranded nucleic acids will preferably have some or all of the nucleotide linkages substituted with stable, nonphosphodiester linkages, including, for example, phosphorothioate, phosphorodithioate, phophoroselenate, or O-alkyl phosphotriester linkages.

The nucleic acids used in the present invention will also include those nucleic acids in which modifications have been made in one or more sugar moieties and/or in one or more of the pyrimidine or purine bases. Examples of sugar modifications include replacement of one or more hydroxyl groups with halogens, alkyl groups, amines, azido groups or functionalized as ethers or esters. Additionally, the entire sugar can be replaced with sterically and electronically similar structures, including aza-sugars and carbocyclic sugar analogs. Modifications in the purine or pyrimidine base moiety include, for example, alkylated purines and pyrimidines, acylated purines or pyrimidines, or other heterocyclic substitutes known to those of skill in the art.

Synthetic nucleic acids can be prepared by a variety of solution or solid phase methods. Generally, solid phase synthesis is preferred. Detailed descriptions of the procedures for solid phase synthesis of nucleic acids by phosphite-triester, phosphotriester, and H-phosphonate chemistries are widely available. *See,* for example, Itakura, U.S. Pat. No. 4,401,796; Caruthers, *et al.,* U.S. Pat. Nos. 4,458,066 and 4,500,707; Beaucage, *et al., Tetrahedron Lett.,* **22:**1859-1862 (1981); Matteucci, *et al., J. Am. Chem. Soc.,* **103:**3185-3191 (1981); Caruthers, *et al., Genetic Engineering,* **4:**1-17 (1982); Jones, chapter 2, Atkinson, *et al.,* chapter 3, and Sproat, *et al.,* chapter 4, in *Oligonucleotide Synthesis: A Practical Approach,* Gait (ed.), IRL Press, Washington D.C. (1984); Froehler, *et al., Tetrahedron Lett.,* **27:**469-472 (1986); Froehler, *et al., Nucleic Acids Res.,* **14**:5399-5407 (1986); Sinha, *et al. Tetrahedron Lett. ,* **24:**5843-5846 (1983);

and Sinha, *et al., Nucl. Acids Res.,* **12:**4539-4557 (1984) which are incorporated herein by reference.

Lipids which are useful in the present invention can be any of a variety of lipids including both neutral lipids and charged lipids. Carrier systems having desirable properties can be prepared using appropriate combinations of lipids, targeting groups and circulation enhancers. Additionally, the compositions provided herein can be in the form of liposomes or lipid particles, preferably lipid particles. As used herein, the term "lipid particle" refers to a lipid bilayer carrier which "coats" a nucleic acid and has little or no aqueous interior. More particularly, the term is used to describe a self-assembling lipid bilayer carrier in which a portion of the interior layer comprises cationic lipids which form ionic bonds or ion-pairs with negative charges on the nucleic acid (<i>e.g</i>., a plasmid phosphodiester backbone). The interior layer can also comprise neutral or fusogenic lipids and, in some embodiments, negatively charged lipids. The outer layer of the particle will typically comprise mixtures of lipids oriented in a tail-to-tail fashion (as in liposomes) with the hydrophobic tails of the interior layer. The polar head groups present on the lipids of the outer layer will form the external surface of the particle.

Selection of suitable lipids for use with plasmids will typically involve consideration of the lipid's bilayer-forming capabilities, bilayer-stabilizing capabilities and fusogenic properties. The capabilities, or properties, of a lipid can often be estimated based on the physical shape of the lipid. For example, lipids can be classified according to the three basic structures which lipids can form *(see,* Figure 1). Lipids which form micelles typically have large headgroup cross-sectional areas in relation to that of the lipid tail or hydrophobic region. Examples of these lipids are detergents, such as n-octyl β-D-glycopyranoside (OGP), and lysolipids, such as lysophosphatidylcholine (lysoPC). Bilayer-forming lipids or bilayer-stabilizing lipids are typically those which are cylindrical in shape ( *<i>e.g</i>*., DOPC, DOPS, and DODAC). Lipids which form an inverted micelle (the precursor to the hexagonal II phase) have larger tails than heads (*<i>e.g</i>*., DOPE, *see*, Figure 2). Inverted micelles cannot exist in aqueous solution so they must be solubilized in the membrane and form long tube structures called hexagonal II phase ($H_{II}$ phase). The $H_{II}$ phase is thought to be a precursor to fusion of two adjacent membranes. For this reason, DOPE is a powerful membrane fusogen (otherwise referred to as a fusogenic lipid).

Fusogenic lipids such as DOPE, lysolipids and free fatty acids can be accommodated in a bilayer configuration with the appropriate quantities of bilayer-forming lipids. For example, about 20 mol% DOPC will stabilize DOPE in a bilayer. Alternatively, about 30% DODAC (a less effective bilayer-forming lipid) will stabilize DOPE in a bilayer, while only about 10% or perhaps less of PEG-Ceramide is necessary to stabilize DOPE in a bilayer. Similarly, non-micelle forming lipids can be stabilized within micelles with the appropriate quantities of micelle forming lipids such as detergents (*<i>e.g</i>.,* OGP). As the detergent is removed by dialysis, the micelle becomes unstable and becomes a bilayer if enough bilayer-forming lipid is present (Figure 3). Similarly, a bilayer stabilized by PEG-Ceramide will become unstable once the PEG-Ceramide exchanges out of the outer monolayer.

Considering lipid properties, as noted above, the compositions of the present invention are optimized for the delivery of nucleic acid (*<i>e.g</i>*., plasmids) to cells. In particular, lipid-plasmid compositions are provided in which the carriers (lipid portions) are composed of at least two types of lipids including (i) fusogenic, nonbilayer forming lipids, and (ii) bilayer-forming or -stabilizing lipids. Preferably, the compositions will further comprise (iii) an aggregation-preventing agent (*<i>e.g</i>*., PEG-lipids, ATTA-lipids, gangliosides, *etc.*). In particularly preferred embodiments, the compositions will comprise cationic lipids (as the bilayer-forming or -stabilizing lipids), fusogenic lipids and PEG-lipids.

Cationic lipids which are useful in the present compositions include, for example, DODAC, DOTMA, DDAB, DOTAP, DC-Chol and DMRIE. These lipids and related analogs, which are also useful in the present invention, have been described in co-pending USSN 08/316,399; U.S. Patent Nos. 5,208,036, 5,264,618, 5,279,833 and 5,283,185, the disclosures of which are incorporated herein by reference. Particularly preferred within this group is DODAC.

Fusogenic lipids which are useful in the present invention include, for example, DOPE, lysolipids and free fatty acids. Each of these lipids (or lipid groups) can be accommodated in a bilayer configuration with an appropriate quantity of bilayer-forming or bilayer-stabilizing lipids (*<i>e.g</i>*., DOPC, DODAC, ATTA-lipids, PEG-lipids, such as PEG-Ceramides, *etc.*). Preferably, the fusogenic lipid is DOPE, or a related phosphatidylethanolamine having two attached fatty acyl chains, preferably unsaturated fatty acyl chains.

Preferably, the lipid-nucleic acid compositions of the present invention contain an aggregation-preventing agent, *<i>i.e</i>*., a compound (or mixture of compounds) that prevents aggregation during formulation of the lipid-nucleic acid compositions. In addition, such aggregation-preventing agents can also serve as cloaking agents, which help to reduce elimination of the lipid-nucleic acid compositions by the host immune system. These agents can also be targeting agents that help the lipid-nucleic acid formulations to accumulate in the area of the disease or target site. These agents can also be compounds that improve features of the formulation, such as leakiness, longevity in circulation, reduction in toxicity, encapsulation efficiency, *<i>etc</i>.* Examples of suitable aggregation-preventing agents, include but are not limited to, ATTA-lipid conjugates, such as those disclosed in U.S. Patent Application Ser. No. 08/996,783, filed December 23, 1997, and U.S. Patent Application Ser. No. 60/073,852, February 2, 1998; PEG-lipid conjugates, such as those disclosed in U.S. Patent Application Serial Nos. 08/486214, 08/316407, and 08/485608; and gangliosides, *<i>e.g</i>.,* $G_{M1}$, such as those disclosed in U.S. Patent No. 4,837,028, the teachings of all of which are incorporated herein by reference. Examples of these components and others that can usefully be included in the formulations of the invention are known to and used by those skilled in the art.

In a preferred embodiment, PEG-modified lipids are incorporated into the compositions of the present invention as the aggregation-preventing agent. The use of a PEG-modified lipid positions bulky PEG groups on the surface of the liposome or lipid carrier and prevents binding of DNA to the outside of the carrier (thereby inhibiting cross-linking and aggregation of the lipid carrier). The use of a PEG-ceramide is often preferred and has the additional advantages of stabilizing membrane bilayers and lengthening circulation lifetimes. Additionally, PEG-ceramides can be prepared with different lipid tail lengths to control the lifetime of the PEG-ceramide in the lipid bilayer. In this manner, "programmable" release can be accomplished which results in the control of lipid carrier fusion. For example, PEG-ceramides having $C_{20}$-acyl groups attached to the ceramide moiety will diffuse out of a lipid bilayer carrier with a half-life of 22 hours (*see,* Figure 4). PEG-ceramides having $C_{14}$- and $C_8$-acyl groups will diffuse out of the same carrier with half-lives of 10 minutes and less than 1 minute, respectively. As a result, selection of lipid tail length provides a composition in which the bilayer becomes destabilized (and thus fusogenic) at a known rate. Though less preferred, other PEG-lipids or lipid-polyoxyethylene conjugates are useful in the present compositions. Examples of suitable PEG-modified lipids include PEG-modified phosphatidylethanolamine and phosphatidic acid, PEG-modified diacylglycerols and dialkylglycerols, PEG-modified dialkylamines and PEG-modified 1,2-diacyloxypropan-3-amines. Particularly preferred are PEG-ceramide conjugates (*<i>e.g</i>.,* PEG-Cer-$C_8$, PEG-Cer-$C_{14}$ or PEG-Cer-$C_{20}$) which are described in co-pending USSN 08/486,214, now U.S. Patent No. 5,820,873, incorporated herein by reference.

In one group of particularly preferred embodiments, the compositions comprise a nucleic acid (*<i>e.g</i>.,* plasmid), DODAC, DOPE and an aggregation-preventing agent (*<i>e.g</i>*., ATTA-lipids, PEG-lipids, such as PEG-Ceramides), more preferably with the plasmid being encapsulated in an amount of from about 30 μg to about 400 μg per milligram of lipid. Still further preferred are those embodiments in which DODAC is present in an amount of from about 5 mol % to about 50 mol%, DOPE is present in an amount of from about 30 mol % to about 70 mol%, and the aggregation-preventing agent (*<i>e.g</i>.,* PEG-Ceramide) is present in an amount of about 5 mol % to about 20 mol%.

The compositions of the present invention can be prepared by the methods described below to provide compositions which are about 50 nm to about 100 nm in size. One of skill in the art will understand that the size of the compositions can be larger or smaller depending of the size of the plasmid which is encapsulated. Thus, for larger plasmids, the size distribution will typically be from about 80 nm to about 180 nm and, more preferably, from about 50 nm to about 150 nm and, more preferably from about 50 nm to about 90 nm. Additionally, the methods described below result in encapsulation of about 40 % to 80 % of the plasmids in solution. Surprisingly, compositions having the above properties can be prepared by a detergent dialysis method via manipulation of the salt concentration present in the formulation mixture.

**III. Methods of Encapsulating Nucleic Acids in a Lipid Bilayer Carrier**

In another aspect, the present invention provides methods for the encapsulation of nucleic acids, preferably plasmids, in a lipid bilayer carrier. The plasmids or nucleic acids present in the compositions formed by these methods exhibit preferably less than about 30 % degradation, more preferably, less than about 15 % degradation and, even more preferably, less than about 5 % degradation when subjected to standard nucleases, such as DNase or normal serum nuclease.

The methods for encapsulating a nucleic acid or plasmid in a lipid bilayer carrier, comprise:

(a) combining the nucleic acid, *<i>i.e</i>*., antisense, ribozyme or plasmid, with a lipid-detergent mixture, the lipid-detergent mixture comprising a lipid mixture of an aggregation-preventing agent (*<i>e.g</i>*. , a PEG-ceramide) in an amount of about 5 mol % to about 20 mol%, cationic lipids in an amount of about 0.5 mol % to about 50 mol % , and neutral or, alternatively, fusogenic lipids in an amount of from about 30 mol % to about 70 mol % and a detergent, to provide a nucleic acid-lipid-detergent mixture; and

(b) dialyzing the nucleic acid-lipid-detergent mixture against a buffered salt solution to remove the detergent and to encapsulate the nucleic acid in a lipid bilayer carrier. In these methods, the salt concentration of the buffered salt solution is adjusted depending on the cationic lipid concentration in the lipid mixture to encapsulate from about 40 % to about 80 % of the nucleic acid.

In one group of embodiments, the methods further comprise the following step:

(c) removing substantially all of the unencapsulated nucleic acids to provide a purified lipid-bilayer-nucleic acid composition having from about 20 μg to about 400 μg of nucleic acid per about 1 mg of lipid.

The plasmids, antisense, ribozyme or nucleic acids, cationic lipids, fusogenic lipids and aggregation-preventing agent (*<i>e.g</i>*., ATTA-lipids, PEG-lipids, *<i>etc</i>*.) that are useful in the present invention are those which have been described above. In preferred embodiments, the amount of cationic lipid is from about 5 mol % to about 50 mol % by weight, more preferably about 10 mol % to about 40 mol % by weight, and most preferably about 20 mol % to about 40 mol % by weight. In a preferred embodiment, the amount of cationic lipids is 10 mol%, 11 mol%, 12 mol%, 13 mol%, 14 mol%, 15 mol%. 16 mol%, 17 mol%, 18 mol%, 19 mol%, 20 mol%, 21 mol%, 22 mol%, 23 mol%, 24 mol%, 25 mol%, 26 mol%, 27 mol%, 28 mol%, 29 mol%, 30 mol%, 31 mol%, 32 mol%, 33 mol%, 34 mol%, 35 mol%, 36 mol%" 37 mol%, 38 mol%, 39 mol%, 40 mol%, 41 mol%, 42 mol%, 43 mol%. 44 mol%, 45 mol%, 46 mol%, 47 mol%, 48 mol%, 49 mol% or 50 mol%.

Similarly, the amount of aggregation-preventing agent (<i>e.g</i>., PEG-Lipid) can vary from about 1 mol % to about 25 mol % and, more preferably, from about 5 mol % to about 20 mol%. For instance, the amount of PEG-ceramide can preferably vary from about 5 mol % to about 20 mol % , depending on the nature of the PEG-ceramide (*<i>e.g</i>.,* PEG-Cer-$C_8$, PEG-Cer-$C_{14}$ or PEG-Cer-$C_{20}$), or the combination of PEG-ceramides used. Selection of the amounts of each can provide compositions in which the fusogenic properties are programmable (*<i>i.e</i>.,* become fusogenic within a predetermined timeframe, depending on the rate at which the PEG-ceramide diffuses out of the composition).

A nucleic acid-lipid-detergent mixture is formed by combining the nucleic acid or plasmid with a lipid-detergent mixture. The lipid-detergent mixture is a combination of aggregation-preventing agent (<i>e.g</i>., ATTA-modified lipids, PEG-modified lipids, such as PEG-ceramides), cationic lipids, neutral or fusogenic lipids and a detergent. The detergent is preferably an aqueous solution of a neutral detergent having a critical micelle concentration of 15-300 mM and, more preferably, 20-50 mM. Examples of suitable detergents include, for example, N,N'-((octanoylimino)-bis-(trimethylene))-bis-(D-gluconamide) (BIGCHAP); BRIJ 35; Deoxy-BIGCHAP; dodecylpoly(ethylene glycol) ether; Tween 20; Tween 40; Tween 60; Tween 80; Tween 85; Mega 8; Mega 9; Zwittergent® 3-08; Zwittergent® 3-10; Triton X-405; hexyl-, heptyl-, octyl- and nonyl-β-D-glucopyranoside; and heptylthioglucopyranoside; with octyl β-D-glucopyranoside being the most preferred. The concentration of detergent in the detergent solution is typically about 100 mM to about 2 M, preferably about 200 mM to about 1.5 M.

The lipid-detergent mixture and nucleic acids typically be combined to produce a charge ratio (+/-) of about 1: 1 to about 20:1, preferably in a ratio of about 3:1 to about 15:1. Additionally, the overall concentration of nucleic acid in solution will typically be from about 25 μg/mL to about 1 mg/mL, preferably from about 25-500 μg/mL and, more preferably, from about 100-300 μg/mL. The combination of nucleic acids and lipids in detergent solution is kept, typically at room temperature, for a period of time which is sufficient for complete mixing to occur. While not intending to be bound by any particular theory, it is believed that coated complexes form in which the negative charges of the nucleic acid are paired with positively charged lipids. Excess lipids complete the formation of a bilayer surrounding and encapsulating the nucleic acids. In other embodiments, the nucleic acids and lipid-detergent mixture can be combined and warmed to temperatures of up to about 37°C. For those embodiments in which temperature-sensitive plasmids are used, the mixtures or coated complexes can be formed at lower temperatures, typically down to about 4°C.

The resulting nucleic acid-lipid-detergent mixture is then subjected to dialysis against a buffered salt solution to remove the detergent from the mixture. The removal of the detergent results in the completed formation of a lipid-bilayer which surrounds the nucleic acids or plasmid providing serum-stable nucleic acid-lipid particles which have a size of from about 50 nm to about 150 nm. The particles thus formed do not aggregate.

The buffered salt solution which is used in the dialysis step will typically be a solution of alkali or alkaline earth halides (*<i>e.g</i>.,* NaCl, KCl, and the like), phosphates (*<i>e.g</i>.,* sodium or potassium phosphate), citrates (*<i>e.g</i>.,* sodium citrate) or combinations thereof. The buffer which is used will typically be HEPES or an equivalent buffer. In particularly preferred embodiments, the buffered salt solution is a HEPES-buffered NaCl solution.

Changes in the salt concentration of the dialysis buffer requires significant changes in the lipid composition for efficient encapsulation in the above process. More particularly, the encapsulation efficiency, particle size and the amount of cationic lipid which is used to achieve optimum loading is altered upon changing the salt concentration in the dialysis buffer.

For example, a dialysis buffer containing 150 mM NaCl provides optimal loading of a plasmid in compositions of about 6 mol% DODAC (by weight). By adding citrate to the dialysis buffer, the amounts of DODAC which are used to achieve optimal encapsulation of a plasmid are increased, while maintaining a narrow distribution of particle sizes.

Still higher levels of DODAC (or other suitable cationic lipids) can be used and provide high levels of plasmid encapsulation (typically greater than 30 % encapsulation with plasmid/lipid ratios of > 20 μg plasmid/mg lipid). For example, a dialysis buffer of 150 mM NaCl and 150 mM sodium phosphate is useful for compositions of about 40-45 mol% DODAC.

Once the plasmid-lipid compositions have formed (typically as particles), any unencapsulated plasmid or empty liposomes can be removed by ion-exchange chromatography or gel filtration, respectively, and any empty liposomes can be removed by density gradient centrifugation using techniques which are well known in the art.

## IV. Pharmaceutical Preparations

The nucleic acid (*<i>e.g</i>*., plasmid)-lipid compositions of the present invention can be administered either alone or in mixture with a physiologically-acceptable carrier (such as physiological saline or phosphate buffer) selected in accordance with the route of administration and standard pharmaceutical practice.

Pharmaceutical compositions comprising the nucleic acid (*<i>e.g</i>.,* plasmid)-lipid compositions (*<i>e.g</i>.,* in particle or liposome form) of the invention are prepared according to standard techniques and further comprise a pharmaceutically acceptable carrier. Generally, normal saline will be employed as the pharmaceutically acceptable carrier. Other suitable carriers include, *<i>e.g</i>.,* water, buffered water, 0.4 % saline, 0.3 % glycine, and the like, including glycoproteins for enhanced stability, such as albumin, lipoprotein, globulin, *<i>etc</i>.* These compositions may be sterilized by conventional, well known sterilization techniques. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, *<i>etc</i>*. Additionally, the particle suspension may include lipid-protective agents which protect lipids against free-radical and lipid-peroxidative damages on storage. Lipophilic free-radical quenchers, such as alphatocopherol and water-soluble iron-specific chelators, such as ferrioxamine, are suitable.

The concentration of particles in the pharmaceutical formulations can vary widely, <i>i.e</i>., from less than about 0.05 %, usually at or at least about 2-5 % to as much as 10 to 30 % by weight and will be selected primarily by fluid volumes, viscosities, *<i>etc</i>.,* in accordance with the particular mode of administration selected. For example, the concentration may be increased to lower the fluid load associated with treatment. This may be particularly desirable in patients having atherosclerosis-associated congestive heart failure or severe hypertension. Alternatively, particles composed of irritating lipids may be diluted to low concentrations to lessen inflammation at the site of administration. For diagnosis, the amount of particles administered will depend upon the particular label used, the disease state being diagnosed and the judgement of the clinician but will generally be between about 0.01 and about 50 mg per kilogram of body weight, preferably between about 0.1 and about 5 mg/kg of body weight.

As noted above, it is often desirable to include polyethylene glycol (PEG), PEG-lipids (*<i>e.g</i>.,* PEG-ceramides), ATTA-lipids, or ganglioside $G_{M1}$-modified lipids to the particles. Addition of such components prevents particle aggregation and provides a means for increasing circulation lifetime and increasing the delivery of the plasmid-lipid particles to the target tissues. Typically, the concentration of the PEG, PEG-lipids (<i>e.g</i>., PEG-ceramide), ATTA-lipids or $G_{M1}$-modified lipids in the particle will be about 1-25 mol%, preferably about 5-20 mol%.

Overall particle charge is also an important determinant in particle clearance from the blood, with negatively charged complexes being taken up more rapidly by the reticuloendothelial system (Juliano, *Biochem. Biophys. Res. Commun.* **63:**651 (1975)) and thus having shorter half-lives in the bloodstream. Particles with prolonged circulation half-lives are typically desirable for therapeutic and diagnostic uses. For instance, particles which can be maintained from 8, 12, or up to 24 hours in the bloodstream are particularly preferred.

In another example of their use, the nucleic acid-lipid particles can be incorporated into a broad range of topical dosage forms including but not limited to gels, oils, emulsions and the like. For instance, a suspension containing the plasmid-lipid particles can be formulated and administered as topical creams, pastes, ointments, gels, lotions and the like.

The present invention also provides nucleic acid-lipid particles in kit form. The kit will typically be comprised of a container which is compartmentalized for holding the various elements of the kit. The kit will contain the compositions of the present inventions, preferably in dehydrated form, with instructions for their rehydration and administration. In still other embodiments, the particles and/or compositions comprising the particles will have a targeting moiety attached to the surface of the particle. Methods of attaching targeting moieties (<i>e.g</i>., antibodies, proteins) to lipids (such as those used in the present particles) are known to those of skill in the art.

Dosage for the nucleic acid-lipid particle formulations will depend on the ratio of nucleic acid to lipid and the administrating physician's opinion based on age, weight, and condition of the patient.

## V. Administration of Nucleic Acid-Lipid Particle Formulations

The serum-stable nucleic acid-lipid compositions particles of the present invention are useful for the introduction of plasmids into cells. Accordingly, the present invention also provides methods for introducing a plasmid into a cell. The methods are carried out *in vitro* or *in vivo* by first forming the particles or compositions as described above, then contacting the particles with the cells for a period of time sufficient for transfection to occur.

The particles of the present invention can be adsorbed to almost any cell type. Once adsorbed, the particles can either be endocytosed by a portion of the cells, exchange lipids with cell membranes, or fuse with the cells. Transfer or incorporation of the nucleic acid portion of the particle can take place via any one of these pathways. In particular, when fusion takes place, the particle membrane is integrated into the cell membrane and the contents of the particle combine with the intracellular fluid. Contact between the cells and the plasmid-lipid particles, when carried out *in vitro,* will take place in a biologically compatible medium. The concentration of particles can vary widely depending on the particular application, but is generally between about 1 μmol and about 10 mmol. Treatment of the cells with the plasmid-lipid particles will generally be carried out at physiological temperatures (about 37°C) for periods of time of from about 1 to 6 hours, preferably of from about 2 to 4 hours. For *in vitro* applications, the delivery of nucleic acids can be to any cell grown in culture, whether of plant or animal origin, vertebrate or invertebrate, and of any tissue or type. In preferred embodiments, the cells will be animal cells, more preferably mammalian cells, and most preferably human cells.

In one group of preferred embodiments, a nucleic acid-lipid particle suspension is added to 60-80% confluent plated cells having a cell density of from about $10^3$ to about $10^5$ cells/mL, more preferably about $2 \times 10^4$ cells/mL. The concentration of the suspension added to the cells is preferably of from about 0.01 to 0.2 μg/mL, more preferably about 0.1 μg/mL.

Typical applications include using well known transfection procedures to provide intracellular delivery of DNA or mRNA sequences which code for therapeutically useful polypeptides. However, the compositions can also be used for the delivery of the expressed gene product or protein itself. In this manner, therapy is provided for genetic diseases by supplying deficient or absent gene products (*<i>i.e</i>.,* for Duchenne's dystrophy, *see,* Kunkel, *et al., Brit. Med. Bull.* **45(3)**:630-643 (1989), and for cystic fibrosis, *see,* Goodfellow, *Nature,* **341:**102-103 (1989)). Other uses for the compositions of the present invention include introduction of antisense oligonucleotides in cells *(see,* Bennett, *et al., Mol. Pharm.,* **41**:1023-1033 (1992)).

Alternatively, the compositions of the present invention can also be used for the transfection of cells *in vivo,* using methods which are known to those of skill in the art. In particular, *Zhu, et al., Science,* **261**:209-211 (1993), incorporated herein by reference, describes the intravenous delivery of cytomegalovirus (CMV)-chloramphenicol acetyltransferase (CAT) expression plasmid using DOTMA-DOPE complexes. Hyde*, et al., Nature,* **362:**250-256 (1993), incorporated herein by reference, describes the delivery of the cystic fibrosis transmembrane conductance regulator (CFTR) gene to epithelia of the airway and to alveoli in the lung of mice, using liposomes. Brigham, *et al., Am. J. Med. Sci. ,* **298:**278-281 (1989), incorporated herein by reference, describes the *in vivo* transfection of lungs of mice with a functioning prokaryotic gene encoding the intracellular enzyme, chloramphenicol acetyltransferase (CAT).

For *in vivo* administration, the pharmaceutical compositions are preferably administered parenterally, i. e. , intraarticularly, intravenously, intraperitoneally, subcutaneously, or intramuscularly. More preferably, the pharmaceutical compositions are administered intravenously or intraperitoneally by a bolus injection. For example, *see,* Stadler, *et al.,* U.S. Patent No. 5,286,634, which is incorporated herein by reference. Intracellular nucleic acid delivery has also been discussed in *Straubringer, et al.,* METHODS IN ENZYMOLOGY, Academic Press, New York, **101:**512-527 (1983); Mannino, *et al., Biotechniques,* **6:**682-690 (1988); Nicolau, *et al., Crit. Rev. Ther. Drug Carrier Syst.,* **6:**239-271 (1989), and Behr, *Acc. Chem. Res.,* **26:**274-278 (1993). Still other methods of administering lipid-based therapeutics are described in, for example, Rahman, *et al.,* U.S. Patent No. 3,993,754; Sears, U.S. Patent No. 4,145,410; *Papahadjopoulos, et al.,* U.S. Patent No. 4,235,871; Schneider, U.S. Patent No. 4,224,179; *Lenk, et al.,* U.S. Patent No. 4,522,803; and *Fountain, et al.,* U.S. Patent No. 4,588,578.

In other methods, the pharmaceutical preparations may be contacted with the target tissue by direct application of the preparation to the tissue. The application may be made by topical, "open" or "closed" procedures. By "topical", it is meant the direct application of the pharmaceutical preparation to a tissue exposed to the environment, such as the skin, oropharynx, external auditory canal, and the like. "Open" procedures are those procedures which include incising the skin of a patient and directly visualizing the underlying tissue to which the pharmaceutical preparations are applied. This is generally accomplished by a surgical procedure, such as a thoracotomy to access the lungs, abdominal laparotomy to access abdominal viscera, or other direct surgical approach to the target tissue. "Closed" procedures are invasive procedures in which the internal target tissues are not directly visualized, but accessed via inserting instruments through small wounds in the skin. For example, the preparations may be administered to the peritoneum by needle lavage. Likewise, the pharmaceutical preparations may be administered to the meninges or spinal cord by infusion during a lumbar puncture followed by appropriate positioning of the patient as commonly practiced for spinal anesthesia or metrazamide imaging of the spinal cord. Alternatively, the preparations may be administered through endoscopic devices.

The nucleic acid-lipid particles can also be administered in an aerosol inhaled into the lungs (*see, Brigham, et al., Am. J. Sci.,* **298(4)**:278-281 (1989)) or by direct injection at the site of disease (Culver, HUMAN GENE THERAPY, MaryAnn Liebert, Inc., Publishers, New York. pp.70-71 (1994)).

In accordance with the above administration methods, the compositions of the present invention can be used to inhibit tumor cell growth, the method comprising contacting the tumor cell with an effective amount of a lipid-nucleic acid composition of the present invention. Tumor cells include, but are not limited to, lung, colon, breast, ovarian, prostate and hepatic tumor cells as well as squamous cell carcinomas. In a presently preferred embodiment, the tumor cells are present in a mammalian subject. Mammalian subjects include, but are not limited to, humans, laboratory animals, domestic pets and farm animals. Preferred hosts include humans, nonhuman primates, dogs, cats, cattle, horses and sheep. In a further preferred embodiment, the above method further comprises the step of observing for a reduction in the growth of the tumor cells.

In another embodiment, the present invention provides a method of treating cancer, the method comprising administering to a mammalian subject having cancer a therapeutically effective amount of a lipid-nucleic acid composition of the present invention. The compositions of the present invention are useful for treating a wide variety of cancers. Such cancers include, by way of example and not limitation, carcinomas such as pharynx, colon, rectal, pancreatic, stomach, liver, lung, breast, skin, prostate, ovary, cervical, uterine and bladder cancers; leukemias; lymphomas; gliomas; retinoblastomas; and sarcomas. Moreover, in accordance with the above method, mammalian subjects include, but are not limited to, humans, laboratory animals, domestic pets and farm animals.

Lipid-nucleic acid compositions suitable for use in the methods of the present invention can readily be identified using *in vitro* and *in vivo* screening assays. Such assays may screen for the ability of a particular composition to inhibit tumor cell growth or to abolish tumorigenicity of malignant cells *in vitro* or *in vivo.* For instance, tumor cell lines can be exposed to varying concentrations of a composition of interest, and the viability of the cells can be measured at set time points using the alamar Blue® assay (commercially available from BioSource, International of Camarillo, California). When alamar Blue dye is added to the culture medium, the dye is reduced by cellular mitochondrial enzymes yielding a soluble product with substantially enhanced fluorescence. This fluorescence can be measured with a fluorimeter, whereby the signal is directly proportional to the cell number. Using this information, $IC_{50}$ (concentration of composition lethal to 50 % of a cell culture as compared to a control culture) values for the compositions of interest can be readily be calculated.

As will be appreciated by the skilled artisan, many varieties of tumor cell cultures and cell lines can be used to screen for activity including, but not limited to, MDA MB 231 (breast), MCF-7 (breast), MDA MB 468 (breast), Siha (squamous cell carcinoma), A549 (nonsmall cell lung), HL-60 (leukemia) Ovcar-3 (ovarian), *<i>etc</i>.* Of course, other *in vitro* and/or *in vivo* assays to screen for anti-tumor and/or anti-cancer activity known to and used by the skilled artisan can also be employed to identify effective compositions useful in the methods of the present invention.

## VII. Example 1 - Formulation of Lipid-Nucleic Acid Compositions

### *A. Materials and Methods*

#### 1. Materials

N,N-dioleyl-N,N-dimethyl ammonium chloride (DODAC), monomethoxy polyethylene2000 glycol succinate-(C8:0-ceramide) (PEG-Ceramide-$C_8$), pACN53, pINEXP005, pINEXL002 and pINEX018 plasmids and pL002 (luciferase) plasmid were manufactured and supplied by INEX Pharmaceuticals Corp. Dioleyl-phosphatidylethanolamine (DOPE) was obtained from Northern Lipids (Vancouver, British Columbia, Canada). Picogreen dsDNA quantitation reagent was obtained from Molecular Probes (Eugene, Oregon). Dialysis buffers were prepared from commercially available reagents (HEPES, NaCl, dibasic sodium phosphate, monobasic sodium phosphate, trisodium citrate) by standard methods. Octyl-β-D-glucoside (OGP), Spectrapor dialysis tubing and ACS or higher grade reagents were obtained from VWR Scientific, Fisher Scientific or Sigma Chemical Company.

#### 2. Method of Formulation

A variety of formulations were prepared using the procedures outlined below. In a first method, a formulation of plasmid with DOPE: DODAC: PEG-Ceramide-$C_8$ (42.5:42.5:15 mol%) was prepared. In other methods, the PEG-Ceramide was held constant and formulations were prepared by altering the amounts of DODAC present.

#### a. DOPE:DODAC:PEG-Ceramide-$C_8$ (42.5:42.5:15 mol%)

In a preparation containing 5 mg/ml total lipid, the concentration of each lipid at the above mol% quantities are DOPE (1.69 mg/ml), DODAC (1.315 mg/ml) and PEG-Ceramide-C8 (2.005 mg/ml) based on molecular weights calculated at 744, 582 and 2515, respectively. Each of these can be dissolved stock solutions using absolute ethanol, 2:1 chloroform: methanol or 9:1 benzene:methanol. If stock solutions of > 20 mg/ml are required, the latter two solvent mixtures are not suitable. Lipids prepared in benzene:methanol have the added advantage that they can be lyophilized (freeze-dried) to a fluffy powder. A dried film of the above lipids are prepared in glass test-tubes (or in round bottomed flasks when prepared in large scale). The combined lipids are dried under a stream of nitrogen (small scale) or in a rotary evaporator (large scale) followed by incubation *in vacuo* (< 100 microns Hg) for at least 2 hours at room temperature. Alternatively, benzene:methanol solutions may be freeze-dried directly. A 1 M solution of OGP (100 μl) is added to each tube containing dried lipid. The plasmid suspension (typically 200-300 μl at 1 mg/mL in TRIS-EDTA buffer) is added to the lipid film. The suspension is made up to 1.0 ml with dialysis buffer, and the suspension is mixed by vortexing until the lipid film is dissolved and a clear solution is formed. Alternatively, the plasmid may be added after the lipid film is dissolved, if desired. The tube is allowed to stand for approximately 30 minutes at room temperature and then the contents are loaded into prepared dialysis bags. Dialysis conditions are found to vary slightly with the quality and type of the plasmid. However, at this DODAC concentration optimum formulations are obtained with 150 mM $NaPO_4$, pH 7.4 with 150 to 175 mM NaCl. The formulations are dialyzed against 2 changes of 2 L of the appropriate buffer (per 1 to 10 ml of formulation).

**b. Varying DODAC and DOPE amounts**

Lipid mixtures of DODAC/DOPE/PEG-Cer-C8 containing 15 mol % of PEG-Cer-C8 were used for all samples. The amount of DODAC and DOPE were varied to reach desired mole% concentrations. For formulations containing greater than 30 mol% DODAC, the total lipid concentration is typically 5 mg/mL. Formulations containing 30 mol % DODAC and less are prepared at 10 mg/mL total lipid. Five or ten mg of lipid mixture of DODAC/DOPE/PEG-Cer-C8 was dissolved in ethanol or organic solvent ($MeOH/CHCl_3$: 1/1). The solvent was removed by gas $N_2$ and then dried under vacuum for at least 3 hrs. The lipid mixture is dried to a film in a glass tube or flask as described above. The detergent suspension is prepared as described above. Where DODAC concentrations are below 42.5 mol% (and DOPE increased correspondingly), the buffer and buffer salt concentrations must be adjusted accordingly to allow optimum encapsulation and the selection of specific buffer concentrations is described in the examples below (*see*, Table 1).

**3. Method of Removing Unencapsulated Plasmid**

In order to remove unencapsulated plasmid DNA and to determine the absolute recovery of encapsulated plasmid the formulations are *cleaned* by running through a short column (typically 3 cm x 1 cm or larger) of DEAE Sepharose which had been pre-equilibrated in either HBS or the respective dialysis buffer for the formulation (*<i>i.e</ i>.,* with 150 mM sodium phosphate (NaPO4), 150 mM NaCl, pH 7.4). After running through the column the preparations are normally dialyzed against HBS and concentrated in the dialysis bag using Aquacide II (Calbiochem) to a desired plasmid concentration.

**4. Method of Determining Percent Encapsulation**

For the determination of percent encapsulation a -/+ Triton X-100 method was used. Typically, aliquots of the formulation taken directly from dialysis were diluted 1:400 in HBS and 2 μL of Picogreen reagent were added to 1 mL of the diluted samples. The fluorescence was measured at 495 nm (excitation) and 525 nm (emission), both in the absence and presence of 10 μL, 10% Triton-X100. The percent encapsulation was calculated as:

$$1 - (\text{Fluorescence} - \text{Triton} / \text{Fluorescence} + \text{Triton}) \times 100.$$

For the determination of absolute plasmid DNA concentration, aliquots of formulation were measured for fluorescence as above in the presence of Triton X100 and compared to standard plasmid concentrations identically prepared.

The following examples are offered solely for the purposes of illustration, and are intended neither to limit nor to define the invention. In each of these examples, the term "DNA" or "plasmid" refers to the plasmid pCMV4-CAT.

**5. Method for Separation of Empty Liposomes from Plasmid Containing Vesicles**

**a. Sucrose Density Gradient Isolation.** Sucrose density gradients are used for the removal of lipid which is not associated with plasmid. The particular gradient used varies with the DODAC concentration as formulations containing the highest DODAC concentrations are the least dense. Gradients are formed by layering decreasing concentrations of HBS-sucrose (W/V) solutions in Beckman (13.2 mL) ultraclear ultracentrifuge tubes above one another. This process is simplified if the solutions are prechilled (at 4°C) to increase the viscosity of the solutions and using a short Pasteur pipette where the tip has been bent upward (done under a gentle flame). Two useful gradients have been employed. For INEX 351 formulations (42.5 mol% DODAC), a gradient of 5% (3 ml):2.5 % (5 ml):1% (2 ml) sucrose (w/v in 20 mM HBS) has been employed. The formulation has been found to settle on the 2.5%-5% sucrose interface. For formulations containing 20-30 mol % DODAC, a gradient containing 10% (2 ml):5 % (2 ml):2.5% (4 ml):1% (2 ml) sucrose has been employed. The plasmic containing TCS has been found to settle on the 5%-10% interface. With either gradient, the formulations are centrifuged at 36,000 rpm in a Beckman SW41 Ti rotor at 25°C for 7-14 hours. A half deceleration speed has not been found to disturb the bands which form. In every case, 3-4 diffuse bands are observed in the upper portion of the gradient, while a narrower band is generally observed at the lower interface of each gradient. The lower band normally contains 90% of the applied DNA and is removed by piercing the centrifuge tube with a needle and removing the band using a syringe (3 ml normally adequate). Excellent separation of the desired band has been accomplished with starting plasmid DNA containing up to 400 μg of plasmid per tube. After removal from the gradient, the formulation is dialyzed against 2 X 21 HBS at 4°C, and the resulting preparation is analyzed for DNA content and particle size.

## *B. Experiments*

### 1. Formulation: Detergent Dialysis

The ionic strength and the counter-ion concentration of the dialysis buffer required for efficient encapsulation of plasmid DNA are critical and there is a direct relationship between the ionic strength and the cationic lipid concentration used in the formulation. Both the ionic strength and the counter-ion concentration can be adjusted by varying the salt concentration and/or the type of salt ions in the buffer. The ionic strength/counter-ion concentration necessary for efficient encapsulation increases with increasing cationic lipid concentration in the formulation. Phosphate and citrate, respectively, are used as counter-ions to compete with the charges on the polynucleotide for interaction with the cationic charge on the lipid headgroup. When the ionic strength (salt concentration) is too high, it results in vesicle formation with little or no encapsulation, and when it is below the optimum concentration, there is formation of lipid/DNA complexes and of aggregates. An indication for aggregation is a wide size distribution (high polydispersity) of the particles/vesicles formed (*see*, Figure 5). Therefore, the optimum salt concentration needs to be determined for each desired cationic lipid concentration in the formulation. The optimum salt concentration required in the buffer for efficient encapsulation with different concentrations of cationic lipid is summarized in Table 1. Examples for two salt combinations, NaCl with citrate and NaCl with phosphate, are illustrated (Figures 5 through 9). Figure 5 shows the effect of the NaCl concentration in the citrate/NaCl buffer on the encapsulation efficiency at a given cationic lipid concentration. Figure 6 shows the relationship between the cationic lipid concentration (DODAC) and the citrate concentration in the buffer to obtain efficient encapsulation. NaCl/citrate was suitable for formulations with DODAC concentration of up to 30 mol%, while NaCl/phosphate could be used over the entire DODAC concentration range tested.

**Table 1. Characterization of representative large scale TCS formulations.**

| DODAC concentration | Buffer | Encapsulation efficiency | Nicomp particle size (nm)[a] |
|---|---|---|---|
| 7% | 0.0 mM citrate, 150 mM NaCl, | 80.5% | 37±18 |
| 17.1% | 40.0 mM citrate, 150 mM NaCl, | 38.5% | 39±20 |
| 22.2 % | 70.0 mM citrate, 150 mM NaCl, | 53.5% | 43 ±22 |
| 32.0% | 105 mM citrate, 150 mM NaCl, | 51.0% | 53±35 |
| | Phosphate | | |
| 20% | 105 mM $NaPO_4$ | 63.% | 178 |
| 24% | 130 mM $NaPO_4$ | 50.7% | 250 |
| 30% | 150 mM $NaPO_4$ | 56.8% | 109 |
| 42.5% | 150 mM $NaPO_4$, 130 mM NaCl | 49% | 131 |

[a] Nicomp analysis of mean particle size, gaussian dist., volume weighting, before DEAE cleaning and isolation

Figures 7 through 11 represent examples where NaCl/phosphate buffers are applied. The effect of a small change in DODAC (cationic lipid) concentration on the encapsulation efficiency is shown in Figure 7. The % encapsulation as a function of NaCl concentration is presented in Figure 8 for four different DODAC concentrations. The NaCl concentration required increased with increasing DODAC concentration. Similarly, the phosphate concentration was adjusted to obtain good encapsulation at different DODAC concentrations (*see*, Figure 9). An additional effect on the encapsulation efficiency was observed by the lipid and polynucleotide concentration, respectively *(see,* Figures 9, 10 and 11).

The particles/vesicles containing polynucleotides can be separated from empty vesicles by density gradient centrifugation. DNA containing particles accumulate at a higher sucrose concentration (<i>i.e</i>., lower band) than the empty vesicles (<i>i.e</i>., upper two bands) *(see,* Figures 12 and 13). Figure 13 shows the results of lipid and DNA analysis of the different gradient fractions, with DNA exclusively in the lower band. The size distribution of the particles/vesicles in the lower band is very narrow (small number for polydispersity, $\chi^2$) and the mean diameter increases slightly with increasing DODAC concentration from about 65 to 94 nm *(see,* Table 2). The particles isolated from formulations with different DODAC concentrations had a similar lipid/DNA ratio (*see*, Table 3). The homogeneous size distribution of the isolated particles was also seen by electron microscopy *(see,* Figure 14).

**Table 2. Effect of Isolation on Particle Size Parameters**

| Particle Size Parameters | | | | |
|---|---|---|---|---|
| | Before Isolation | | After Isolation | |
| Formulation % DODAC | Mean diameter (nm) | $\chi^2$ | Mean diameter (nm) | $\chi^2$ |
| Citrate | | | | |
| 7% | 37±18 | 0.92 | 101±11 | 0.36 |
| 17.1% | 39±20 | 2.7 | 96±20 | 0.2 |
| 22.2% | 43±22 | 2.6 | 92±25 | 0.1 |
| 32.0% | 53±35 | 23 | 114±57 | 1.7 |
| | | | ± | |
| Phosphate | | | | |
| 20% | 178 | 22.9 | 64.0 | 0.3 |
| 24% | 250 | 78.6 | 77.2 | 0.2 |
| 30% | 109 | 1.77 | 89.3 | 0.18 |
| 42.5% | 131 | 2.96 | 93.8 | 0.31 |

**Table 3. Lipid/DNA ratio of TCS after isolation.**

| | Total Lipid/DNA ratio (mg/mg) | |
|---|---|---|
| Mol % DODAC | Before Sucrose Gradient Isolation | After Sucrose Gradient Isolation |
| Citrate | | |
| 17.1% | 30.3 | 15.9 |
| 22.2% | 45.0 | 14.0 |
| 23.0% | 56.4 | 16.2 |
| Phosphate | | |
| 20 | 48.8 | 14.8 |
| 24 | n.d. | 16.6 |
| 30 | 66.3 | 18.5 |
| 42.5 | 53.2 | 13.5 |
| n.d. - not determined | | |

The DNA inside the particles was largely protected from serum nucleases and DNase *(see,* Figures 15 and 16). Formulations were incubated in either DNase or serum, and then separated by gel chromatography. Figure 15 illustrates the separation profile from Sepharose CL-4B gel filtration chromatography for free plasmid DNA after serum incubation, and for a formulation with 21 mol % DODAC before sucrose density gradient isolation *(see,* Figure 15A) and after the isolation *(see,* Figure 15B). DNA cleaved by nucleases eluted in fractions > 7, while the encapsulated intact DNA eluted together with the lipids in the exclusion volume fractions 4-7. The integrity of the encapsulated DNA was characterized further by electrophoresis and, as illustrated in Figure 16, the plasmid remained intact.

**2. Transfection: In Vitro**

The transfection activity and toxicity of formulations containing various concentrations of the cationic lipid DODAC were tested *in vitro* in COS-7 and Hep-G2 cells *(see,* Figures 17 through 22). Luciferase plasmid was formulated and expression of luciferase was determined at times indicated. Cell viability was used as an indication for toxicity. The transfection efficiency was determined as a function of DODAC concentration used in the formulation (*see*, Figures 17 through 22). Furthermore, transfection activity was evaluated as a function of the DNA dose applied (*see*, Figures 19 and 20) and as a function of time *(see,* Figure 21). There is limited transfection activity with formulations containing less than 16-18 mol% DODAC. The best transfection activities are obtained with DODAC concentration in the range of 20-30 mol % in the formulations. The cell viability indicates that toxicity increases significantly with preparations containing >30 mol % DODAC. The toxic effect of the preparation particularly for formulations with high DODAC concentrations can be reduced greatly by removal of the empty vesicles by gradient centrifugation. Furthermore, these isolated preparations showed a significant increase in the transfection activity *(see,* Figure 22).

**3. Transfection: In Vivo**

A murine tumor model was chosen to determine the transfection activity *in vivo.* Mice (C57) were injected by the intra peritoneal (i.p.) route with 100,000 B16 tumor cells. Formulations were administered i.p. on day 7 of B16 tumor growth. After 24 hours (unless indicated otherwise), the animals were sacrificed and the tumors, liver and spleen were analyzed for luciferase expression. The tumor transfection activity was dependent on the concentration of DODAC in the formulation (*see*, Figures 23 and 25). The highest luciferase activity was observed from formulations with 20 to 30 mol% DODAC. Isolated preparations consistently showed increased transfection activity when compared to nonisolated preparations for the same DNA dose (*see*, Figure 25). The luciferase expression in tumors was higher 24 hours after injection than after 48 hours *(see,* Figure 24). Luciferase activity was also observed in liver *(see,* Figure 27) and in spleen *(see,* Figure 26). None of the formulations with the different DODAC concentrations showed significant liver toxicity following i.p. administration as based on the AST levels in the plasma (*see*, Figure 28).

**VII. <u>Example 2 - Immunological Effect of Repeated Injections of Lipid-Nucleic Acid Compositions</u>**

**A. Materials and Methods**

A formulation of plasmid with DOPE:DODAC:PEG-Ceramide-C8 (61:24:15 mol %) (INEX324) was prepared using the methods of Example 1. The formulations were prepared containing pCMVβ and pINEXL018. They were isolated and assayed as described in Example 1.

**1. Administration of INEX324 to Balb/c mice.**

Mice, 12 per group, were injected intravenously (i.v.) with TCS formulations as described below. In this study four injections were given at intervals of seven days over a period of five weeks (Table 4). **Group A** was injected with INEX324 expressing LacZ reporter gene on day 0, 7 and 14 (150 μl per mouse of INEX324-LacZ, 75 μg DNA). Two weeks after the last LacZ injection, mice were injected with INEX324-Luc (150 μl per injection, 80 μg DNA). To evaluate the immunogenicity of INEX324 lipid, **Group B** received three injections of INEX324 empty vesicles (150 μl per mouse) and one injection of INEX324-Luc two weeks later. **Group C** served as a base-line control, mice were given 150 μl of diluent at each time point. Mice from **Group D** received three diluent injections, followed by a single injection of INEX324-Luc, and served as a positive control for luciferase expression. Three mice from each group were sacrificed at 18 hours after each injection, or 24 hours prior to the following injection. Blood was collected and the serum samples were analyzed for the presence of antibodies specific for β-galactosidase using an ELISA assay. Spleens were harvested and splenocytes were processed for various immunological assays: carrier specific, β-gal, LacZ, or mitogen induced clonal expansion and cytokine (IL-2 and IL-4) release. Splenocytes were monitored for expression of differentiation (CD4, CD8, CD22, CD11b) and activation (CD86, MHC-II, Ly-6A/E, CD54 and CD25) markers. The remaining mice from all groups were sacrificed 12 h following the last injection with INEX324-Luc. At this point, the organs (lung, liver and spleen) were fast frozen in liquid nitrogen and assayed for luciferase expression.

**Table 4. Treatment Schedule**

| Group | Formulation | day 0<br>1st injection | Day 7<br>2nd injection | day 14<br>3rd injection | day 28<br>4th injection |
|---|---|---|---|---|---|
| A | 324/LacZ | 324/LacZ | 324/LacZ | 324/LacZ | 324/Luc |
| B | 324 lipid | 324 lipid | 324 lipid | 324 lipid | 324/Luc |
| C | Diluent | Diluent | Diluent | Diluent | Diluent |
| D | Diluent | Diluent | Diluent | Diluent | 324/Luc |

**2 Flow cytometry analysis.**

Splenocytes from all groups were analyzed for expression of differentiation and activation markers. Spleen

cells ($1x10^6$) were stained with appropriate PE-conjugated antibodies (anti-CD22, anti-CD4, anti-CD8, anti-CD11b, anti-CD54, anti-CD86, anti-Ly6A/E and anti-CD25) and phenotypic analysis was performed on a FACSsort flow cytometer (Becton Dickinson, San Jose, CA). Splenocytes were analyzed either after 18 h and 6 days after *in vivo* treatment.

**3. Cell proliferation assay.**

Unseparated spleen cells were tested *in vitro* for their ability to proliferate upon re-stimulation with the empty INEX324 vesicles, INEX324-LacZ formulation, LacZ, transgene product (r-β-galactosidase), or with polyclonal activators for T (Concanavalin A, Con A) and β cells (Lipopolysaccharide, LPS). Single-cell suspensions of lymphocytes were prepared from whole spleens by grinding the spleens using the frosted ends of sterile glass slides in RPMI media containing 10 % FBS. The suspension was allowed to settle standing on ice in a 15 ml polypropylene culture tube. An isolated cell suspension was separated from debris by removing the supernatant and the cell number was quantified using a Coulter counter (Coulter Instruments, Miami, FL). Aliquots of cell suspensions (100 μl, $5x10^6$/ml) in the above media were placed into 96-well plates along with the equal volumes of various appropriate stimuli. Cells were labeled with $^3$H-thymidine for 48 h, and after 3 days incubation, they were harvested. The levels of incorporated radioactivity were measured in a scintillation counter. $^3$H-thymidine incorporation is expressed total $^3$H-incorporation (DPMs), or as a mean percentage (± SD) of media control and plotted versus lipid concentration values.

**4. Measurement of cytokine release.**

Splenocytes ($1x10^6$ cells/ml) were either nonstimulated or cultured in the presence of various concentrations of either empty INEX324 vesicles, or INEX324-LacZ formulation at different times after culture initiation (24 h and 48 h). The levels of cytokine in the cell culture supernatant (Interleukin-2 and Interleukin-4) were determined by an ELISA assay (below).

**5. Cytokine ELISA assays.**

Cytokine-specific ELISA assays were performed using the protocol and specific anti-interleukin antibodies provided by reagent mini-kit (Endogen, Woburn MA). Briefly, Immuno-module (F8-maxisorb) 96-well plates were coated overnight with anti-IL-2, or anti-IL-4 antibody. Plates were washed with PBS-Tween 20 (0.05 %) and blocked with PBS-Tween 20-BSA (2 %) for 1 hour at room temperature. Supernatant samples and standards (diluted in blocking buffer) were added and allowed to incubate overnight. Plates were washed and biotinylated anti-IL-2 or anti-IL-4 antibody was added. After 2 hours incubation, plates were washed and HRP-Extravidin, followed by TMB, was added to each well. Plates were read on a plate reader at $OD_{450nm}$. The amount of released cytokine was determined by comparing the O.D. of test supernatants to a standard curve of serially diluted cytokine standards.

**6. ELISA for detection of β-gal-specific antibodies.**

β-gal specific antibodies in the serum were measured using an ELISA assay. Immuno-module (F8-maxisorb) 96-well plates were coated overnight with r-β-gal (10 μg/ml, 100 μl/well) diluted in bicarbonate buffer (pH 9.6). Plates were washed with PBS-Tween 20 (0.05 %) and blocked with PBS-Tween 20-BSA (1 %) for 30 minutes. Serum samples and standard anti-β-gal IgM and IgG, diluted in blocking buffer, were added to the wells and allowed to incubate overnight. Plates were washed and biotinylated anti-mouse IgG antibody was added. After 2 hours incubation, plates were washed and HRP-Extravidin, followed by TMB substrate, was added to each well. The development of a colored reaction product was quantified on plate reader at $OD_{450}$. The amount of IgG was determined by comparing OD of test serums to a standard curve of serially diluted antibodies as standards.

**7. *In vivo* gene expression.**

Balb/c mice were given 3 intravenous injections of INEX324-LacZ or empty INEX324 vesicles on days 0, 7 and 14. After two additional weeks, mice from all groups (except for the diluent treated group) received one injection of INEX324-Luc (80 μg of DNA) and 12 h later the organs (liver, lung, spleen) were collected and assayed for luciferase activity. A standard assay for the determination of luciferase from the tissue samples was employed. Tissue homogenization was performed using a FastPrep Instrument (FastPrep™ FP120 Instrument, Bio 101) using supplied tubes and beads (FastDNA tubes with MS Matrix). Tissues were homogenized in Cell Culture Lysis Reagent (1xCCLR, Promega) supplemented with BSA (1 mg/ml). FastPrep Instrument settings: speed - 5; time - 8 sec twice. Samples were transferred to new microcentrifuge tubes and briefly centrifuged (2 min., 10,000 rpm) to remove debris. The luciferase assay was performed on luminometer (Dynatech Microlite™ ML3000) using a 96-well microlite plate. A set of purified luciferase standard solutions was prepared (Firefly luciferase) by serially diluting 1 μg/μl luciferase in 1 x CCLR supplemented with BSA (1 mg/ml). For the standard curve, luciferase protein was diluted in a control tissue homogenate to compensate for quenching and 20 μl aliquots (in duplicates) were assayed for each sample/standard. Settings for Luminometer: grade -medium; delay time - 2 sec; integrate time - 10 sec; substrate - 100 μl (Luciferase Assay System, Promega). The results were converted to pg of luciferase protein/g of tissue.

**8. *In vitro* transfection of BHK cells.**

BHK-21 cells were plated at a density of 1x$10^6$ cells (in 10 ml media) per 75 $cm^2$ tissue culture flasks. The following day, when the cells were 60-70 % confluent, the media was aspirated and replaced with 4.8 ml of fresh culture media 2-3 hours prior to transfection. INEX324-LacZ (12.5, 6.2 or 3.1 μg of DNA per 1x$10^6$ cells) was added to the culture media (total volume did not exceeded more than 200 μl) and particles were allowed to remain in contact with the cells for the next 24 hours (at 37°C, 5 % $CO_2$). Separate flasks for appropriate transfection controls: untreated cells, lipid only, plasmid only, and gene unrelated plasmid/particles (<i>i.e</i>., INEX324-Luc) were included. The cell viability and gene expression assays were performed 24 hours post-transfection.

**9. FACS assay for in vitro detection of transgene β-galactosidase.**

Single cell suspensions from transfected cell monolayers were prepared by mechanical dissociation. Dissociated cells were transfered into polystyrene tubes, and stained for the presence of the transgene product, β-galactosidase, using the FDG assay. In this assay, aliquots containing $10^6$ cells were pelleted and re-suspended in 100 μl of staining medium in a 6 ml polystyrene FACS tube and were incubated for 10 min. at 37°C for 10 minutes. The FDG reagent was diluted into 2 ml of distilled water and was pre-warmed to 37°C for no more than 10 minutes. A 100 μl aliquot of the FDG solution was added to each sample. The suspensions were vortexed and incubated an additional minute at 37°C. After incubation, 1.8 ml ice-cold staining medium was added to each tube and the tubes were placed on ice in darkness. Each sample was read within 5 min. after staining using the FACSort flow cytometer.

**B. RESULTS AND DISCUSSION**

**1. Analysis of Differentiation Markers.**

Spleen cells analyzed for the expression of differentiation markers were isolated, stained and assayed either 18 h, or six days following INEX324-LacZ administration. The analysis of differentiation markers 18 h after *in vivo* treatment shows that there are no appreciable differences in the frequency of cells expressing CD22 (B cells) and CD11b (macrophages) markers between the groups (Table 5). The percentage of $CD4^+$ cells and $CD8^+$ cells within the spleen cell population from INEX324-LacZ treated group was approximately 40 % higher compared with diluent or lipid treated group, however, alteration in the ratio of $CD4^+$ and $CD8^+$ was not observed. Spleen cells were processed for the expression of the same set of differentiation markers six days following last injection. Examination of the spleen cell population from INEX324-LacZ treated group revealed changes in phenotypic profile of spleen cells: a moderate decrease in the percentage of T cells (15 %) and a significant 30 % decrease of $CD22^+$ cells. Furthermore, INEX324-LacZ treatment resulted in a five-fold increase of macrophage number as defined by expression of CD11b antigen, compared with diluent treated mice and those that received the empty liposome. It might be that repeated administration of lipid/DNA induces alteration in the pool of splenic cells, evidenced by the decreased number of lymphocytes subsets, T and B cells, and increased the number of macrophages. On the other hand, it is possible that the number of T and B cells is not decreased and these changes are due to the increased spleen cellularity in this group (40 % increase compared with control groups).

A possible explanation for the observed increased spleen cellularity (also observed as splenomegaly) is an infiltration of monocytes into the spleen. In this case, the promoted infiltration of monocytes (and consequently an increased number of CD11b cells), would make the relative contribution of other cell subpopulations within unfractionated spleen samples appear relatively lower (Table 5). In addition, an increased number of macrophages suggests that INEX324 lipid is not toxic. Macrophages have an innate capacity for nonspecific phagocytosis of large quantities of foreign particles such as liposomes. Following internalization and processing of phagocytosed liposomes, their survival depends on the toxicity of the liposomal constituents. In the present study, repeated administration of INEX324 lipid is not accompanied with a decrease in $CD11b^+$ cells, on the contrary, their number is significantly increased and this strongly indicates that INEX324 lipid and INEX324/DNA are not toxic, even following repeated systemic administration.

**Table 5. Expression of differentiation markers by spleen cells treated in vivo**

| | After 18 hours | | | | After 6 days | | | |
|---|---|---|---|---|---|---|---|---|
| **Formulation** | **CD4** | **CD8** | **CD22** | **CD11b** | **CD4** | **CD8** | **CD22** | **CD11b** |
| Diluent | 22.38 | 9.47 | 57.64 | 6.02 | 25.76 | 11.33 | 57.88 | 4.19 |
| INEX324 lipid control | 19.65 | 9.32 | 57.03 | 5.32 | 25.57 | 11.62 | 57.51 | 5.33 |
| INEX324-LacZ | 30.76 | 13.51 | 52.71 | 6.31 | 21.85 | 9.65 | 42.58 | 25.10 |

Expression of differentiation markers is quantified as the percentage of positive cells in the unseparated splenic suspension.

**2. Expression of activation markers.**

The expression of activation markers was analyzed either 18 h, or six days after the last injection *in vivo.* The results show that there was a significant difference in their expression that was both time and treatment dependent (Table 6). The expression of activation markers was up-regulated in spleen cells from animals injected with INEX324-LacZ compared with the untreated and control lipid treated groups 18 h following treatment. As up-regulated expression of activation markers is always associated and positively correlates with the activation of immune cells, these results indicate that only lipid/DNA administration results in activation of spleen cells. However, when splenocytes were analyzed six days following the third injection, expression of activation markers in lipid/DNA treated group, returned to normal level and there was no difference between the groups. These results clearly show that activation is not induced by the repeated administration of the INEX324 TCS. In addition, activation is only detected as a transient increase in mice injected with INEX324-LacZ and is probably associated with the inflammatory properties of DODAC in the INEX324 formulation combined with potential immunogenicity of plasmid DNA.

**Table 6. Expression of activation markers by spleen cells treated in vivo**

| | After 18 hours | | | | After 18 hours After 6 days | | | |
|---|---|---|---|---|---|---|---|---|
| **Formulation** | **CD54** | **CD86** | **MHC-II** | **Ly6A/E** | **CD54** | **CD86** | **MHC-II** | **Ly6A/E** |
| Diluent | 40.14 | 1.27 | 53.97 | 1.49 | 6.18 | 0.76 | 53.16 | 1.20 |
| 324 lipid | 39.87 | 2.14 | 55.83 | 2.38 | 7.06 | 0.99 | 53.03 | 2.13 |
| 324-LacZ | 57.53 | 6.74 | 62.62 | 84.96 | 7.19 | 1.27 | 52.85 | 5.73 |

Expression of activation markers is quantified as the percentage of positive cells in the unseparated splenic suspension.

**3. Influence of repeated administration of INEX324/DNA on cell proliferation.**

Unseparated spleen cells, from all experimental groups, were tested in vitro for their ability to proliferate upon re-stimulation with empty INEX324 vesicles, INEX324-LacZ formulation, LacZ (naked DNA), with transgene product (r-β-galactosidase), or with polyclonal activators for T (Con A) and B (LPS) cells. The immunogenicity of INEX324 lipid and INEX324-LacZ formulation was evaluated using three concentrations of lipid (0.005, 0.01 and 0.02 ng/ml) and three different encapsulated DNA concentrations (0.01, 0.05 and 0.1 μg/ml). At the same time, the mitogenicity of the same formulation was determined in vitro using spleen cells from control mice (diluent treated group). If *in vivo* treatment with control lipid, or with lipid/DNA formulation, had induced an immune stimulation, than a clone of memory cells would be generated, and *in vitro* restimulation of those cells with the same immunogen would result in augmented cell proliferation. The results show that when mice were primed with lipid or with lipid/DNA formulations, secondary responses to the same formulations in vitro were practically undetectable, indicative of minimal immunostimulation (Table 7).

Similar results were obtained upon *in vitro* stimulation of splenocytes from control mice with the same formulations, regardless of the concentrations used for stimulation none of them induced measurable mitogenic response. These results indicate that lipid and lipid/DNA formulations are not immunogenic in vivo and are not mitogenic *in vitro.* Stimulation of splenocytes from lipid/DNA treated group with plasmid DNA (LacZ) induced minimal, but detectable response that was not significantly different compared to those obtained from control and lipid-treated group *in vitro.* Stimulation of splenocytes from all experimental groups with r-β-Gal did not induce any differences in proliferative response that would correspond with the treatment *in vivo.* When splenocytes from all three groups were stimulated *in vitro* with polyclonal activator LPS (mitogen for B-cells and macrophages), again there were no measurable differences in the magnitude of proliferative response corresponding to the *in vivo* treatment (Table 4.). However, repeated administration of INEX324 into Balb/c mice resulted in a moderate (16 %) decrease and INEX324/DNA caused a marked (40 %) decrease in ConA-induced proliferation compared with diluent injected mice. ConA is a T cell mitogen, and this result indicates that repeated administration of INEX324/DNA is accompanied by a down-regulation of the splenic T cell proliferative response. The factors involved in this suppression are not completely understood, but are consistent with the reported results regarding the ability of increased number of $CD11b^+$ cells to inhibit the proliferation of T cells in unseparated spleen cell population (Ostro MJ, TomBD/Six HR Liposomes and Immunobiology, Elsevier NH Inc., p225-239,(1980); Jaffe et al., Molecular Medicine 2, (6) 692-701, (1996)). The INEX324/DNA induced splenomegaly, increased spleen cellullarity, and five-fold increase of macrophage number observed in this study are consistent with this mechanism.

**Table 7. Cell proliferation and cytokine release following in vitro stimulation of spleen cells.**

| ***In vivo* treatment** | **In vitro stimulation** | **$^3$H-incorporation DPMs** | **Cytokine release ($OD_{450}$)** | |
|---|---|---|---|---|
| | | | **IL-2** | **IL-4** |
| Diluent | Diluent | **1674** | **0.127** | **0.151** |
| Diluent | 324 empty vesicles | 9673 | 0.073 | 0.097 |
| Diluent | 324-LacZ | 9051 | 0.089 | 0.090 |
| Diluent | LacZ | 3352 | 0.082 | 0.095 |
| Diluent | r-β-gal | 18336 | 0.059 | 0.103 |
| Diluent | ConA | 215542 | 0.615 | 0.138 |
| Diluent | LPS | 35676 | - | - |
| **324 empty vesicles** | **324 empty vesicles** | **2447** | **0.083** | **0.099** |
| 324 empty vesicles | 324-LacZ | 6375 | 0.089 | 0.081 |
| 324 empty vesicles | LacZ | 3798 | 0.116 | 0.098 |
| 324 empty vesicles | r-β-gal | 16401 | 0.077 | 0.097 |
| 324 empty vesicles | ConA | 182435 | **0.569** | 0.165 |
| **324 empty vesicles** | **LPS** | **33090** | - | - |
| **324-LacZ** | **324-LacZ** | **16959** | **0.103** | **0.076** |
| 324-LacZ | 324 empty vesicles | 9025 | 0.099 | 0.082 |
| 324-LacZ | LacZ | 7520 | 0.134 | 0.089 |
| 324-LacZ | r-β-gal | 13345 | 0.125 | 0.087 |
| 324-LacZ | ConA | 125450 | **0.350** | **0.157** |
| 324-LacZ | LPS | 41639 | - | - |

**4. Influence of repeated administration of INEX324-DNA on cytokine release.**

Splenocytes were nonstimulated, or cultured in the presence of either lipid, or various concentrations of lipid/DNA formulation, DNA, r-β-Gal, or Con A. Culture supernatants were collected at different times after culture initiation (after 24 h or 48 h), and the levels of released cytokines, IL-2 and IL-4, were measured (Table 4). Following *in vivo* treatment, there was no increased release of Th1 and Th2 cytokines from splenocytes of INEX324 and INEX324/DNA treated mice. TCS treated splenocytes produced IL-2 and IL-4 in levels similar to splenocytes from mice that received diluent only. Following in vivo treatment, there was also no difference in cytokine release pattern between control and lipid treated group, compared to group treated with lipid/DNA formulation. In addition, the splenocytes from all experimental groups released similar insignificant amounts of IL-2 and IL-4 after *in vitro* stimulation with LacZ and r-β-gal. In response to stimulation with T-cell mitogen (with a sub-optimal concentration of ConA) however, unfractionated spleen cells obtained from lipid and lipid/DNA treated mice secreted markedly reduced amounts of IL-2. Splenocytes from lipid treated mice produced 25 % less, and from lipid/DNA treated mice 40 % less IL-2 compared with group of mice receiving diluent only (Table 4.). The decreased amounts of IL-2 release correlate with down-regulated ability of T cells to proliferate following stimulation with ConA. These results suggest that various subsets of cells of the immune system might be differentially affected by *in vivo* treatment with INEX324 and INEX324/DNA.

**5. Effect of repeated INEX324/DNA administration on production of β-gal specific antibodies.**

Serum samples were assayed for presence of antibodies against the transgene protein (β-gal) following third injection of lipid/LacZ formulation. Results indicate that *in vivo* treatment with INEX324-LacZ formulations did not elicit measurable amounts of IgM antibodies, following first injection, and IgG antibodies, after three consecutive i.v. injections (Figure 29). The amount of IgM and IgG antibodies assayed on the same level as in a control and lipid treated group. The absence of detectable levels of anti-transgene IgG antibodies suggests that although there is measurable expression of transgene protein, the immune response against the expressed transgene protein has not been elicited.

**6. Gene expression *in vitro.***

Transfectability of the INEX324-LacZ formulation was tested in vitro. BHK cells were transduced *in vitro* using various concentrations (3.1, 6.2 and 12.5 μg per 1x$10^6$ cells) of INEX324-LacZ and 24 hours later the expression of β-gal was assayed by FACS. The FDG assay was developed to evaluate the establishement of stable transformed cell lines (constititively express β-gal protein) and to test the quality of pINEXLacZ plasmids. When complexes (INEX100 series TCS) were used for *in vitro* transfection of various cell lines, in most cases transduced cells were 60-80 % positive for the expression of β-gal protein. pINEXLacZ was encapsulated in 302 and 303 vesicles and used for transfection in *vitro,* but the expression of the transgene protein was not detected, even though expression can be detected *in vivo.* Using INEX324 to encapsulate pINEXLacZ, for the first time we were able to measure gene expression *in vitro.* INEX324 lipid and INEX324-Luc formulation were used as negative controls. Transfection of BHK cells with INEX324-LacZ was very efficient: 85 % of cells transfected with 12.5 μg of DNA and 40 % of cells transfected with 6.2 μg of DNA expressed transgene β-gal protein (Figure 30.).

**7. Gene expression *in vivo***

A small-scale *in vivo* experiment was performed in order to determine the time course and the magnitude of gene expression following systemic (i.v.) administration of INEX324-Luc formulation. Balb/c mice (three per group) were given a single i.v. injection of INEX324-Luc (100 μg of DNA per mouse) and the expression of luciferase was determined either 12 h or 24 h post-injection. The organs (lung, liver and spleen) from both INEX324-Luc injected mice and diluent treated controls, were processed the same time and luciferase expression was evaluated. The results demonstrate that a single administration of INEX324-Luc resulted in significant gene expression in the spleen. Luciferase expression in the liver was measurable, but at a much lower level. No expression was detected in the lung. The time course of transgene expression was highest at 12 hours post injection and is still measurable at lower levels 24 hours post-injection (Figure 31.).

In the next study, Balb/c mice were given 3 intravenous injections of INEX324-LacZ or empty 324 vesicles on day 0, 7 and 14. Two weeks later the administration was repeated with INEX324-Luc (80 μg of DNA per mouse) and 12 h later the organs (liver, lung and spleen) were collected and assayed for luciferase activity. The results show that there was a significant luciferase expression in the spleen from all experimental groups (Figure 32).

Previous *in vivo* treatment with INEX324 empty vesicles or INEX324-LacZ formulation, did not result in decreased gene expression in the spleen, luciferase expression in both groups assayed at levels similar to that from control group. Although the level of gene expression in the liver was much lower, it demonstrates the same pattern as in the spleen confirming that previous *in vivo* treatment did not compromise the ability of TCS to deliver gene of interest (Figure 33.). There was no measurable gene expression in the lungs from any of the groups tested (results not shown).

**VIII. Conclusion**

As discussed above, in accordance with one of its aspects, the present invention provides compositions and methods for preparing serum-stable nucleic acid (*<i>e.g</i>*., plasmid)-lipid particles which are useful for the transfection of cells, both *in vitro* and *in vivo.*

Still further, nucleic acids (<i>e.g</i>., plasmid DNA) can now be formulated using a variety of lipids to provide compositions having extremely high plasmid/lipid ratios. The process can be performed in a predictable manner, by generating theoretical curves for any set of lipids, whereby accurate predictions of the salt concentration necessary to achieve a serum stable formulation can be made.

Still further, nucleic acids (<i>e.g</i>., plasmid DNA) can be formulated using a variety of lipids to provide compositions that can be administered in repeat doses without eliciting an immune response, while still maintaining gene expression.

All publications, patents and patent applications mentioned in this specification are herein incorporated by reference into the specification to the same extent as if each individual publication, patent or patent application was specifically and individually indicated to be incorporated herein by reference.

## Claims

**1.** A method of encapsulating a nucleic acid, preferably a plasmid, in a lipid bilayer carrier, said method comprising:

(a) combining said nucleic acid with a lipid-detergent mixture to provide a nucleic acid-lipid-detergent mixture, said lipid-detergent mixture comprising a detergent and a lipid mixture comprising an aggregation-preventing agent in an amount of about 5 mol% to about 20 mol%, a cationic lipid in an amount of about 15 mol% to about 45 mol%, and a fusogenic lipid;
(b) dialyzing said nucleic acid-lipid-detergent mixture against a buffered salt solution to remove said detergent and to encapsulate said nucleic acid in a lipid bilayer carrier and provide a lipid bilayer-nucleic acid composition, wherein said buffered salt solution has an ionic strength sufficient to encapsulate of from about 40% to about 80% of said nucleic acid;
optionally said method further comprising
(c) removing substantially all of the unencapsulated nucleic acids to provide a purified lipid bilayer-nucleic acid composition having from about 20 $\mu$g to about 400 $\mu$g of nucleic acid per about 1 mg of lipid.

**2.** A method in accordance with claim 1, wherein said detergent is octylglucoside.

**3.** A method in accordance with claim 1 or claim 2, wherein said cationic lipid is DODAC.

**4.** A method in accordance with any one of claims 1 to 3, wherein said aggregation-preventing agent is a member selected from the group consisting of gangliosides, ATTA-lipids and PEG-lipids.

**5.** A method in accordance with claim 4, wherein said aggregation-preventing agent is a PEG-lipid which is a PEG-ceramide, optionally a PEG-ceramide selected from the group consisting of PEG-Cer-$C_8$, PEG-Cer-$C_{14}$ and PEG-Cer-$C_{20}$.

**6.** A method in accordance with any one of claims 1 to 5, wherein said buffered salt solution is HEPES-buffered NaCl solution or is a citrate solution, or wherein said buffered salt solution contains about 150 mM NaCl.

**7.** A method in accordance with any one of claims 1 to 6, wherein about 50% to about 70% of the initial concentration of said nucleic acid becomes encapsulated.

**8.** A method in accordance with any one of claims 1, 2, 6 or 7, wherein said cationic lipid is DODAC, said aggregation-preventing agent is a PEG-ceramide selected from the group consisting of PEG-Cer-$C_8$, PEG-Cer-$C_{14}$ and PEG-Cer-$C_{20}$, said fusogenic lipid comprises DOPE and said buffered salt solution comprises NaCl and sodium phosphate.

**9.** A method in accordance with any one of claims 1 to 8, wherein the lipid bilayer carrier-encapsulated nucleic acid formed has a mean particle diameter of from about 50 nm to about 150 nm; preferably from about 50 nm to about 90 nm, in the absence of extrusion or sonication.

**10.** A lipid-nucleic acid composition, said lipid-nucleic acid composition comprising a nucleic acid encapsulated in a self-assembling lipid vesicle in an amount of from about 20 $\mu$g nucleic acid/mg of lipid to about 400 $\mu$g nucleic acid/mg of lipid, wherein said lipid vesicle comprises cationic lipids and fusogenic lipids, wherein the amount of said cationic lipids is from about 15 mol% to about 45 mol% and wherein the encapsulated nucleic acid is less than about 15% digested after 30 min. at 37°C when said composition is combined with 1 U of a nuclease; optionally wherein the amount of said cationic lipids is from about 20 mol% to about 40 mol%.

**11.** A composition in accordance with claim 10, wherein said lipid vesicle comprises cationic lipid and fusogenic lipids; optionally, wherein said lipid vesicle comprises cationic lipids selected from the group consisting of DODAC, DOTMA, DOGS, DDAB. DOTAP, DC-Chol, DMIRE and aminolipids, fusogenic lipids selected from the group consisting of DOPE, lysolipids, free fatty acids, and PEG-lipids.

**12.** A composition in accordance with claim 10 or claim 11, wherein said lipid vesicle further comprises an aggregation-preventing agent, optionally said aggregation-preventing agent being a member selected from the group consisting of gangliosides. ATTA-lipids and PEG-lipids.

**13.** A composition in accordance with claim 12, wherein said aggregation-preventing agent is a PEG-lipid which is a PEG-ceramide, preferably a PEG-ceramide selected from the group consisting of PEG-Cer-$C_8$, PEG-Cer-$C_{14}$, PEG-Cer-$C_{20}$ and combinations thereof.

**14.** A composition in accordance with claim 13, wherein said lipid vesicle comprises DODAC, DOPE and PEG-Ceramicle, preferably wherein said lipid vesicle comprises DODAC in an amount of from about 15 mol% to about 45 mol%, DOPE in an amount of from about 30 mol% to about 70 mol%, and PEG-Ceramide in an amount from about 5 mol% to about 20 mol%.

**15.** A composition in accordance with claim 13 or claim 14, wherein said lipid vesicle comprises DODAC, DOPE and PEG-Ceramide, and said plasmid is present in an amount of from about 30 μg to about 400 μg per milligram of lipid.

**16.** A composition in accordance with any one of claims 10 to 15, further comprising a targeting moiety.

**17.** An *in vitro* method for introducing a nucleic acid into a cell, preferably a spleen cell, said *in vitro* method comprising:

(a) preparing a lipid-nucleic acid composition according to claim 1: and
(b) contacting said cell with said lipid-nucleic acid composition for a period of time sufficient to introduce said nucleic acid into said cell; and optionally wherein the efficiency of transfection is not diminished by repeat doses administered within 2 weeks.

**18.** The use of a composition of any one of claims 10 to 16, or of a lipid-nucleic acid composition prepared according to claim 1, in the manufacture of a medicament for inhibiting tumour cell growth, preferably in a mammalian subject.

**19.** A composition of any one of claims 10 to 16, or a lipid-nucleic acid composition prepared according to claim 1, for use as a pharmaceutical.

**20.** A pharmaceutical composition, said composition comprising a pharmaceutically acceptable carrier and a lipid-nucleic acid composition prepared according to claim 1 or a composition of any one of claims 10 to 16.

**21.** A method in accordance with claim 1 wherein said cationic lipid is in an amount of from about 20 mol% to about 30 mol%.

**Patentansprüche**

**1.** Verfahren zum Verkapseln einer Nukleinsäure, vorzugweise eines Plasmids, in einen Träger mit einer Lipiddoppelschicht, wobei besagtes Verfahren umfasst:

(a) Kombinieren besagter Nukleinsäure mit einer Lipid-Detergens-Mischung, um eine Nukleinsäure-Lipid-Detergens-Mischung bereitzustellen, wobei besagte Lipid-Detergens-Mischung ein Detergens und eine Lipidmischung umfasst, welche einen aggregationsverhindernden Wirkstoff in einer Menge von etwa 5 mol% bis etwa 20 mol%; ein kationisches Lipid in einer Menge von etwa 15 mol% bis etwa 45 mol% und ein fusogenes Lipid umfasst;
(b) Dialysieren der besagten Nukleinsäure-Lipid-Detergens-Mischung gegen eine gepufferte Salzlösung, um besagtes Detergens zu entfernen und besagte Nukleinsäure in einen Träger mit einer Lipiddoppelschicht zu verkapseln und eine Zusammensetzung aus Lipiddoppelschicht und Nukleinsäure bereitzustellen, wobei besagte gepufferte Salzlösung eine Ionenstärke, die ausreicht, um etwa 40 % bis etwa 80 % der besagten Nukleinsäure zu verkapseln, aufweist;

wobei besagtes Verfahren wahlweise weiter umfasst:

(c) Entfernen von im Wesentlichen allen der unverkapselten Nukleinsäuren, um eine gereinigte Zusammensetzung aus Lipiddoppelschicht und Nukleinsäure, die etwa 20 μg bis etwa 400 μg Nukleinsäure pro etwa 1 mg Lipid aufweist, bereitzustellen.

**2.** Verfahren gemäß Anspruch 1, wobei besagtes Detergens Octylglucosid ist.

**3.** Verfahren gemäß Anspruch 1 oder Anspruch 2, wobei besagtes kationisches Lipid DODAC ist.

**4.** Verfahren gemäß einem der Ansprüche 1 bis 3, wobei besagter aggregationsverhindernder Wirkstoff ein Mitglied ausgewählt aus der Gruppe bestehend aus Gangliosiden, ATTA-Lipiden und PEG-Lipiden ist.

**5.** Verfahren gemäß Anspruch 4, wobei besagter aggregationsverhindernder Wirkstoff ein PEG-Lipid, bei dem es sich um ein PEG-Ceramid, wahlweise um ein PEG-Ceramid, das ausgewählt ist aus der Gruppe bestehend aus PEG-Cer-$C_8$, PEG-Cer-$C_{14}$ und PEG-Cer-$C_{20}$, handelt, ist.

**6.** Verfahren gemäß einem der Ansprüche 1 bis 5, wobei besagte gepufferte Salzlösung HEPES-gepufferte NaCl-Lösung oder eine Citratlösung ist oder wobei besagte gepufferte Salzlösung etwa 150 mM NaCl enthält.

**7.** Verfahren gemäß einem der Ansprüche 1 bis 6, wobei etwa 50 % bis etwa 70 % der Ausgangskonzentration besagter Nukleinsäure verkapselt werden.

**8.** Verfahren gemäß einem der Ansprüche 1, 2, 6 oder 7, wobei besagtes kationisches Lipid DODAC ist, besagter aggregationsverhindernder Wirkstoff ein PEG-Ceramid, das ausgewählt ist aus der Gruppe bestehend aus PEG-Cer-$C_8$, PEG-Cer-$C_{14}$ und PEG-Cer-$C_{20}$, ist, besagtes fusogenes Lipid DOPE umfasst und besagte gepufferte Salzlösung NaCl und Natriumphosphat umfasst.

**9.** Verfahren gemäß einem der Ansprüche 1 bis 8, wobei die gebildete, in einen Träger mit einer Lipiddoppelschicht verkapselte Nukleinsäure ohne Extrusion oder Ultraschallbehandlung einen mittleren Teilchendurchmesser von etwa 50 nm bis etwa 150 nm, vorzugsweise von etwa 50 nm bis etwa 90 nm hat.

**10.** Zusammensetzung aus Lipid und Nukleinsäure, wobei besagte Zusammensetzung aus Lipid und Nukleinsäure eine Nukleinsäure, die in einem sich selbst aufbauenden Lipidvesikel verkapselt ist, in einer Menge von etwa 20 $\mu$g Nukleinsäure /mg Lipid bis etwa 400 $\mu$g Nukleinsäure / mg Lipid umfasst, wobei besagter Lipidvesikel kationische Lipide und fusogene Lipide umfasst, wobei die Menge besagter kationischer Lipide etwa 15 mol% bis etwa 45 mol% beträgt und wobei die verkapselte Nukleinsäure nach 30 Minuten bei 37°C zu weniger als etwa 15 % abgebaut wird, wenn besagte Zusammensetzung mit 1 U einer Nuklease zusammengebracht wird; wobei wahlweise die Menge besagter kationischer Lipide etwa 20 mol% bis etwa 40 mol% beträgt.

**11.** Zusammensetzung gemäß Anspruch 10, wobei besagter Lipidvesikel kationische Lipide und fusogene Lipide umfasst, wobei wahlweise besagter Lipidvesikel kationische Lipide, die ausgewählt sind aus der Gruppe bestehend aus DODAC, DOTMA, DOGS, DDAB, DOTAP, DC-Chol, DMIRE und Aminolipiden, fusogene Lipide, die ausgewählt sind aus der Gruppe bestehend aus DOPE, Lysolipiden, freien Fettsäuren, und PEG-Lipide umfasst.

**12.** Zusammensetzung gemäß Anspruch 10 oder Anspruch 11, wobei besagter Lipidvesikel weiter einen aggregationsverhindernden Wirkstoff umfasst, wobei besagter aggregationsverhindernder Wirkstoff wahlweise ein Mitglied ausgewählt aus der Gruppe bestehend aus Gangliosiden, ATTA-Lipiden und PEG-Lipiden ist.

**13.** Zusammensetzung gemäß Anspruch 12, wobei besagter aggregationsverhindernder Wirkstoff ein PEG-Lipid, bei dem es sich um ein PEG-Ceramid, vorzugsweise um ein PEG-Ceramid, das ausgewählt ist aus der Gruppe bestehend aus PEG-Cer-$C_8$, PEG-Cer-$C_{14}$, PEG-Cer-$C_{20}$ und Kombinationen davon, handelt, ist.

**14.** Zusammensetzung gemäß Anspruch 13, wobei besagter Lipidvesikel DODAC, DOPE und PEG-Ceramid umfasst, wobei besagter Lipidvesikel vorzugsweise DODAC in einer Menge von etwa 15 mol% bis etwa 45 mol%, DOPE in einer Menge von etwa 30 mol% bis etwa 70 mol% und PEG-Ceramid in einer Menge von etwa 5 mol% bis etwa 20 mol% umfasst.

**15.** Zusammensetzung gemäß Anspruch 13 oder Anspruch 14, wobei besagter Lipidvesikel DODAC, DOPE und PEG-Ceramid umfasst und besagtes Plasmid in einer Menge von etwa 30 $\mu$g bis etwa 400 $\mu$g pro Milligramm Lipid vorliegt.

**16.** Zusammensetzung gemäß einem der Ansprüche 10 bis 15, die weiter eine Komponente umfasst, um die Zusammensetzung an ein Ziel zu bringen.

**17.** *In-vitro*-Verfahren zum Einbringen einer Nukleinsäure in eine Zelle, vorzugsweise eine Milzzelle, wobei besagtes *in-vitro*-Verfahren umfasst:

(a) Herstellen einer Zusammensetzung aus Lipid und Nukleinsäure gemäß Anspruch 1 und

(b) In-Kontakt-Bringen besagter Zelle mit besagter Zusammensetzung aus Lipid und Nukleinsäure für eine Zeitdauer, die ausreicht, um besagte Nukleinsäure in besagte Zelle einzubringen; und wobei wahlweise die Transfektionseffizienz durch wiederholte Dosen, die innerhalb von zwei Wochen verabreicht werden, nicht vermindert wird.

**18.** Verwendung einer Zusammensetzung nach einem der Ansprüche 10 bis 16 oder einer Zusammensetzung aus Lipid und Nukleinsäure, die gemäß Anspruch 1 hergestellt wurde, zur Herstellung eines Medikaments zum Hemmen von Tumorzellwachstum, vorzugsweise in einem Säuger.

**19.** Zusammensetzung nach einem der Ansprüche 10 bis 16 oder Zusammensetzung aus Lipid und Nukleinsäure, die gemäß Anspruch 1 hergestellt wurde, zur Verwendung als Arzneimittel.

**20.** Pharmazeutische Zusammensetzung, wobei besagte Zusammensetzung einen pharmazeutisch unbedenklichen Träger und eine Zusammensetzung aus Lipid und Nukleinsäure, die gemäß Anspruch 1 hergestellt wurde, oder eine Zusammensetzung nach einem der Ansprüche 10 bis 16 umfasst.

**21.** Verfahren gemäß Anspruch 1, wobei besagtes kationisches Lipid in einer Menge von etwa 20 mol% bis etwa 30 mol% vorliegt.

**Revendications**

**1.** Une méthode permettant d'encapsuler un acide nucléique, de préférence un plasmide, dans un transporteur lipidique en double couche, ladite méthode comportant :

(a) la combinaison dudit acide nucléique avec un mélange d'un lipide et d'un détergent afin de fournir un mélange acide nucléique-lipide-détergent, ledit mélange lipide-détergent comportant un détergent et un mélange lipidique lequel comporte un agent prévenant l'agrégation selon une quantité environ entre 15 mol% et environ 45 mol%, et un lipide fusogénique ;
(b) la dialyse dudit mélange acide nucléique-lipide-détergent contre une solution tampon d'un sel afin de retirer ledit détergent et d'encapsuler ledit acide nucléique dans une couche double lipidique et de fournir une composition lipide en double couche - acide nucléique, dans laquelle ladite solution tamponnée d'un sel possède un pouvoir ionisant suffisant pour encapsuler environ entre 40% et environ 80% dudit acide nucléique ;
optionnellement ladite méthode comprend de plus
(c) le retrait substantiel de tous les acides nucléiques encapsulés pour fournir une composition purifiée bicouche lipidique - acide nucléique ayant environ entre 20 $\mu$g et environ 400 $\mu$g d'acide nucléique par environ 1 mg de lipide.

**2.** Une méthode en accord avec la revendication 1, dans laquelle ledit détergent est un octylglucoside.

**3.** Une méthode en accord avec la revendication 1 ou la revendication 2, dans laquelle ledit lipide cationique est DODAC.

**4.** Une méthode en accord avec l'une quelconque des revendications 1 à 3, dans laquelle ledit agent de prévention d'agrégation est un membre choisi parmi le groupe constitué de gangliosides, lipides ATTA et lipides PEG.

**5.** Un méthode en accord avec la revendication 4, dans laquelle ledit agent de prévention d'agrégation est un lipide PEG qui est un céramide PEG, optionnellement un céramide PEG choisi parmi le groupe constitué du PEG-Cer-$C_8$, PEG-Cer-$C_{14}$ et PEG-Cer-$C_{20}$.

**6.** Une méthode en accord avec l'une quelconque des revendications de 1 à 5, dans laquelle ladite solution tampon de sel est une solution tampon - HEPES de NaCl ou une solution de citrate, ou dans laquelle ladite solution tampon de sel contient environ 150 mM de NaCl.

**7.** Une méthode en accord avec l'une quelconque des revendications de 1 à 6, dans laquelle entre environ 50% et environ 70% de la concentration initiale dudit acide nucléique devient encapsulé.

**8.** Une méthode en accord avec l'une quelconque des revendications 1, 2, 6, ou 7, dans laquelle ledit lipide cationique est du DODAC, ledit agent de prévention d'agrégation est du PEG-céramide choisi à partir du groupe constitué du

PEG-Cer-$C_8$, PEG-Cer-$C_{14}$ et PEG-Cer-$C_{20}$, ledit lipide fusogénique comprend du DOPE et ladite solution tampon de sel comprenant le NaCl et le phosphate de sodium.

**9.** Une méthode en accord avec l'une quelconque des revendications de 1 à 8, dans laquelle le diamètre moyen particulaire du transporteur en double couche lipidiques encapsulant l'acide nucléique ainsi formé est environ entre 50 nm à environ 150 nm; préférentiellement environ entre 50 nm et environ 90 nm, en l'absence d'expulsion ou de sonification.

**10.** Une composition acide nucléique - lipide, ladite composition acide nucléique - lipide comporte un acide nucléique encapsulé dans une vésicule lipidique auto-formée selon une quantité comprise entre environ 20 μg d'acide nucléique/mg de lipide et environ 400 μg d'acide nucléique/mg de lipide, dans laquelle la quantité desdits lipides cationiques est comprise entre environ 15 mol% et environ 45 mol% et dans laquelle celle de l'acide nucléique encapsulé est inférieur à environ 15% digéré après 30 min. à 37°C lorsque ladite composition est combinée avec 1 U de nucléase ; optionnellement dans laquelle la quantité desdits lipides cationiques est entre environ 20 mol% et environ 40 mol%.

**11.** Une composition en accord avec la revendication 10, dans laquelle ladite vésicule lipidique comporte des lipide cationiques et fusogéniques ; optionnellement dans laquelle, ladite vésicule lipidique comporte les lipides cationiques choisis parmi le groupe constitué des DODAC, DOTMA, DOGS, DDAB, DOTAP, DC-Chol ; DMIRE et aminolipides, les lipides fusogéniques choisis parmi le groupe constitué des DOPE, lysolipides, acides gras libres, et lipides PEG.

**12.** Une composition en accord avec la revendication 10 ou la revendication 11, dans laquelle ladite vésicule lipidique comprend de plus un agent de prévention d'agrégation, optionnellement ledit agent de prévention d'agrégation étant un membre choisi parmi le groupe constitué des gangliosides, lipides-ATTA et lipides-PEG

**13.** Une composition en accord avec la revendication 12, dans laquelle ledit agent de prévention est un lipide PEG qui est un PEG-céramide, préférentiellement un PEG-céramide choisi parmi le groupe constitué de PEG-Cer-$C_8$, PEG-Cer-$C_{14}$, PEG-Cer-$C_{20}$, et les combinaisons de ceux-ci.

**14.** Une composition en accord avec la revendication 13, dans laquelle ladite vésicule lipidique comporte du DODAC, DOPE et PEG-Céramide, dans laquelle préférentiellement, ladite vésicule lipidique comporte du DODAC selon une quantité comprise entre environ 15 mol% et environ 45 mol%, du DOPE selon une quantité comprise entre environ 30 mol% et environ 70 mol%, et du PEG-Céramide selon une quantité comprise entre environ 5 mol% et environ 20 mol%.

**15.** Une composition en accord avec la revendication 13 ou la revendication 14, dans laquelle ladite vésicule lipidique comporte du DODAC, DOPE et PEG-Céramide, et ledit plasmide est présent selon une quantité comprise entre environ 30 μg et environ 400 μg par milligramme de lipide.

**16.** Une composition en accord avec l'une quelconque des revendications 10 à 15, qui de plus comporte un groupement caractéristique cible.

**17.** Une méthode *in vitro* pour introduire un acide nucléique à l'intérieur d'une cellule, préférentiellement une cellule splénique, ladite méthode *in vitro* comportant :

(a) la préparation d'une composition lipide - acide nucléique en accord avec la revendication 1 et
(b) la mise en contact de ladite cellule avec ladite composition lipide-acide nucléique pour une période de temps suffisante pour introduire ledit acide nucléique à l'intérieur de la cellule ; et optionnellement dans laquelle l'efficacité de la transfection n'est pas diminuée par l'administration répétée de doses au cours de deux semaines.

**18.** L'utilisation d'une composition de l'une quelconque des revendications de 10 à 16, ou d'une composition lipide - acide nucléique préparée selon la revendication 1, dans la fabrication d'un médicament pour l'inhibition de la croissance de cellules tumorales, préférentiellement chez des sujets mammifères.

**19.** Une composition de l'une quelconque des revendications de 10 à 16, ou d'une composition lipide - acide nucléique préparée selon la revendication 1, pour une utilisation comme produit pharmaceutique.

**20.** Une composition pharmaceutique, ladite composition comportant un transporteur acceptable sur le plan pharmaceutique et une composition lipide - acide nucléique préparée selon la revendication 1 ou une composition de l'une quelconque des revendications de 10 à 16.

**21.** Une méthode en accord avec la revendication 1 dans laquelle ledit lipide cationique est d'une quantité comprise entre environ 20 mol% et environ 30 mol%,

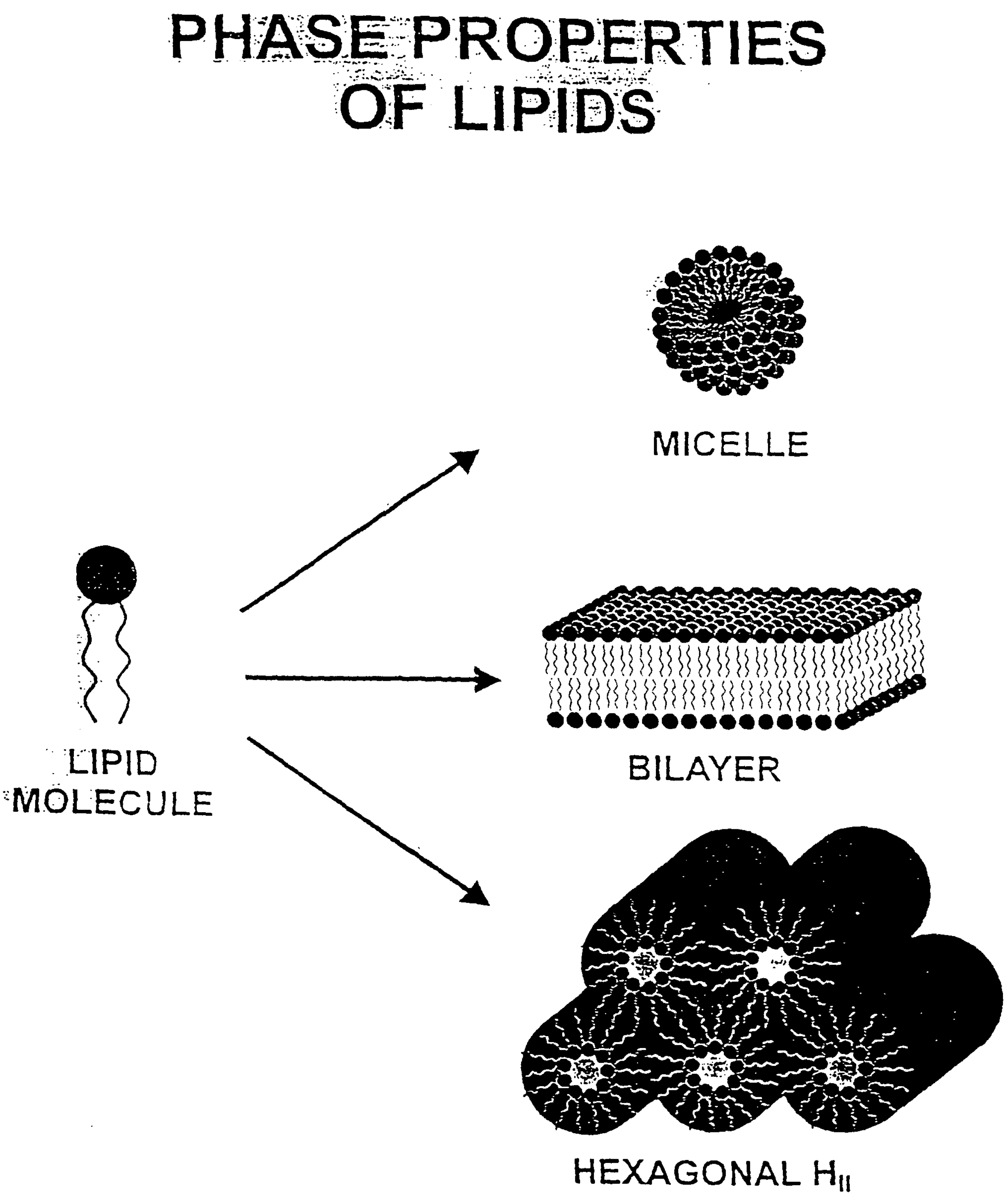
PHASE PROPERTIES
OF LIPIDS
MICELLE
LIPID
MOLECULE
BILAYER
HEXAGONAL $H_{II}$

FIG. 1

# FUSOGENIC LIPIDS

STRUCTURE

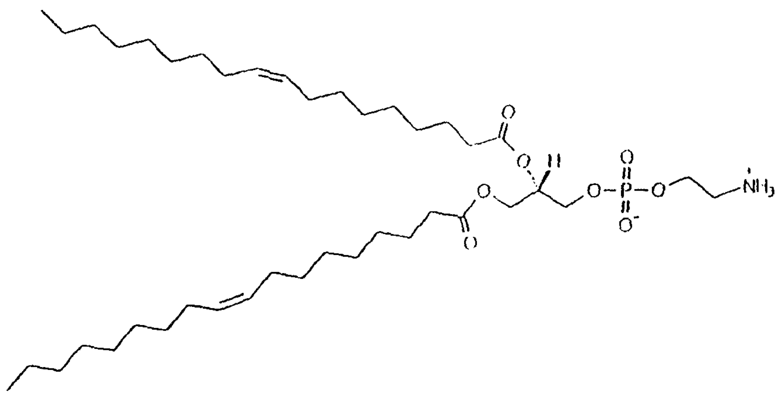

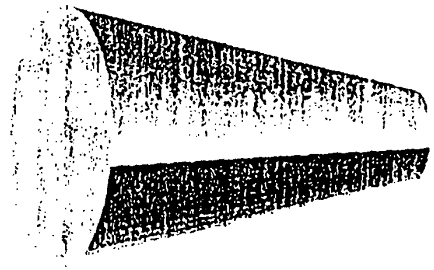

MECHANISM

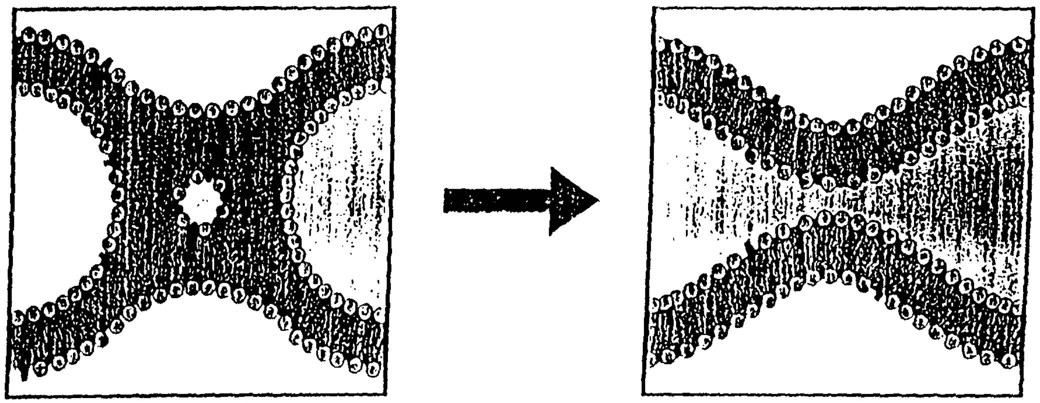

FIG. 2

# DETERGENT DIALYSIS PROCEDURE FOR ENTRAPPING DNA IN FUSION TCS

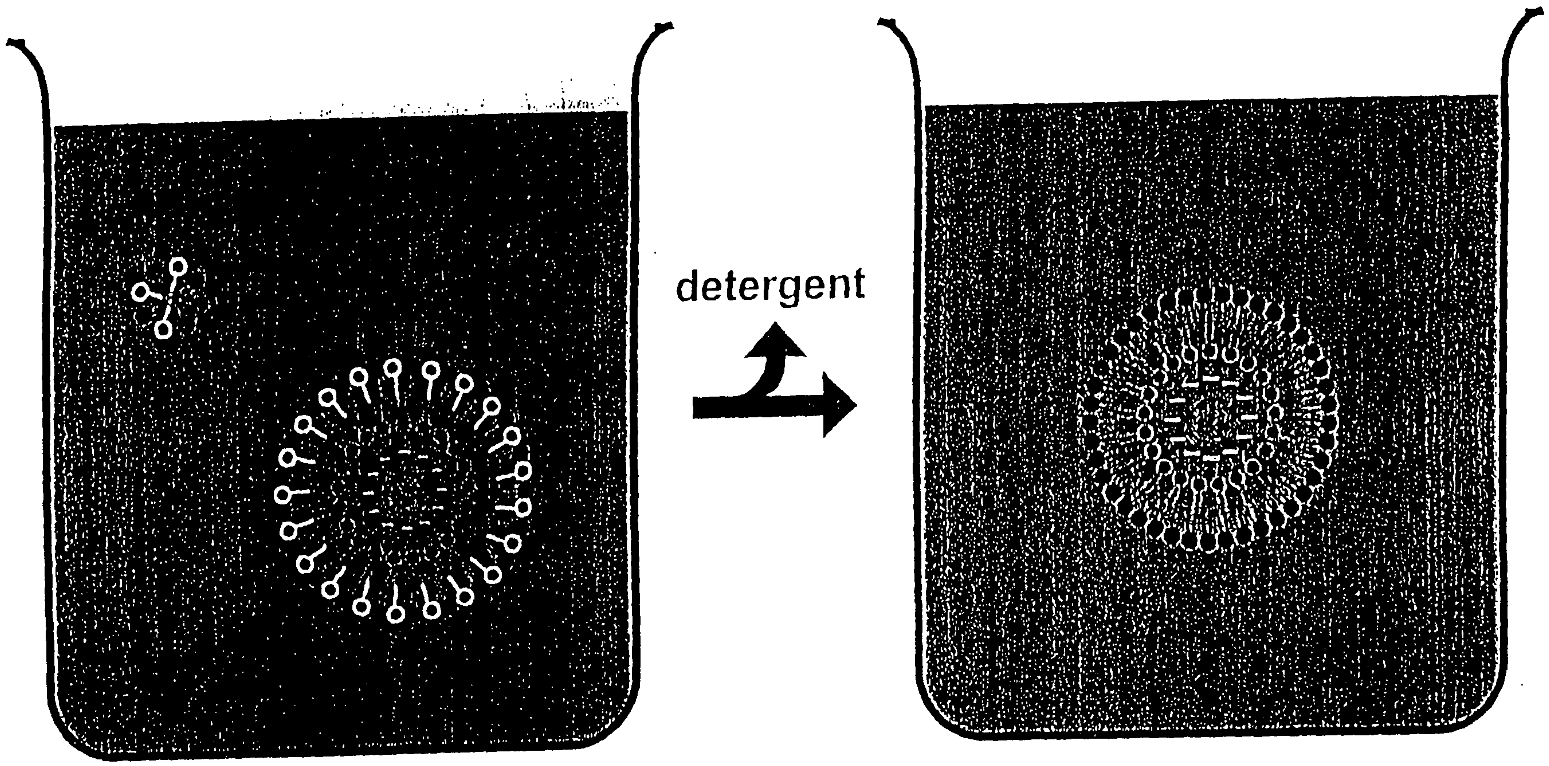


FIG. 3

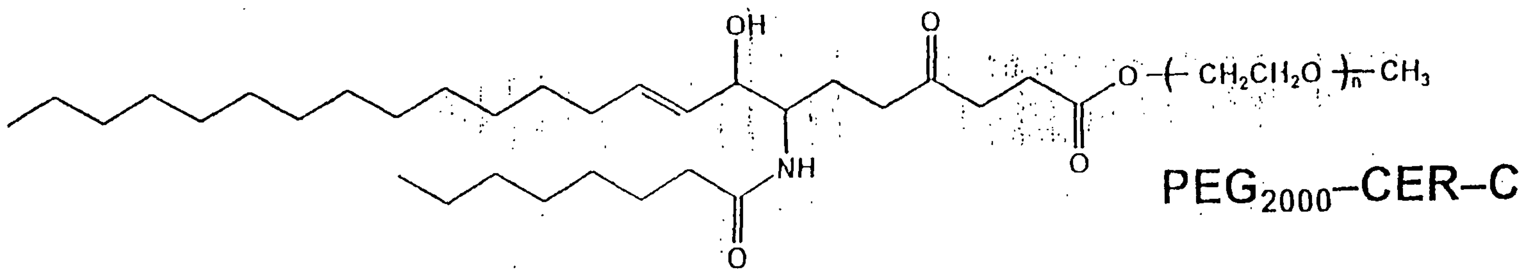

PEG$_{2000}$–CER–C8

| Ceramide | Half-time for dissociation |
|---|---|
| PEG$_{2000}$–CER–C8 | < 1 minute |
| PEG$_{2000}$–CER–C14 | 10 minutes |
| PEG$_{2000}$–CER–C20 | 22 hours |

FIG. 4

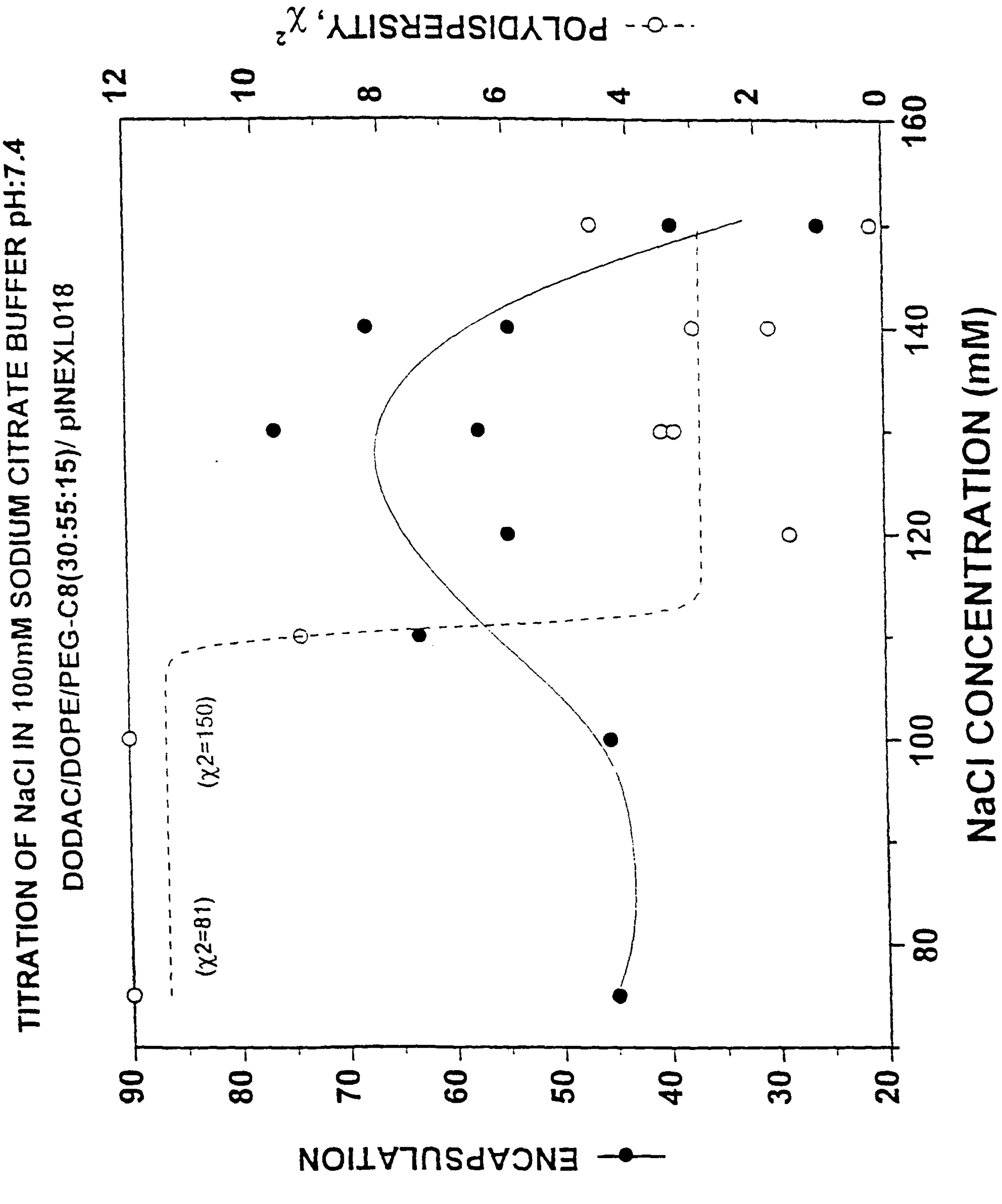
TITRATION OF NaCl IN 100mM SODIUM CITRATE BUFFER pH:7.4
DODAC/DOPE/PEG-C8(30:55:15)/ pINEXL018
ENCAPSULATION
POLYDISPERSITY, $\chi^2$
NaCl CONCENTRATION (mM)
($\chi$2=81)
($\chi$2=150)
90
80
70
60
50
40
30
20
12
10
8
6
4
2
0
80
100
120
140
160

FIG. 5

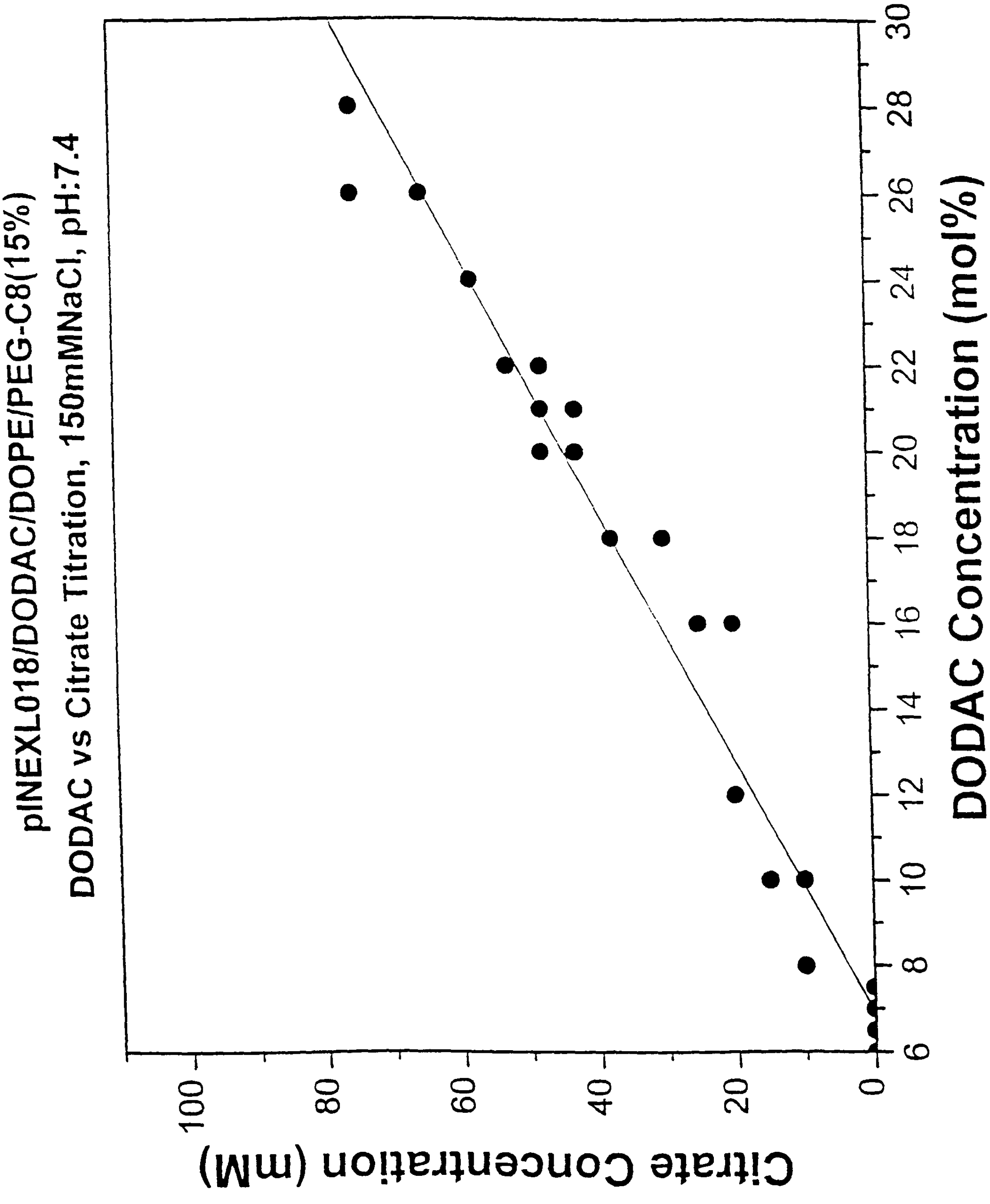
pINEXL018/DODAC/DOPE/PEG-C8(15%)
DODAC vs Citrate Titration, 150mMNaCl, pH:7.4
Citrate Concentration (mM)
0
20
40
60
80
100
DODAC Concentration (mol%)
6
8
10
12
14
16
18
20
22
24
26
28
30

FIG. 6

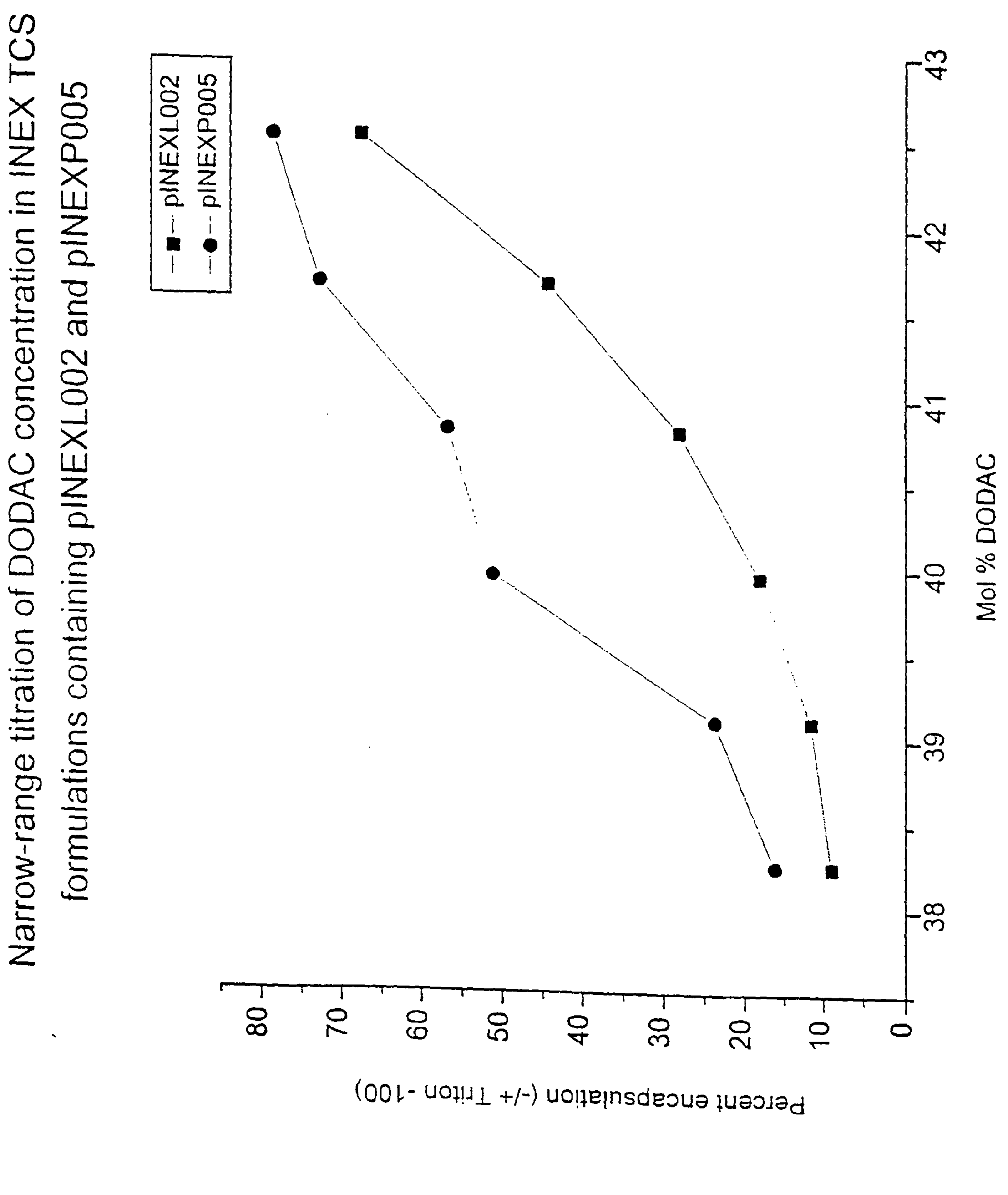
Narrow-range titration of DODAC concentration in INEX TCS formulations containing pINEXL002 and pINEXP005
pINEXL002
pINEXP005
Percent encapsulation (-/+ Triton -100)
0
10
20
30
40
50
60
70
80
38
39
40
41
42
43
Mol % DODAC

FIG. 7

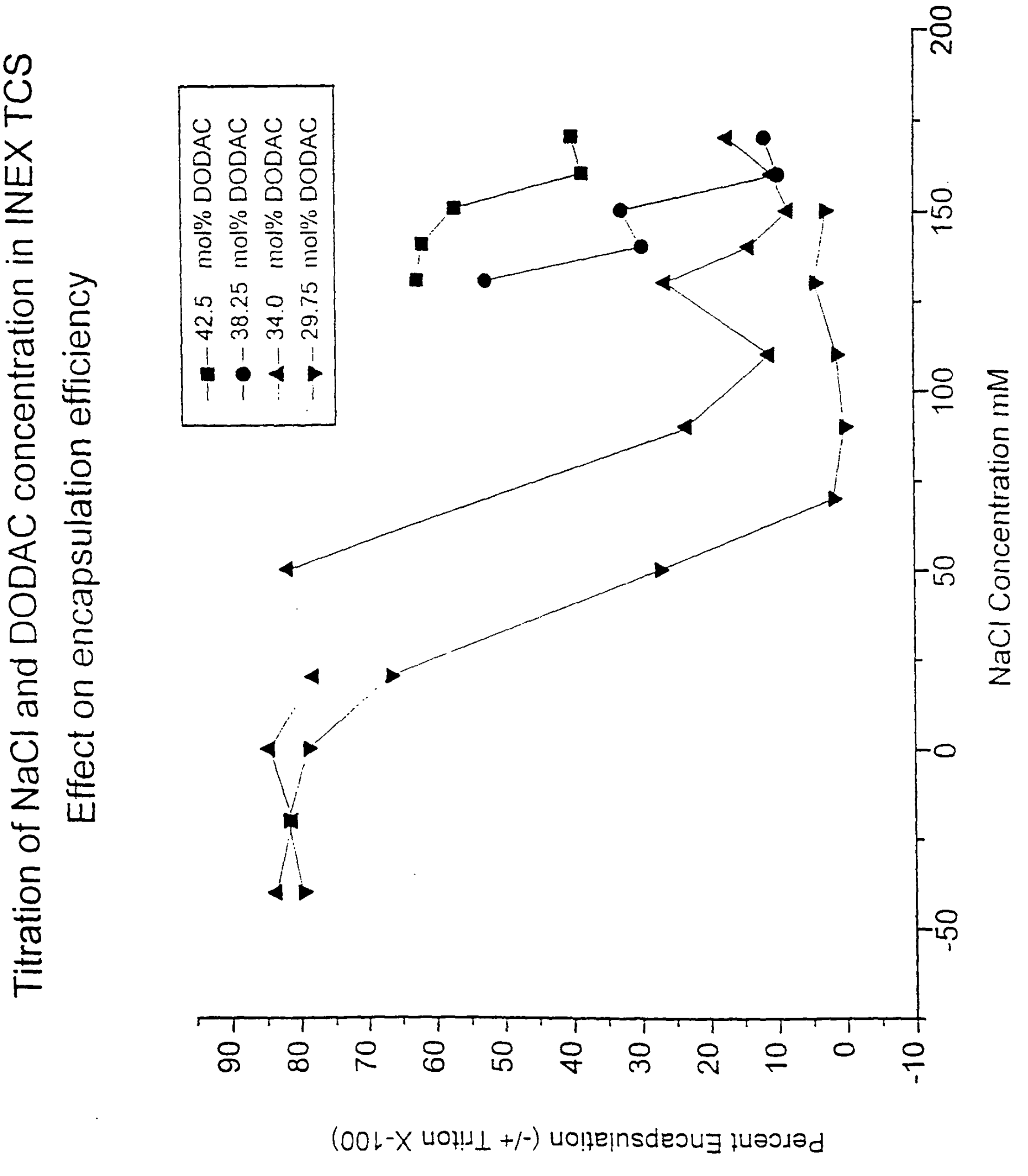
Titration of NaCl and DODAC concentration in INEX TCS
Effect on encapsulation efficiency
42.5 mol% DODAC
38.25 mol% DODAC
34.0 mol% DODAC
29.75 mol% DODAC
Percent Encapsulation (-/+ Triton X-100)
NaCl Concentration mM
-10
0
10
20
30
40
50
60
70
80
90
-50
0
50
100
150
200

FIG. 8

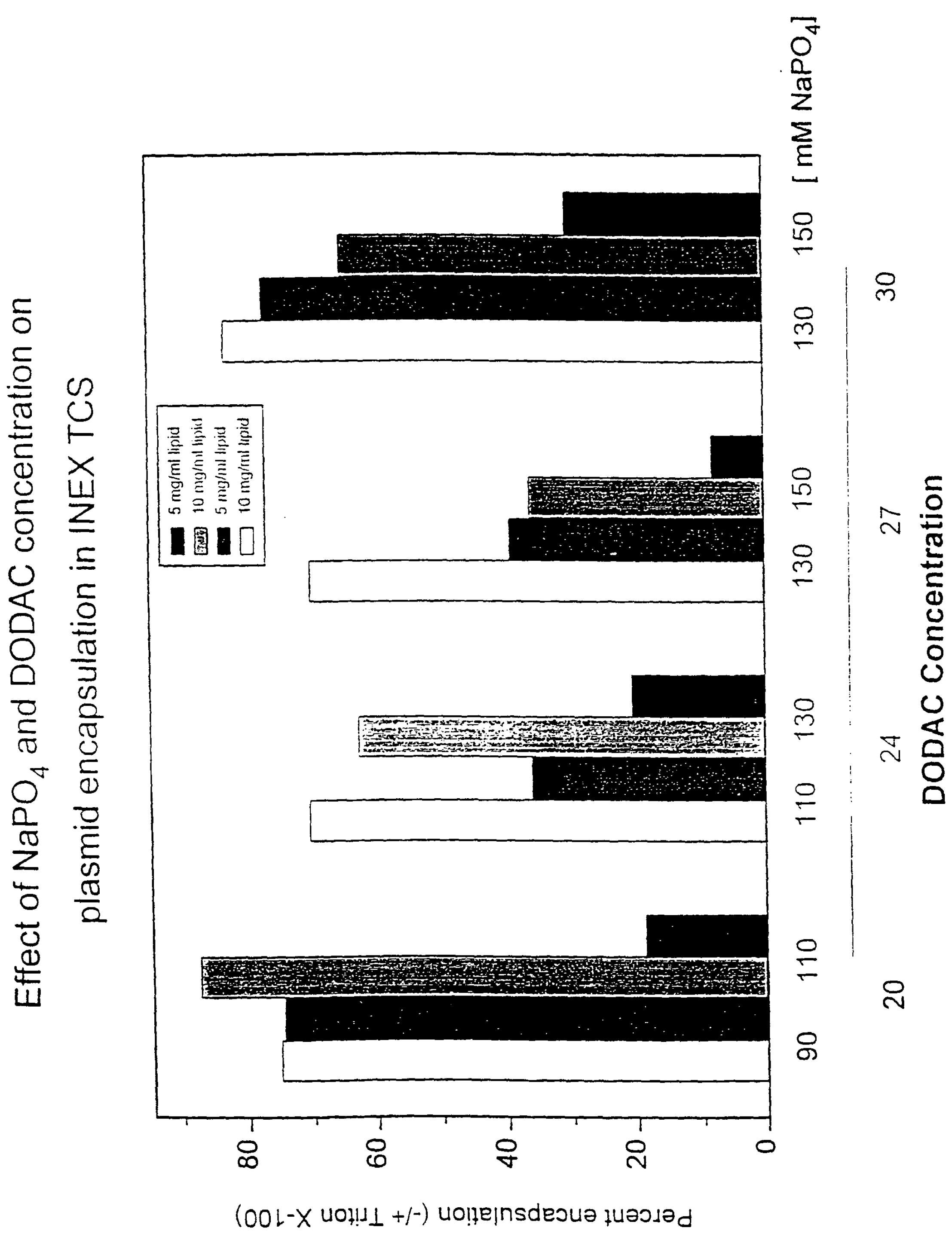
Effect of NaPO4 and DODAC concentration on plasmid encapsulation in INEX TCS
5 mg/ml lipid
10 mg/ml lipid
5 mg/ml lipid
10 mg/ml lipid
Percent encapsulation (-/+ Triton X-100)
0
20
40
60
80
90
110
110
130
130
150
130
150
[ mM NaPO4]
20
24
27
30
DODAC Concentration

FIG. 9

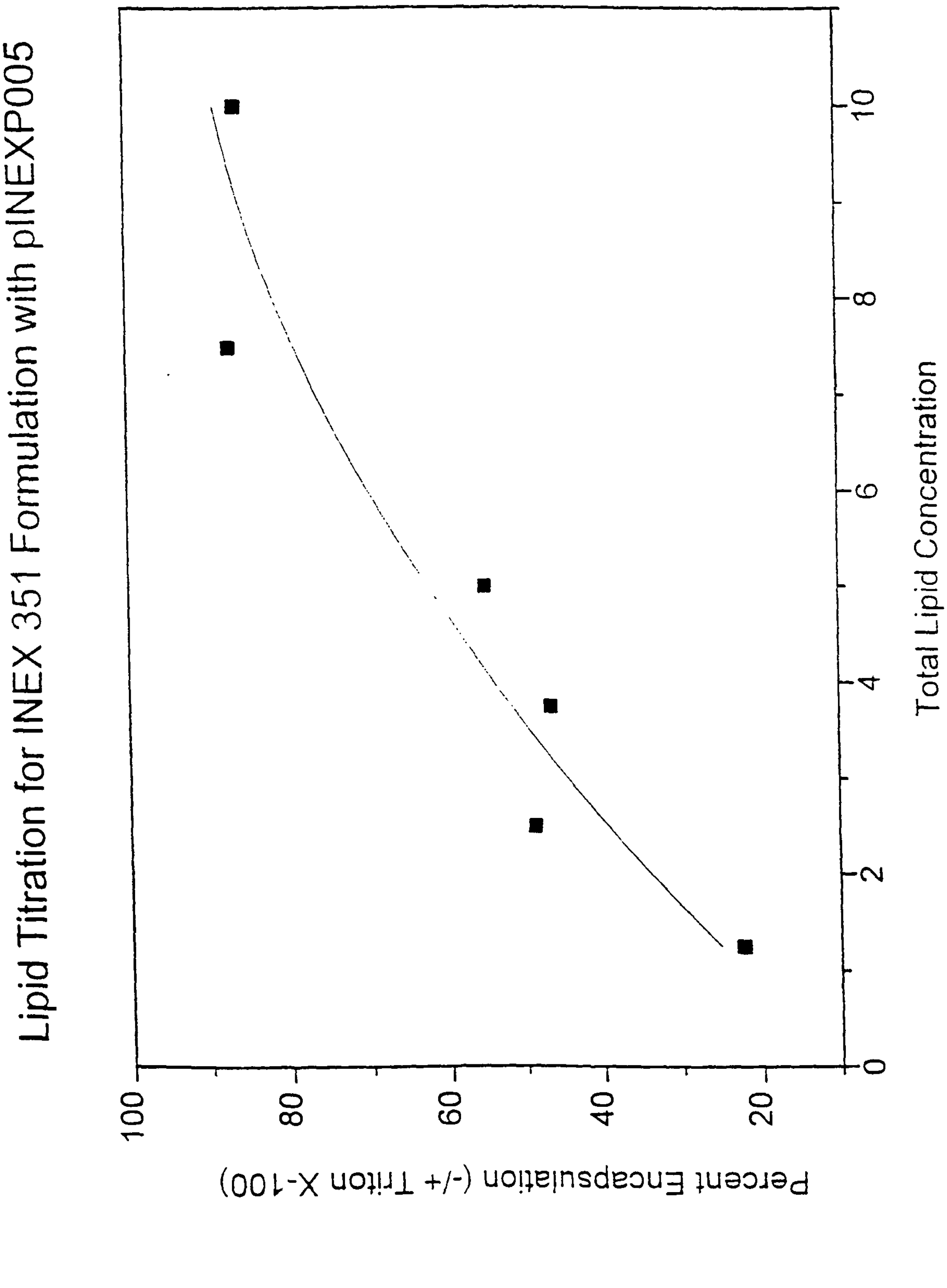
Lipid Titration for INEX 351 Formulation with pINEXP005
Percent Encapsulation (-/+ Triton X-100)
100
80
60
40
20
0
2
4
6
8
10
Total Lipid Concentration

FIG. 10

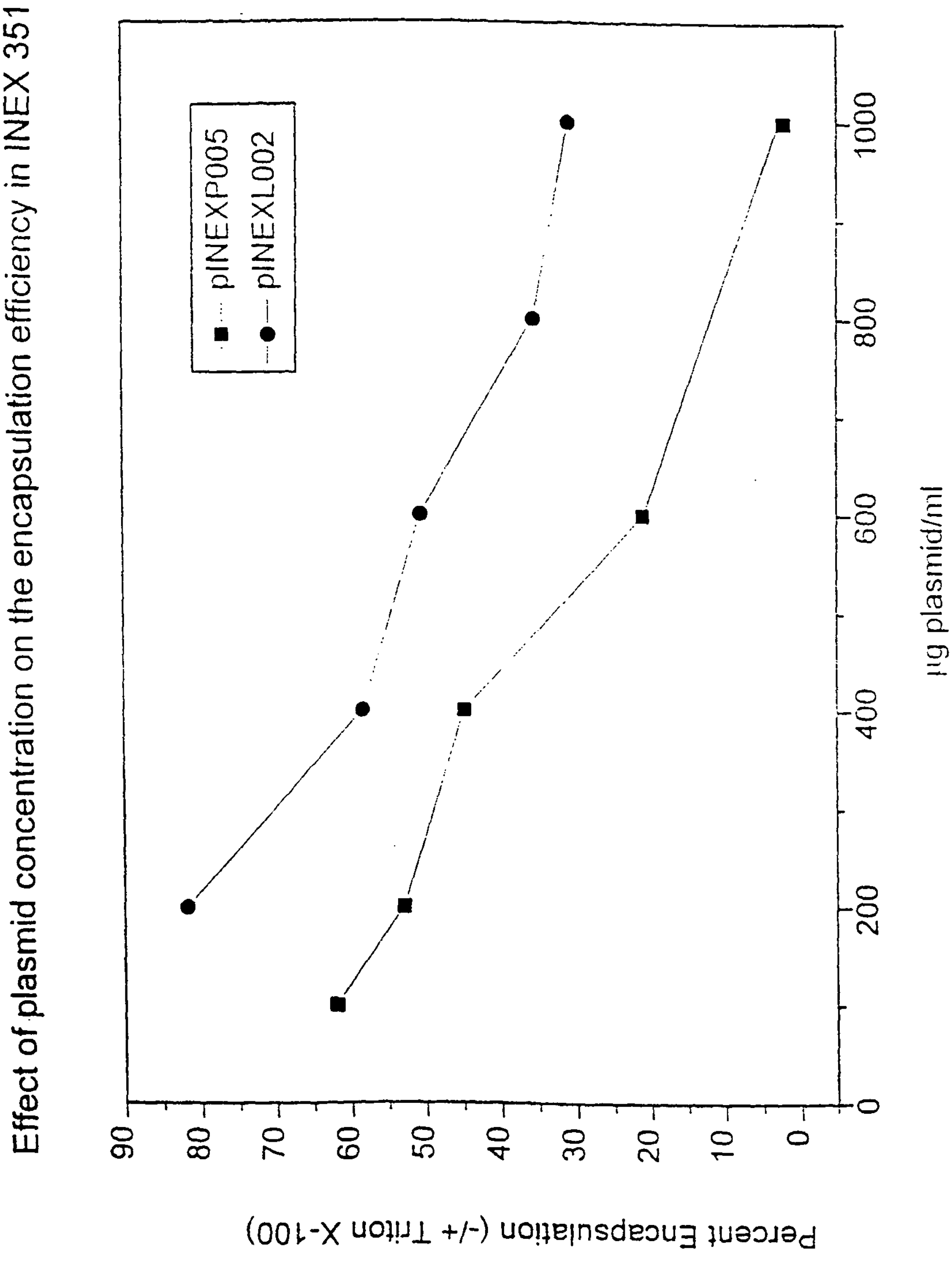
Effect of plasmid concentration on the encapsulation efficiency in INEX 351
pINEXP005
pINEXL002
Percent Encapsulation (-/+ Triton X-100)
µg plasmid/ml
0
10
20
30
40
50
60
70
80
90
0
200
400
600
800
1000

FIG. 11

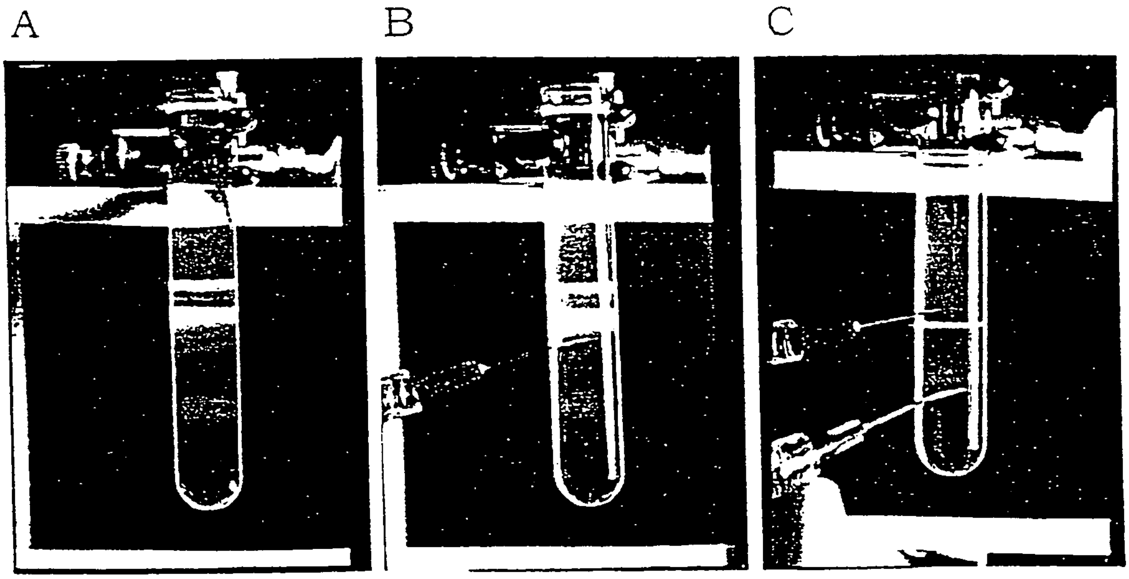


FIG. 12

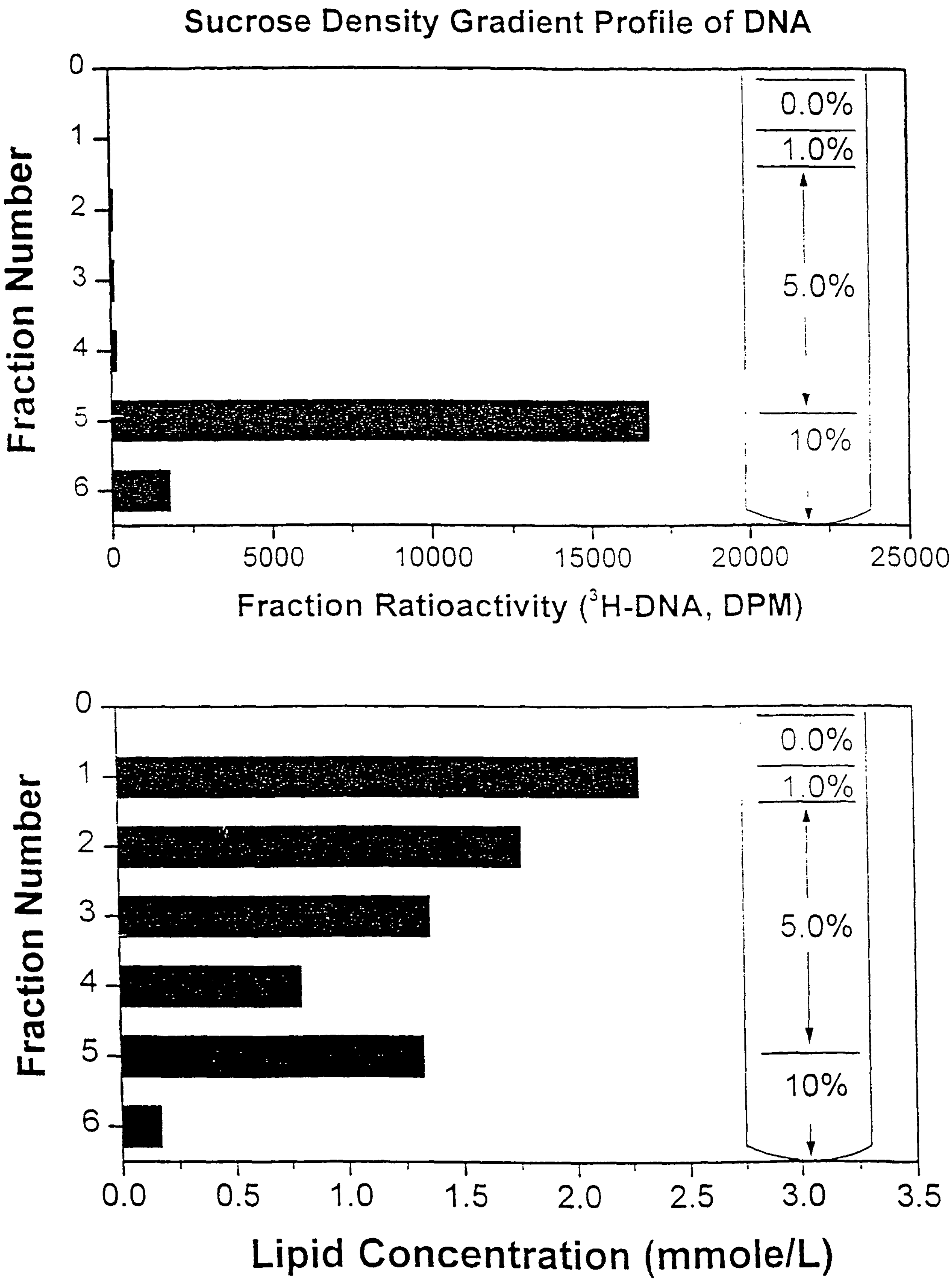
Sucrose Density Gradient Profile of DNA
Fraction Number
0
1
2
3
4
5
6
0.0%
1.0%
5.0%
10%
0
5000
10000
15000
20000
25000
Fraction Ratioactivity (3H-DNA, DPM)
Fraction Number
0.0%
1.0%
5.0%
10%
0.0
0.5
1.0
1.5
2.0
2.5
3.0
3.5
Lipid Concentration (mmole/L)

FIG. 13

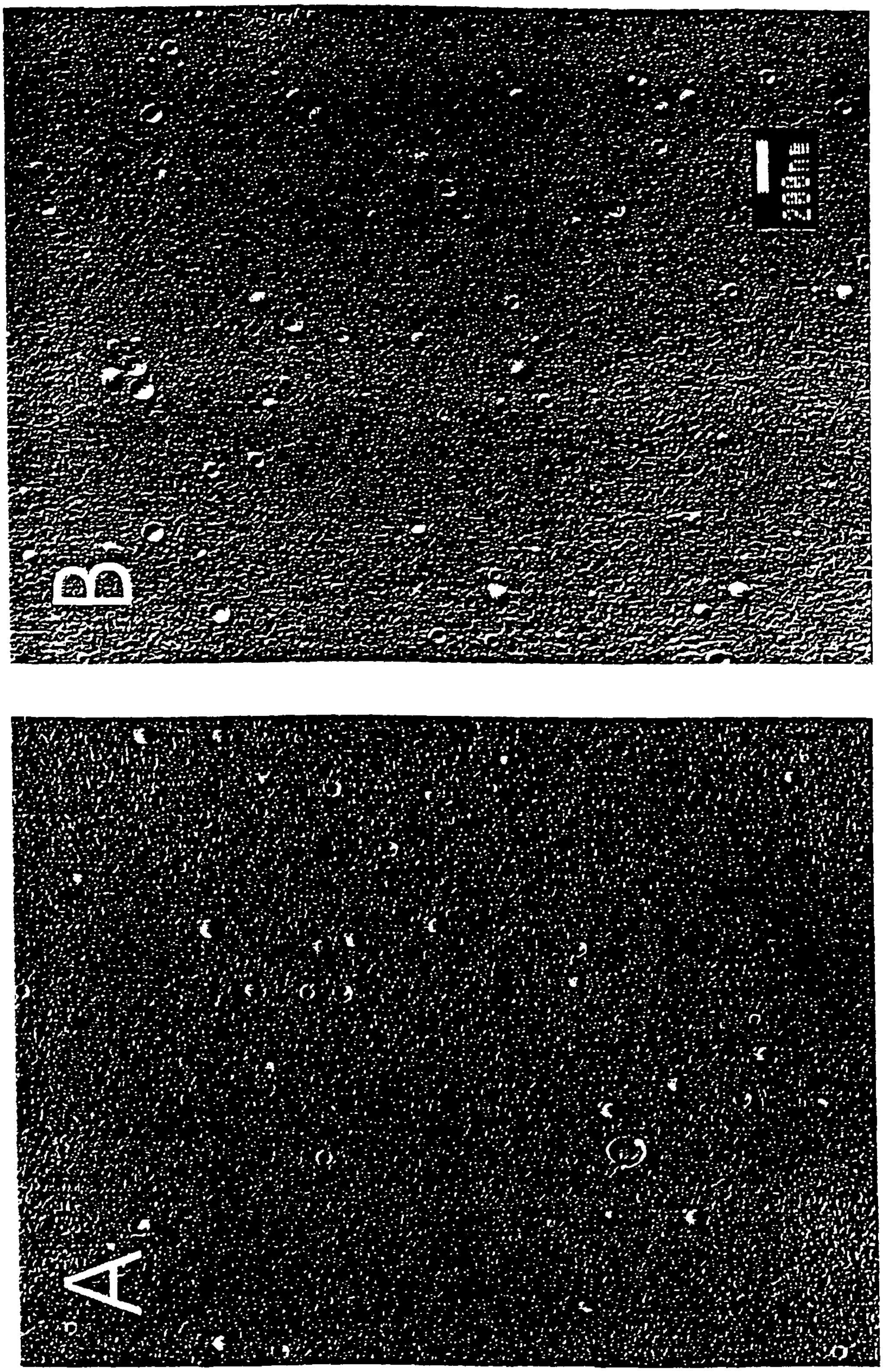


FIG. 14

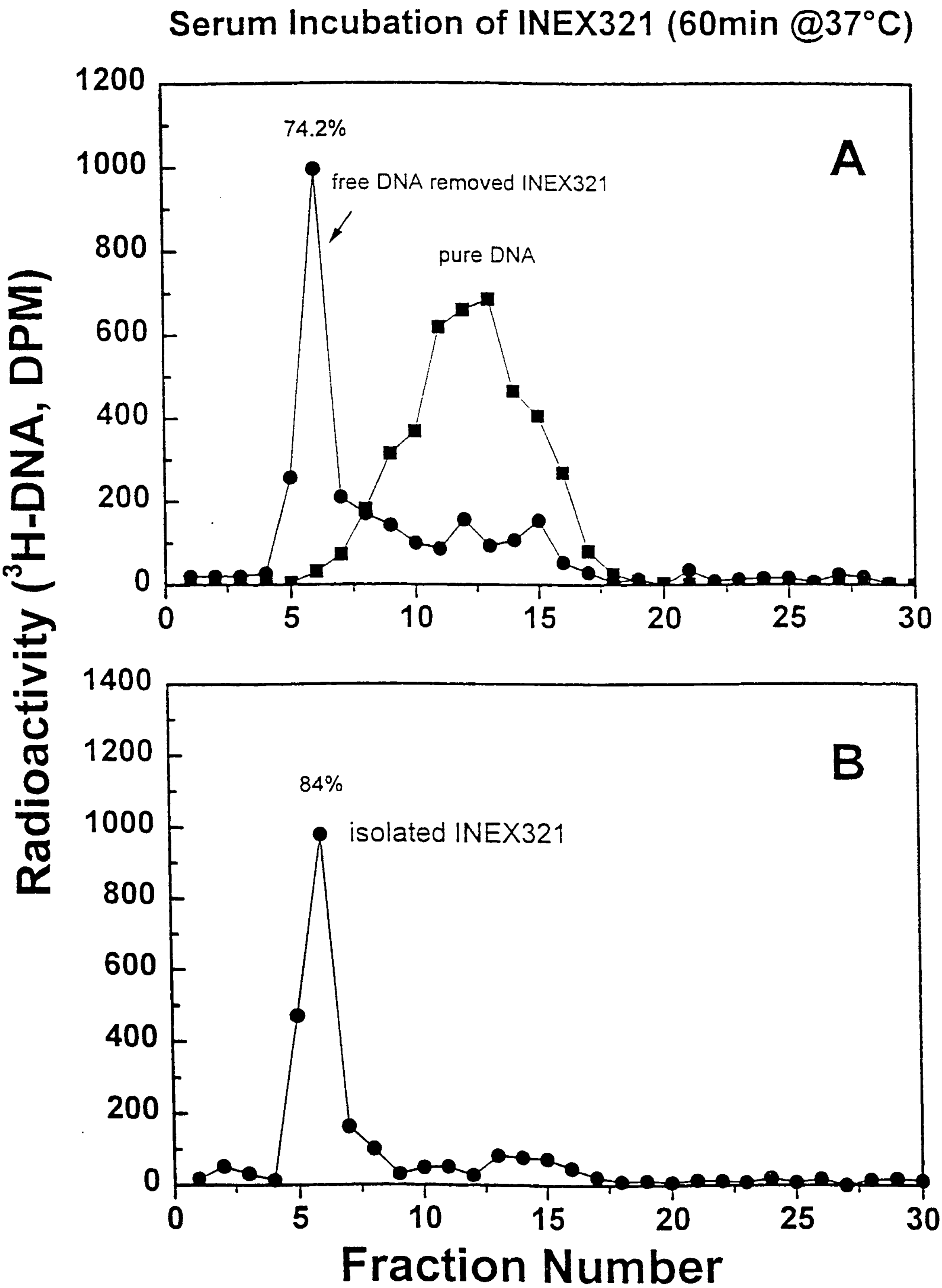
Serum Incubation of INEX321 (60min @37°C)
Radioactivity (³H-DNA, DPM)
A
74.2%
free DNA removed INEX321
pure DNA
B
84%
isolated INEX321
Fraction Number
0
200
400
600
800
1000
1200
1400
0
5
10
15
20
25
30

FIG. 15

| LANE | 1 | 2 | 3 | 4 | 5 | 6 | 7 | 8 | 9 | 10 | 11 | 12 |
|---|---|---|---|---|---|---|---|---|---|---|---|---|
| Serum | - | + | - | - | - | + | - | - | - | + | - | - |
| DNA'se | - | - | + | + | - | - | + | + | - | - | + | + |
| Carrier | + | - | - | + | + | - | - | + | + | - | - | + |

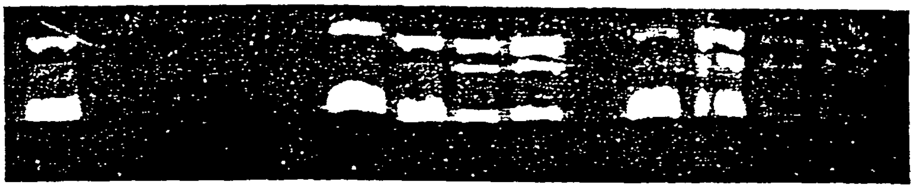

Fig. 16 Serum and DNA'se stability of plasmid, INEX 351 and Complexes. Lanes 1-4 free pINEX L018, Lanes 5-8 pINEXL018 encapsulated INEX351 and Lanes 9-12 DOPE:DODAC complexed pINEX L018. Plasmid and formulations were treated with the above agents and the DNA extracted as described in Materials and Methods. Carrier refers to of PronaseK/SDS inactivated serum added in identical quantities to normal serum.

FIG. 16

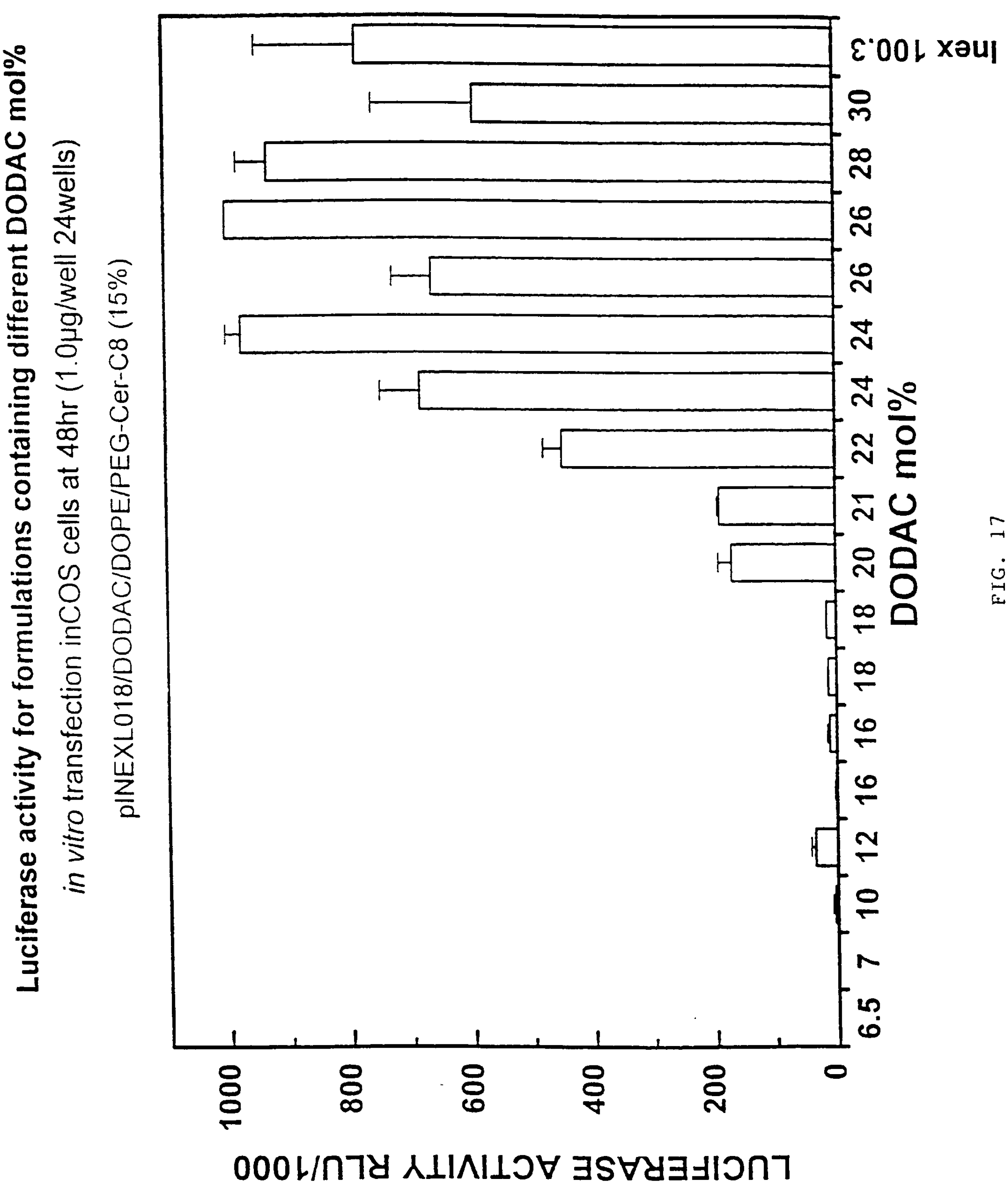
Luciferase activity for formulations containing different DODAC mol%
in vitro transfection inCOS cells at 48hr (1.0µg/well 24wells)
pINEXL018/DODAC/DOPE/PEG-Cer-C8 (15%)
LUCIFERASE ACTIVITY RLU/1000
0
200
400
600
800
1000
6.5
7
10
12
16
16
18
18
20
21
22
24
24
26
26
28
30
Inex 100.3
DODAC mol%

FIG. 17

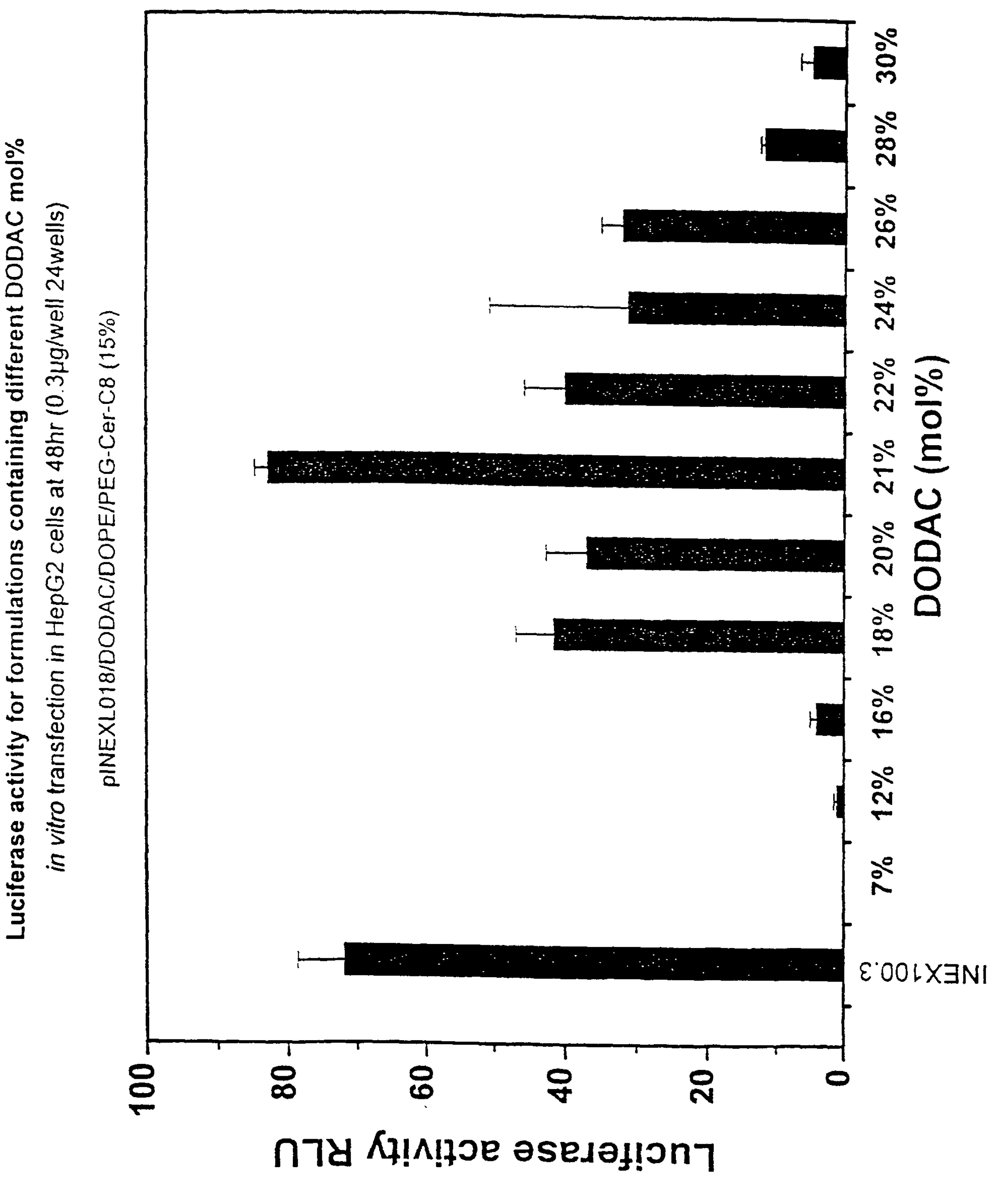
Luciferase activity for formulations containing different DODAC mol%
*in vitro* transfection in HepG2 cells at 48hr (0.3µg/well 24wells)
pINEXL018/DODAC/DOPE/PEG-Cer-C8 (15%)
Luciferase activity RLU
0
20
40
60
80
100
INEX100.3
7%
12%
16%
18%
20%
21%
22%
24%
26%
28%
30%
DODAC (mol%)

FIG. 18

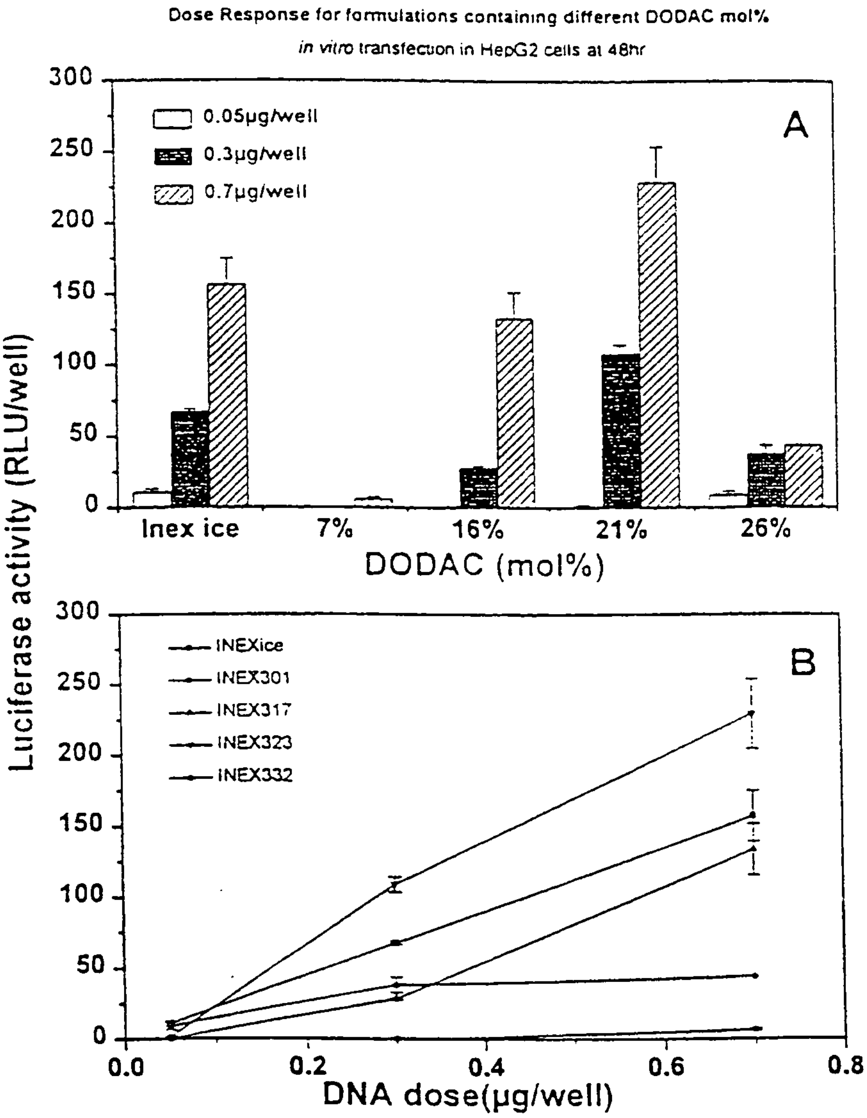
Dose Response for formulations containing different DODAC mol%
in vitro transfection in HepG2 cells at 48hr
A
0.05µg/well
0.3µg/well
0.7µg/well
Inex ice
7%
16%
21%
26%
DODAC (mol%)
Luciferase activity (RLU/well)
B
INEXice
INEX301
INEX317
INEX323
INEX332
DNA dose(µg/well)
0
50
100
150
200
250
300
0.0
0.2
0.4
0.6
0.8

FIG. 19

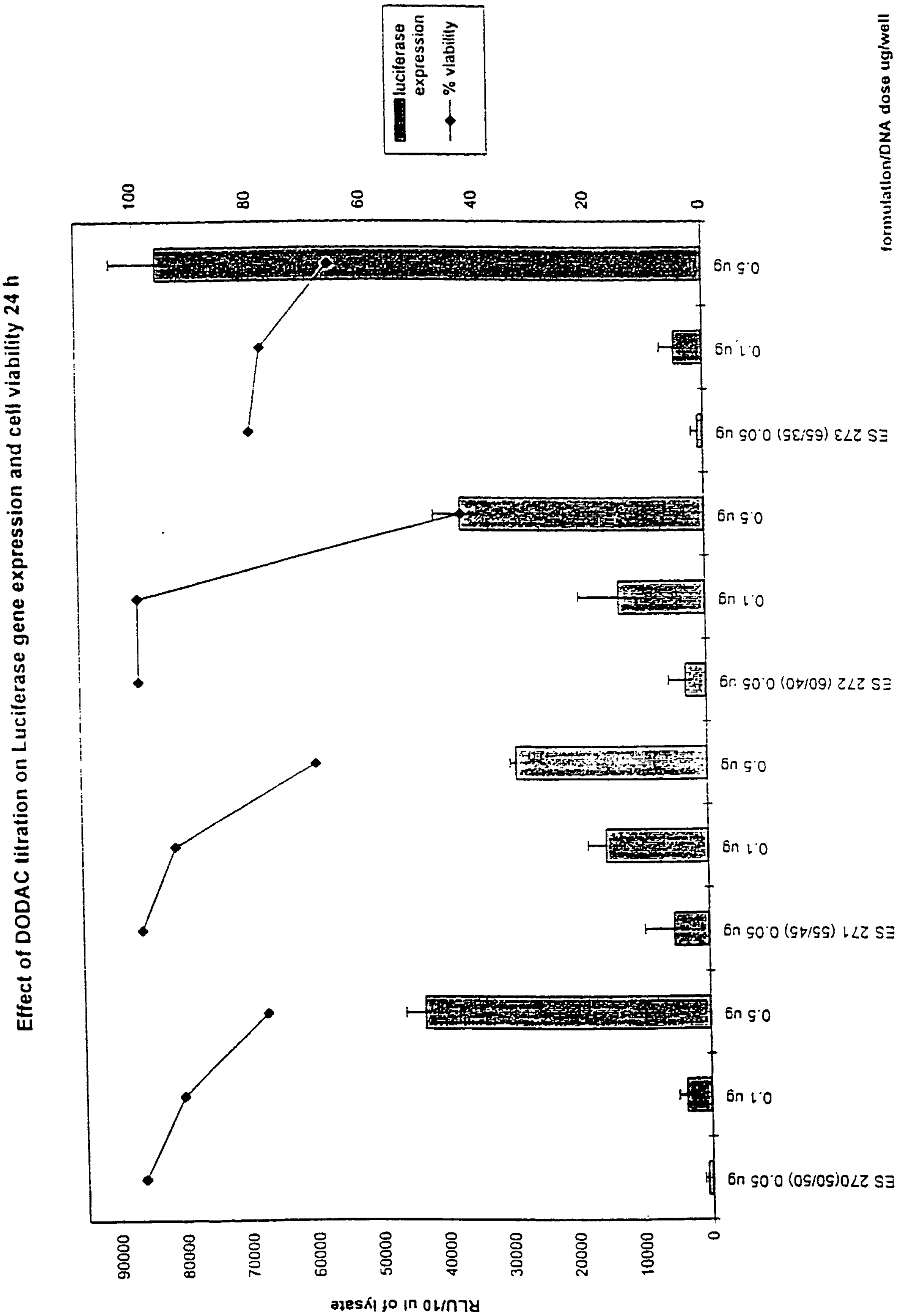
Effect of DODAC titration on Luciferase gene expression and cell viability 24 h
luciferase expression
% viability
RLU/10 ul of lysate
90000
80000
70000
60000
50000
40000
30000
20000
10000
0
100
80
60
40
20
0
ES 270(50/50) 0.05 ug
0.1 ug
0.5 ug
ES 271 (55/45) 0.05 ug
0.1 ug
0.5 ug
ES 272 (60/40) 0.05 ug
0.1 ug
0.5 ug
ES 273 (65/35) 0.05 ug
0.1 ug
0.5 ug
formulation/DNA dose ug/well

FIG. 20

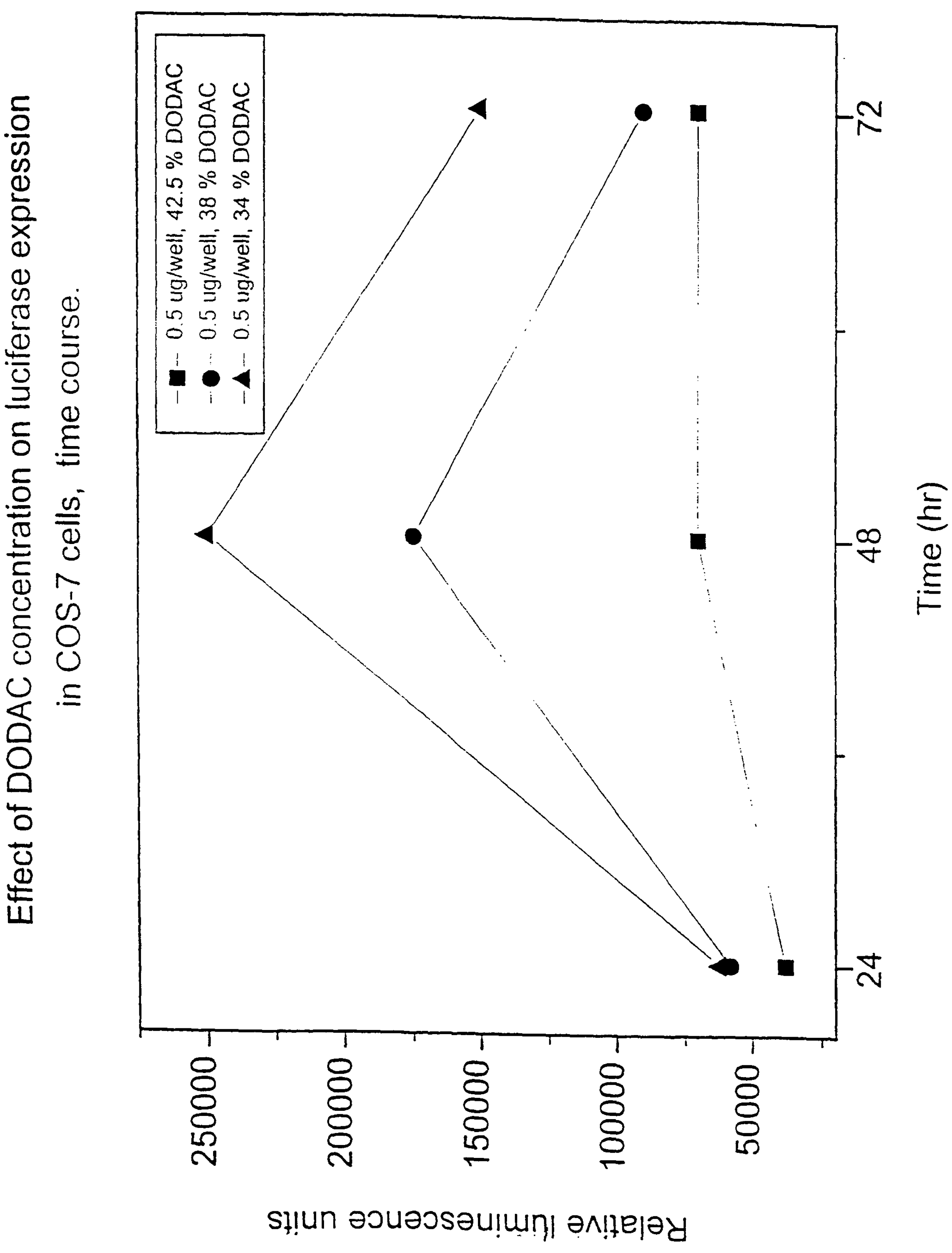
Effect of DODAC concentration on luciferase expression
in COS-7 cells, time course.
0.5 ug/well, 42.5 % DODAC
0.5 ug/well, 38 % DODAC
0.5 ug/well, 34 % DODAC
Relative luminescence units
250000
200000
150000
100000
50000
24
48
72
Time (hr)

FIG. 21

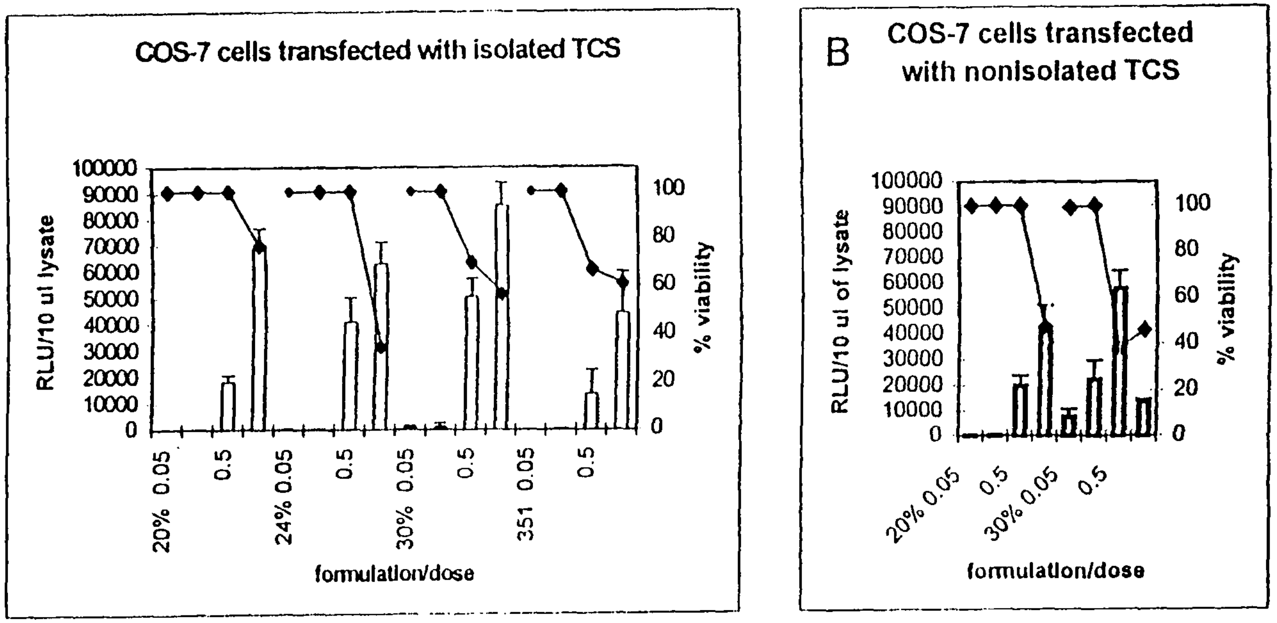


Fig. 22. **Comparison of transfection activity from isolated vs. non-isolated TCS.** 42.5, 30, 24 and 20 mol % DODAC TCS formulations were incubated with 0.05, 0.1, 0.5, 1.0 μg DNA/well for 24 hr with COS-7 cells. Part A: dose response of non-isolated formulations Part B: dose response of isolated formulations.

FIG. 22

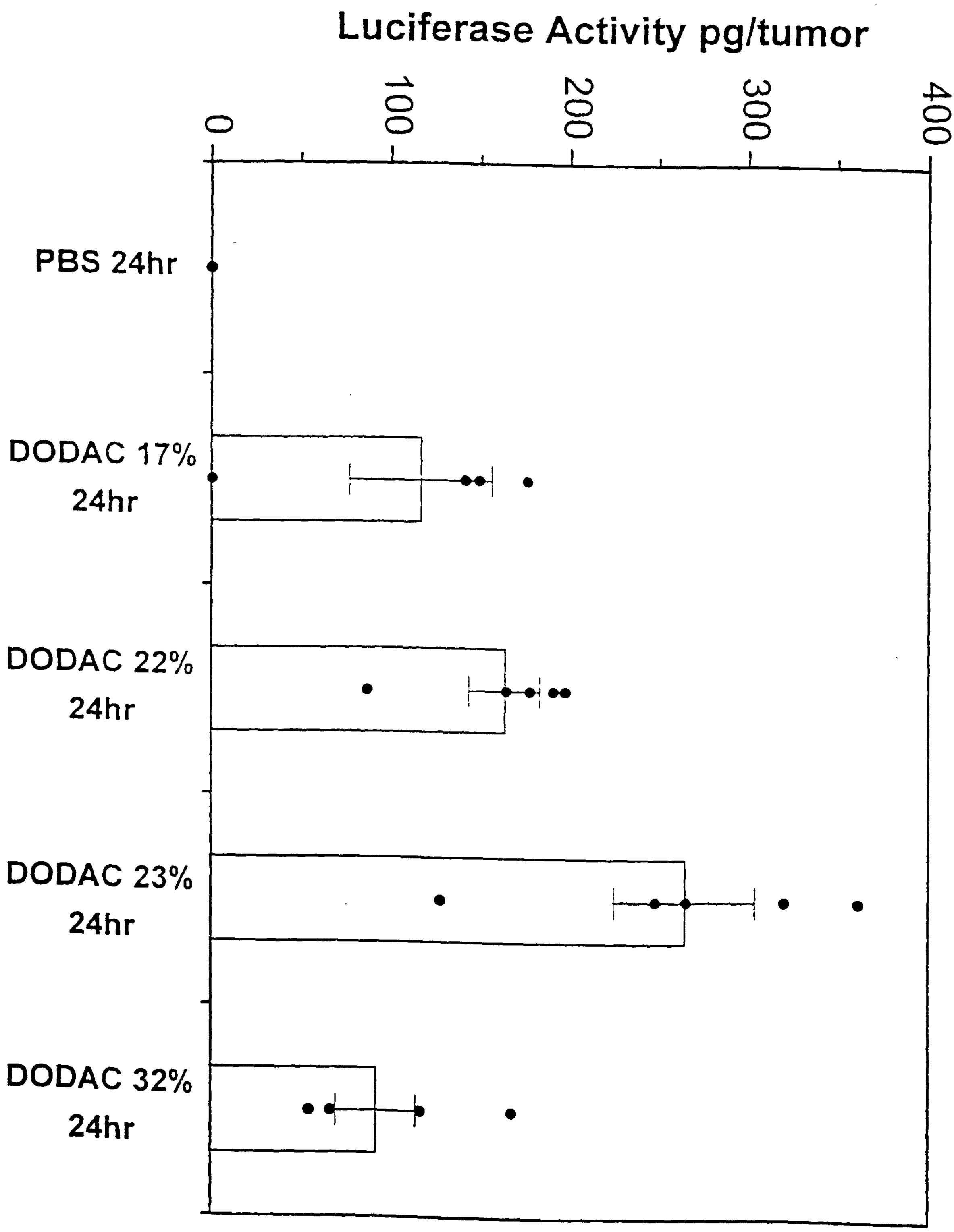
Luciferase Activity pg/tumor
0
100
200
300
400
PBS 24hr
DODAC 17% 24hr
DODAC 22% 24hr
DODAC 23% 24hr
DODAC 32% 24hr

FIG. 23

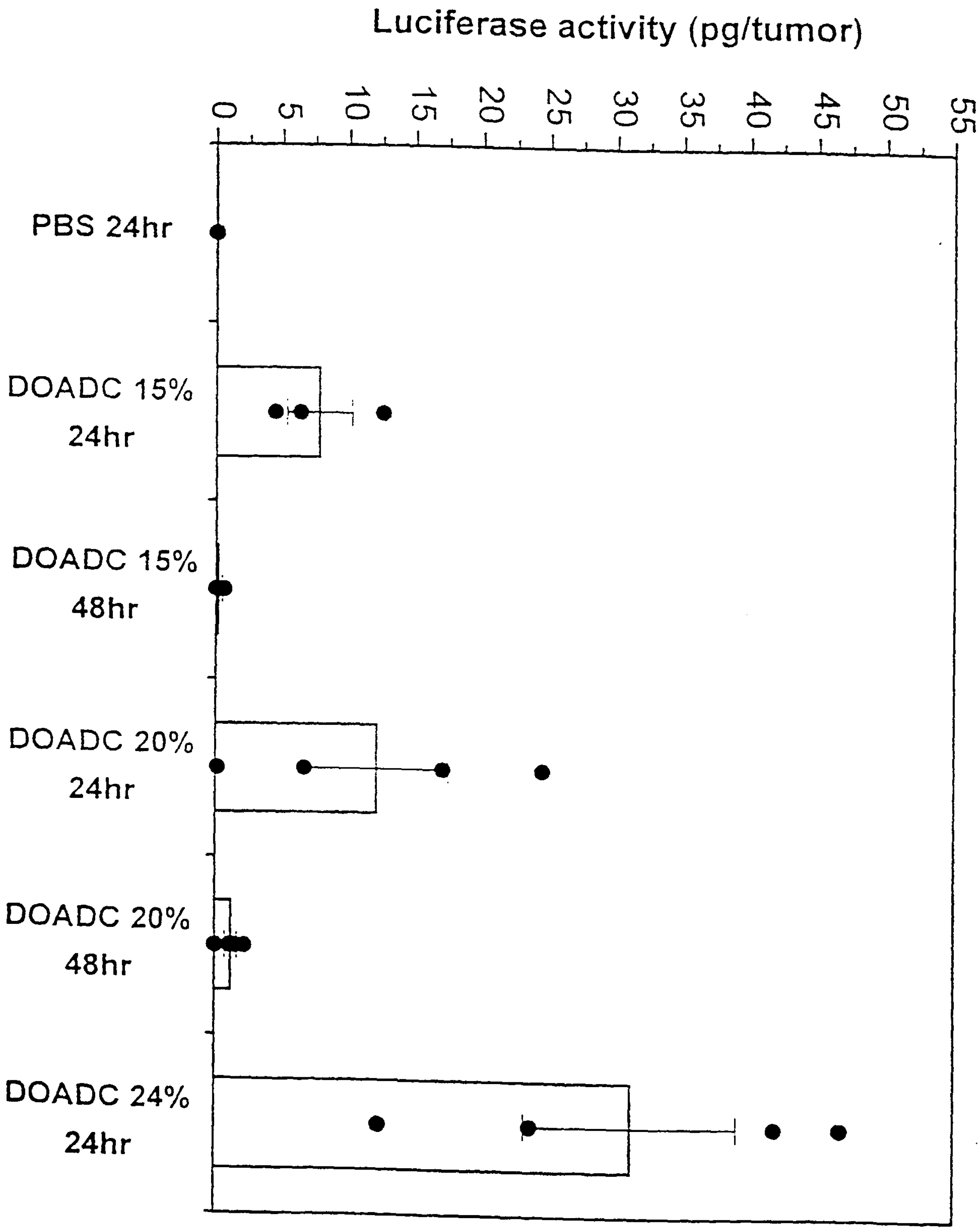
Luciferase activity (pg/tumor)
0
5
10
15
20
25
30
35
40
45
50
55
PBS 24hr
DOADC 15%
24hr
DOADC 15%
48hr
DOADC 20%
24hr
DOADC 20%
48hr
DOADC 24%
24hr

FIG. 24

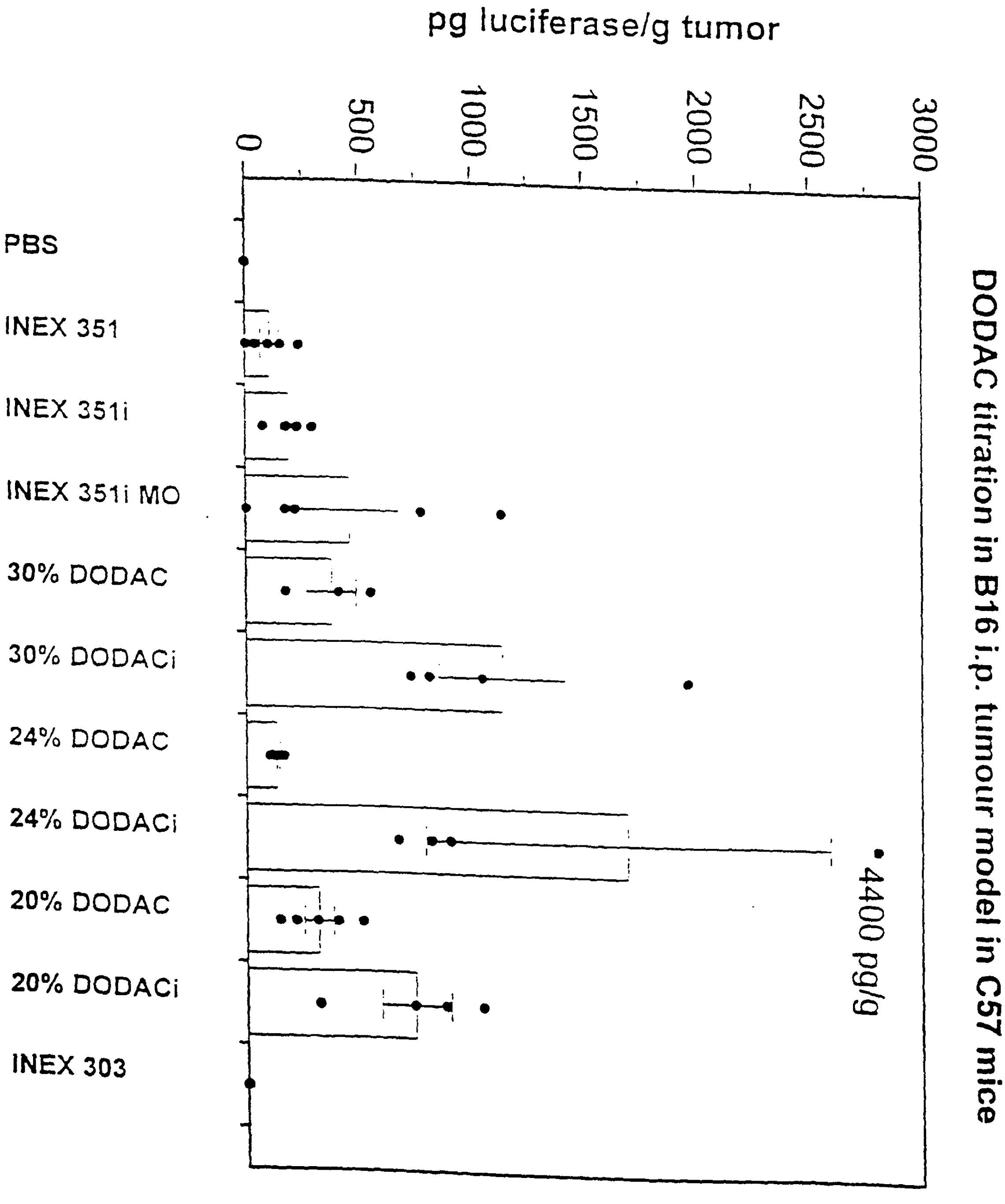
DODAC titration in B16 i.p. tumour model in C57 mice
pg luciferase/g tumor
0
500
1000
1500
2000
2500
3000
PBS
INEX 351
INEX 351i
INEX 351i MO
30% DODAC
30% DODACi
24% DODAC
24% DODACi
20% DODAC
20% DODACi
INEX 303
4400 pg/g

FIG. 25

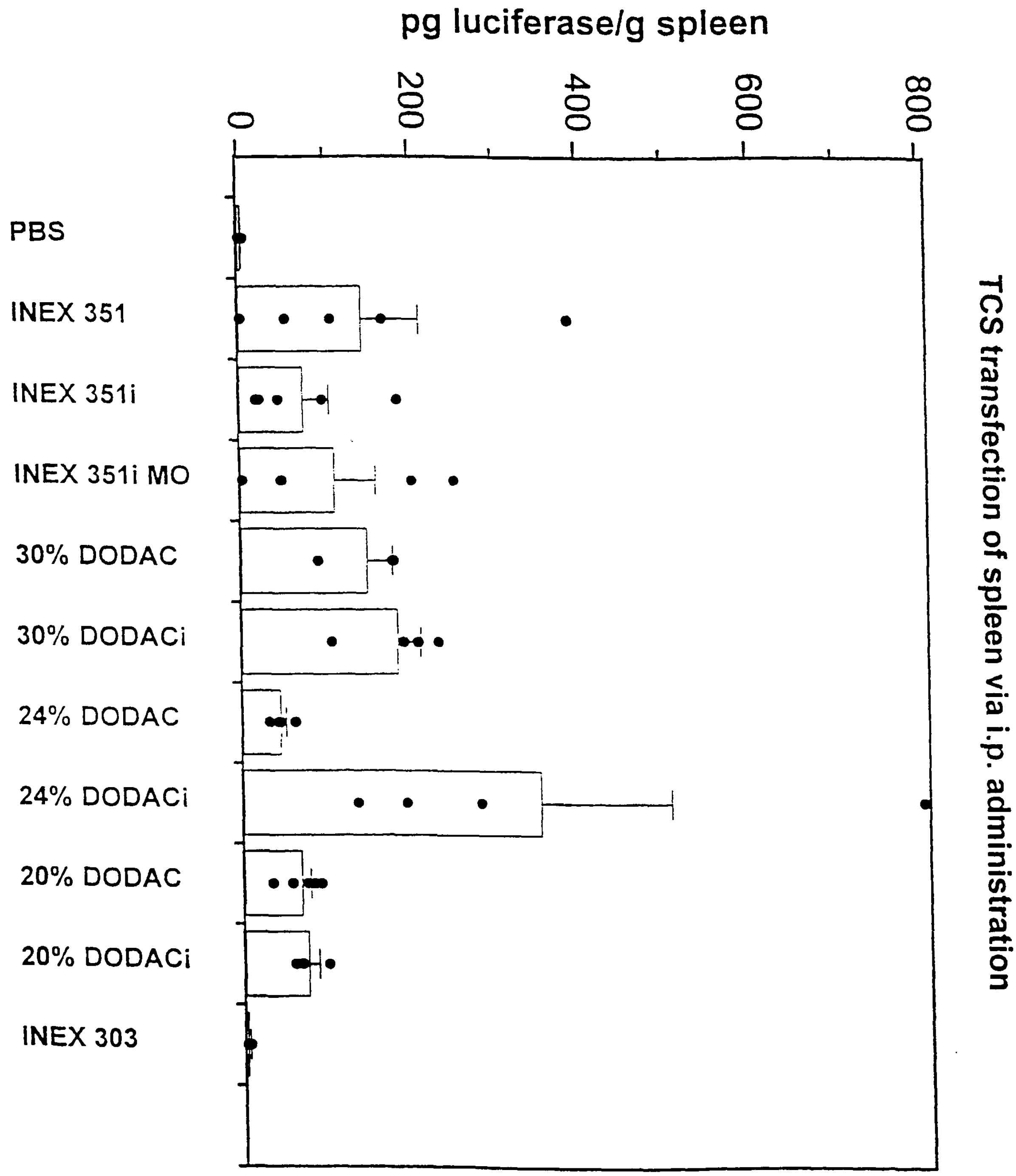
TCS transfection of spleen via i.p. administration
pg luciferase/g spleen
0
200
400
600
800
PBS
INEX 351
INEX 351i
INEX 351i MO
30% DODAC
30% DODACi
24% DODAC
24% DODACi
20% DODAC
20% DODACi
INEX 303

FIG. 26

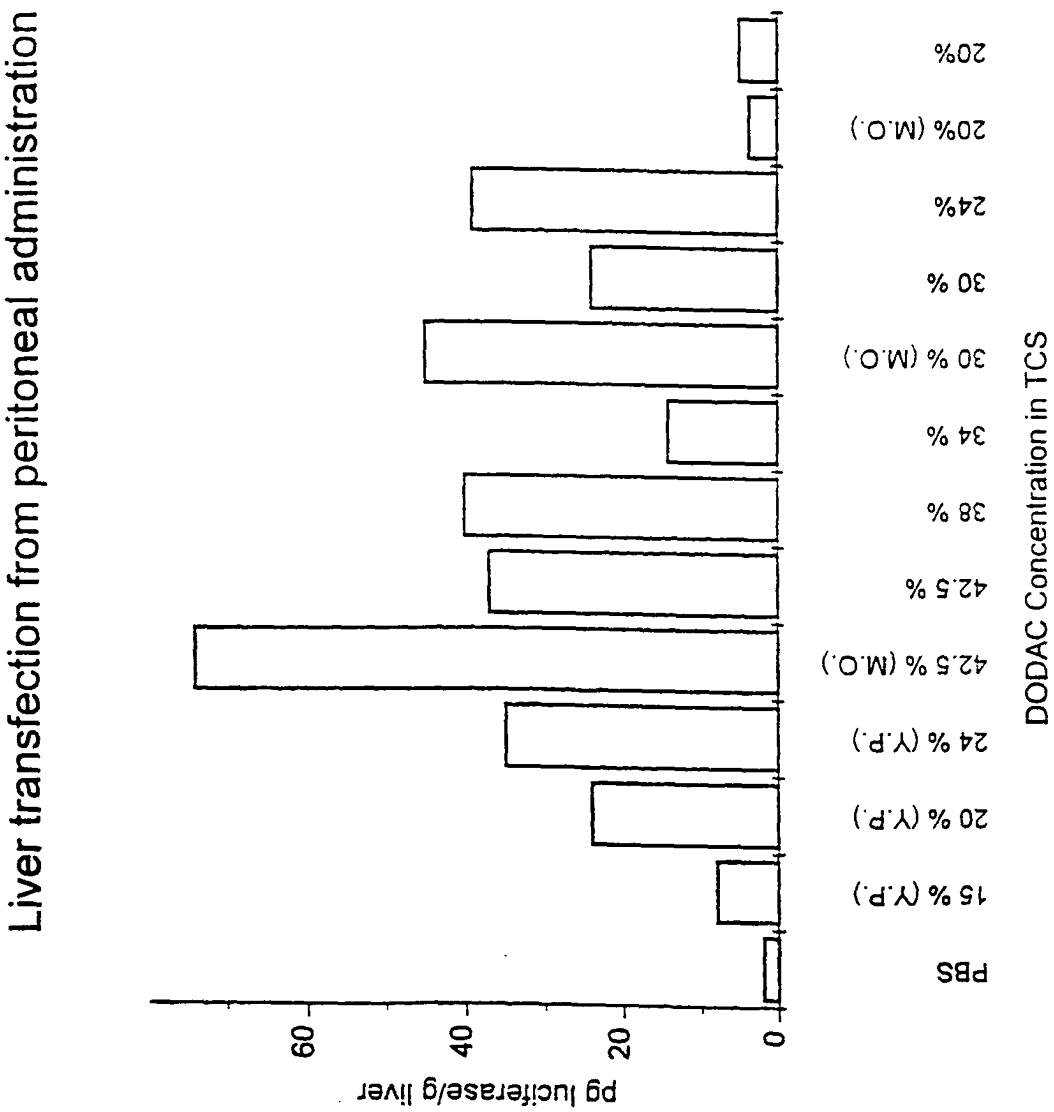
Liver transfection from peritoneal administration
pg luciferase/g liver
0
20
40
60
PBS
15 % (Y.P.)
20 % (Y.P.)
24 % (Y.P.)
42.5 % (M.O.)
42.5 %
38 %
34 %
30 % (M.O.)
30 %
24%
20% (M.O.)
20%
DODAC Concentration in TCS

FIG. 27

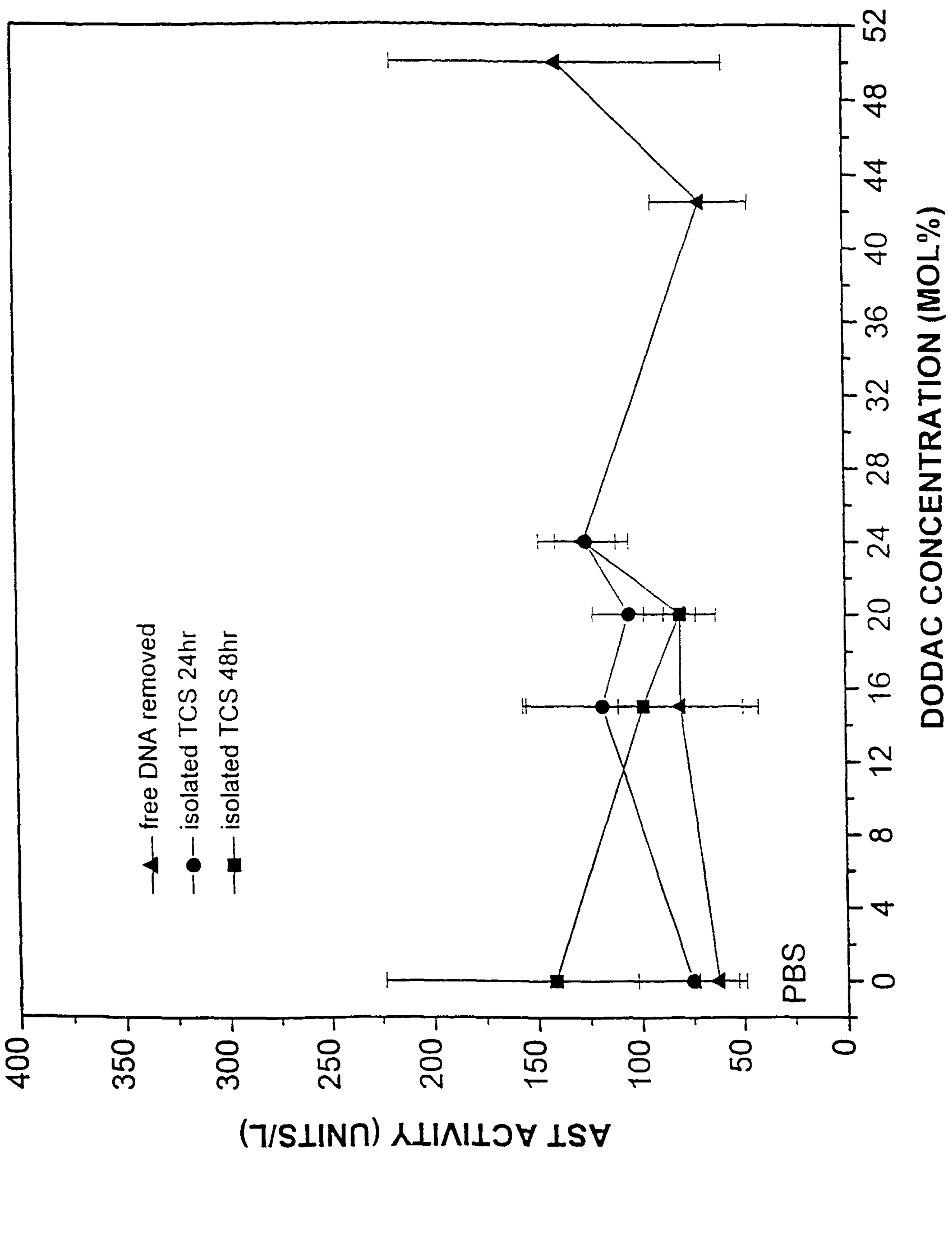
free DNA removed
isolated TCS 24hr
isolated TCS 48hr
PBS
AST ACTIVITY (UNITS/L)
DODAC CONCENTRATION (MOL%)
0
50
100
150
200
250
300
350
400
0
4
8
12
16
20
24
28
32
36
40
44
48
52

FIG. 28

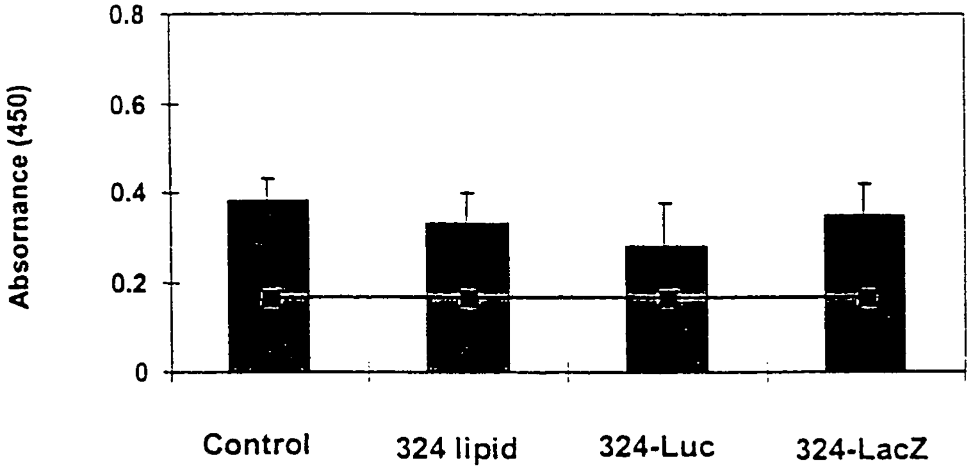
0.8
0.6
0.4
0.2
0
Absornance (450)
Control
324 lipid
324-Luc
324-LacZ

FIGURE 29

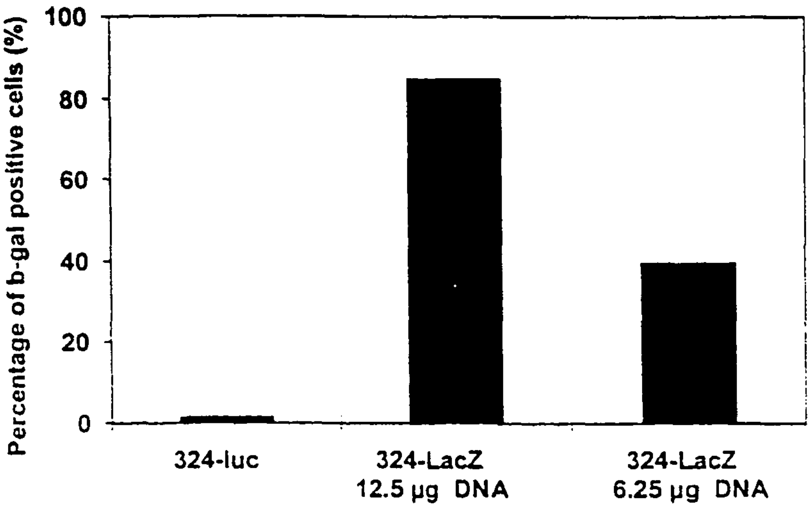
Percentage of b-gal positive cells (%)
100
80
60
40
20
0
324-luc
324-LacZ
12.5 µg DNA
324-LacZ
6.25 µg DNA

FIGURE 30

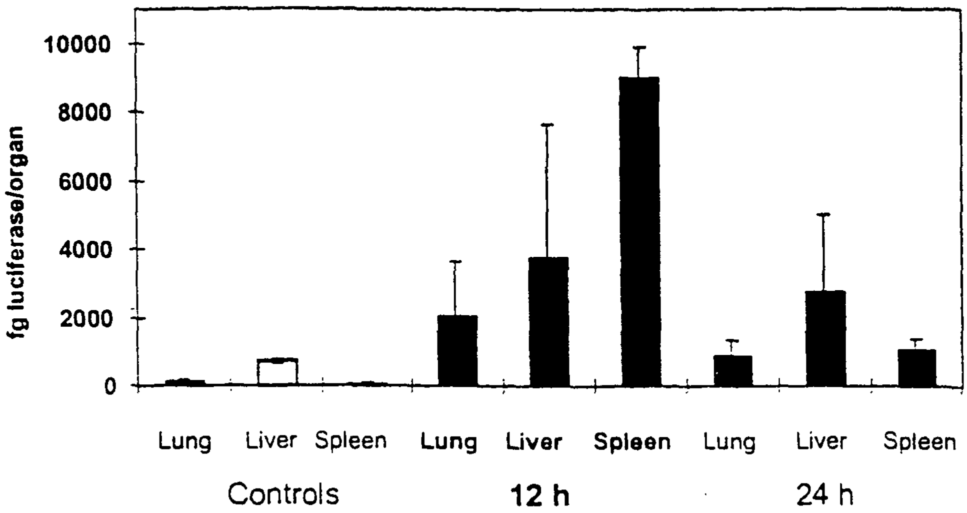
fg luciferase/organ
10000
8000
6000
4000
2000
0
Lung
Liver
Spleen
Lung
Liver
Spleen
Lung
Liver
Spleen
Controls
12 h
24 h

FIGURE 31

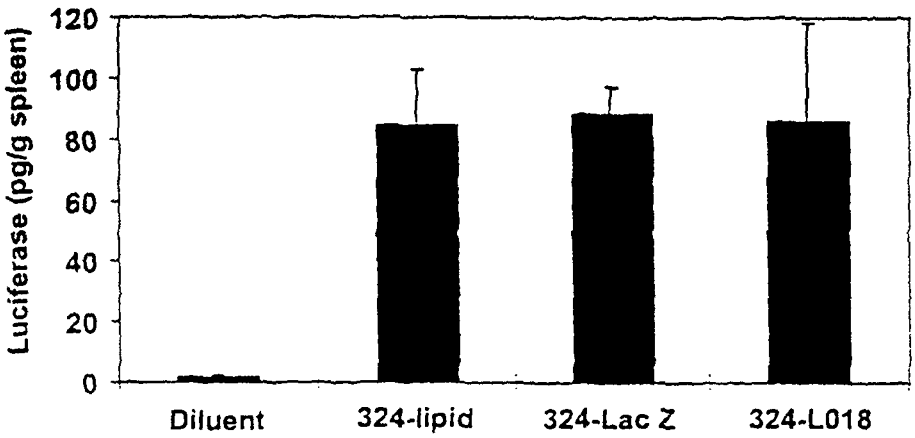
Luciferase (pg/g spleen)
120
100
80
60
40
20
0
Diluent
324-lipid
324-Lac Z
324-L018

FIGURE 32

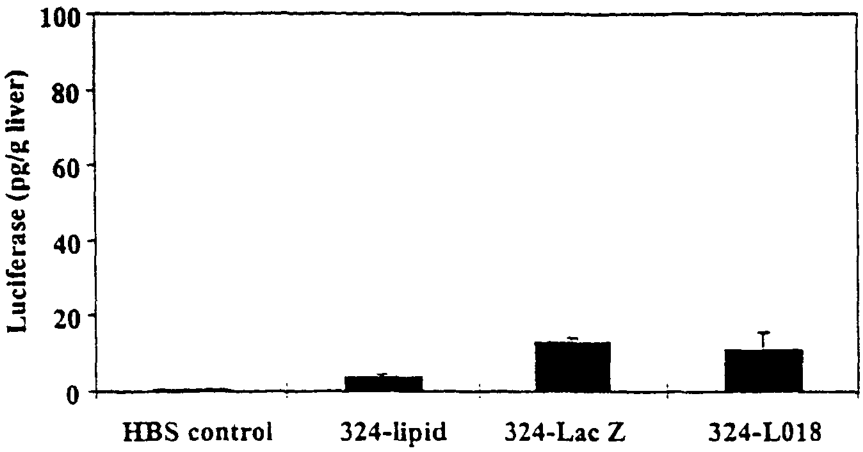
100
80
60
40
20
0
Luciferase (pg/g liver)
HBS control
324-lipid
324-Lac Z
324-L018

FIGURE 33